(19) Europäisches Patentamt / European Patent Office / Office européen des brevets

(11) **EP 3 574 101 B1**

(12) **EUROPEAN PATENT SPECIFICATION**

(45) Date of publication and mention
of the grant of the patent:
**19.04.2023 Bulletin 2023/16**

(51) International Patent Classification (IPC):
**C12N 15/82** (2006.01)   **C12N 15/10** (2006.01)
**C12N 15/11** (2006.01)   **C12N 15/90** (2006.01)
**A01H 5/00** (2018.01)

(21) Application number: **18703730.4**

(22) Date of filing: **30.01.2018**

(52) Cooperative Patent Classification (CPC):
**C12N 15/8213; A61P 35/00; A61P 37/06;
A61P 43/00; C12N 9/22; C12N 15/102;
C12N 15/111;** C12N 2310/20; C12N 2310/3519

(86) International application number:
**PCT/EP2018/052313**

(87) International publication number:
**WO 2018/138385 (02.08.2018 Gazette 2018/31)**

(54) **REPAIR TEMPLATE LINKAGE TO ENDONUCLEASES FOR GENOME ENGINEERING**

REPARATURTEMPLATEBINDUNG AN ENDONUKLEASEN ZUR GENOMMANIPULATION

LIAISON DE MODÈLE DE RÉPARATION À DES ENDONUCLÉASES POUR INGÉNIERIE
GÉNOMIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.01.2017 US 201762451859 P**

(43) Date of publication of application:
**04.12.2019 Bulletin 2019/49**

(73) Proprietor: **KWS SAAT SE & Co. KGaA
37574 Einbeck (DE)**

(72) Inventor: **LABS, Mathias
Frontenac, MO 63131 (US)**

(74) Representative: **Eisenführ Speiser
Patentanwälte Rechtsanwälte PartGmbB
Postfach 31 02 60
80102 München (DE)**

(56) References cited:
**WO-A1-2014/150624   WO-A1-2014/199358
WO-A1-2015/006290   WO-A1-2015/131101
WO-A1-2016/065364   WO-A1-2016/142719
WO-A1-2017/040511   WO-A1-2017/064546
WO-A1-2017/180711   WO-A1-2017/186550
WO-A2-2015/191693**

- **ANDREW D GARST ET AL: "Genome-wide mapping of mutations at single-nucleotide resolution for protein, metabolic and genome engineering", NATURE BIOTECHNOLOGY (ADVANCE ONLINE PUBLICATION), vol. 35, no. 1, 12 December 2016 (2016-12-12), pages 48-55, XP55456827, ISSN: 1087-0156, DOI: 10.1038/nbt.3718**
- **DONALD P. WEEKS ET AL: "Use of designer nucleases for targeted gene and genome editing in plants", PLANT BIOTECHNOLOGY JOURNAL, vol. 14, no. 2, 11 August 2015 (2015-08-11), pages 483-495, XP55456080, GB ISSN: 1467-7644, DOI: 10.1111/pbi.12448**
- **AREUM JO ET AL: "Efficient Mitochondrial Genome Editing by CRISPR/Cas9", BIOMED RESEARCH INTERNATIONAL, vol. 2015, no. 305716, 10 September 2015 (2015-09-10), pages 1-10, XP55403440, ISSN: 2314-6133, DOI: 10.1155/2015/305716**
- **KUNWOO LEE ET AL: "Synthetically modified guide RNA and donor DNA are a versatile platform for CRISPR-Cas9 engineering", ELIFE, vol. 6, 2 May 2017 (2017-05-02), XP55456761, DOI: 10.7554/eLife.25312**

- **JIAN-PING ZHANG ET AL: "Efficient precise knockin with a double cut HDR donor after CRISPR/Cas9-mediated double-stranded DNA cleavage", GENOME BIOLOGY, vol. 18, no. 1, 20 February 2017 (2017-02-20), XP55399694, DOI: 10.1186/s13059-017-1164-8**
- **JARED CARLSON-STEVERMER ET AL: "Assembly of CRISPR ribonucleoproteins with biotinylated oligonucleotides via an RNA aptamer for precise gene editing", NATURE COMMUNICATIONS, vol. 8, no. 1, 23 November 2017 (2017-11-23), XP55456055, DOI: 10.1038/s41467-017-01875-9**
- **ALI ZAHIR ET AL: "Fusion of the Cas9 endonuclease and the VirD2 relaxase facilitates homology-directed repair for precise genome engineering in rice", COMMUNICATIONS BIOLOGY, vol. 3, no. 1, 23 January 2020 (2020-01-23),**
- **SCHEIFFELE P ET AL: "Initiation of Agrobacterium tumefaciens T-DNA processing. Purified proteins VirD1 and VirD2 catalyze site- and strand-specific cleavage of superhelical T-border DNA in vitro.", THE JOURNAL OF BIOLOGICAL CHEMISTRY 20 JAN 1995, vol. 270, no. 3, 20 January 1995 (1995-01-20), pages 1269-1276, ISSN: 0021-9258**
- **YOSHIMI KAZUTO ET AL: "ssODN-mediated knock-in with CRISPR-Cas for large genomic regions in zygotes.", NATURE COMMUNICATIONS 20 JAN 2016, vol. 7, 20 January 2016 (2016-01-20), page 10431, ISSN: 2041-1723**
- **DYM ORLY ET AL: "Crystal structure of the Agrobacterium virulence complex VirE1-VirE2 reveals a flexible protein that can accommodate different partners.", PROCEEDINGS OF THE NATIONAL ACADEMY OF SCIENCES OF THE UNITED STATES OF AMERICA 12 AUG 2008, vol. 105, no. 32, 12 August 2008 (2008-08-12), pages 11170-11175, ISSN: 1091-6490**

Remarks:
The complete document including Reference Table(s) and the Sequence Listing(s) can be downloaded from the EPO website

## Description

### Technical Field

The present invention relates to artificial molecular complexes and particularly uses thereof comprising at least one site-specific nuclease and directly interacting therewith at least one repair template docking domain, said repair template docking domain interacting with at least one repair template nucleic acid sequence. An artificial complex can further comprise at least one interaction domain. The artificial molecular complexes are configured to mediate repair of a DNA target sequence in a prokaryotic or eukaryotic or viral organism or genome with high precision in a targeted way and can thus be used for genome engineering in a prokaryotic or a eukaryotic cell or organism or genome engineering with a prokaryotic, eukaryotic, or viral genome *in vivo* or *in vitro.* Further provided are methods of modifying at least one DNA target sequence in a prokaryotic or eukaryotic cell, or a viral genome, e.g., for trait development, or for treating a disease. Additionally, there is provided a method for manufacturing a plant, plant cell, a plant material, or a derivative, or a progeny thereof comprising or edited by at least one artificial molecular complex. There is thus provided the use of an artificial molecular complex suitable for any site-specific nuclease which directs a repair template in close physical proximity to a DNA target sequence to be modified to allow ready availability of a repair template *in situ* at the site of an induced DNA double-strand break to guarantee high efficiency and predictability for a variety of genome engineering approaches.

### Background of the Invention

Precision gene editing or genome engineering has evolved as one of the most important areas of genetic engineering allowing the targeted and site-directed manipulation of a genome of interest. An indispensable prerequisite for site-directed genome engineering are programmable nucleases, which can be used to break a nucleic acid of interest at a defined position to induce either a double-strand break (DSB) or one or more single-strand breaks. Alternatively, said nucleases can be chimeric or mutated variants, no longer comprising a nuclease function, but rather operating as recognition molecules in combination with another enzyme. Those nucleases or variants thereof are thus key to any gene editing or genome engineering approach. In recent years, many suitable nucleases, especially tailored endonucleases have been developed comprising meganucleases, zinc finger nucleases, TALE nucleases, Argonaute nucleases, derived, for example, from *Natronobacterium gregoryi,* and CRISPR nucleases, comprising, for example, Cas, Cpf1, CasX or CasY nucleases as part of the Clustered Regularly Interspaced Short Palindromic Repeats (CRISPR) system.

CRISPRs (Clustered Regularly Interspaced Short Palindromic Repeats) in their natural environment originally evolved in bacteria where the CRISPR system fulfils the role of an adaptive immune system to defend against viral attack. Upon exposure to a virus, short segments of viral DNA are integrated into the CRISPR locus. RNA is transcribed from a portion of the CRISPR locus that includes the viral sequence. That RNA, which contains sequence complementary to the viral genome, mediates targeting of a CRISPR effector protein to a target sequence in the viral genome. The CRISPR effector protein cleaves and thereby interferes with replication of the viral target. Over the last years, the CRISPR system has successfully been adapted for gene editing or genome engineering also in eukaryotic cells. Editing in animal cells and therapeutic applications for human beings are presently of significant research emphasis. The targeted modification of complex animal and also plant genomes still represents a demanding task.

A CRISPR system in its natural environment describes a molecular complex comprising at least one small and individual non-coding RNA in combination with a Cas nuclease or another CRISPR nuclease like a Cpf1 nuclease (Zetsche et al., "Cpf1 Is a Single RNA-Guides Endonuclease of a Class 2 CRISPR-Cas System", Cell, 163, pp. 1-13, October 2015) which can produce a specific DNA double-stranded break. Presently, CRISPR systems are categorized into two classes comprising five types of CRISPR systems, the Type II system, for instance, using Cas9 as effector and the Type V system using Cpf1 as effector molecule (Makarova et al., Nature Rev. Microbial., 2015). In artificial CRISPR systems, a synthetic non-coding RNA and a CRISPR nuclease and/or optionally a modified CRISPR nuclease, modified to act as nickase or lacking any nuclease function, can be used in combination with at least one synthetic or artificial guide RNA or gRNA combining the function of a crRNA and/or a tracrRNA (Makarova et al., 2015, supra). The immune response mediated by CRISPR/Cas in natural systems requires CRISPR-RNA (crRNA), wherein the maturation of this guiding RNA, which controls the specific activation of the CRISPR nuclease, varies significantly between the various CRISPR systems which have been characterized so far. Firstly, the invading DNA, also known as a spacer, is integrated between two adjacent repeat regions at the proximal end of the CRISPR locus. Type II CRISPR systems code for a Cas9 nuclease as key enzyme for the interference step, which systems contain both a crRNA and also a trans-activating RNA (tracrRNA) as the guide motif. These hybridize and form double-stranded (ds) RNA regions which are recognized by RNAse III and can be cleaved in order to form mature crRNAs. These then in turn associate with the Cas molecule in order to direct the nuclease specifically to the target nucleic acid region. Recombinant gRNA molecules can comprise both, the variable DNA recognition region and also the Cas interaction region, and can be specifically designed, independently of the specific target nucleic acid and the desired Cas nuclease. As a further safety mechanism, PAMs (protospacer adjacent motifs) must be present in the target nucleic acid region; these are DNA sequences which follow on directly from the Cas9/RNA complex-recognized DNA. The PAM sequence for the Cas9 from *Streptococcus pyogenes* has been described to be "NGG" or "NAG" (Standard IUPAC nucleotide code) (Jinek et al, "A programmable dual-RNA-guided DNA endonuclease in adaptive bacterial immunity", Science 2012, 337: 816-821). The PAM sequence for Cas9 from *Staphylococcus aureus* is "NNGRRT" or "NNGRR(N)". Further variant CRISPR/Cas9 systems are known. Thus, a *Neisseria meningitidis* Cas9 cleaves at the PAM sequence NNNNGATT. A *Streptococcus thermophilus* Cas9 cleaves at the PAM sequence NNAGAAW. Recently, a further PAM motif NNNNRYAC has been described for a CRISPR system of *Campylobacter* (WO 2016/021973 A1). For Cpf1 nucleases it has been described that the Cpf1-crRNA complex efficiently cleaves target DNA proceeded by a short T-rich PAM in contrast to the commonly G-rich PAMs recognized by Cas9 systems (Zetsche et al., supra). Furthermore, by using modified CRISPR polypeptides, specific single-stranded breaks can be obtained. The combined use of Cas nickases with various recombinant gRNAs can also induce highly specific DNA double-stranded breaks by means of double DNA nicking. By using two gRNAs, moreover, the specificity of the DNA binding and thus the DNA cleavage can be optimized.

Presently, for example, Type II systems relying on Cas9, or a variant or any chimeric form thereof, as endonuclease have been modified for genome engineering. Synthetic CRISPR systems consisting of two components, a guide RNA (gRNA) also called single guide RNA (sgRNA) and a non-specific CRISPR-associated endonuclease can be used to generate knock-out cells or animals by co-expressing a gRNA specific to the gene to be targeted and capable of association with the endonuclease Cas9. Notably, the gRNA is an artificial molecule comprising one domain interacting with the Cas or any other CRISPR effector protein or a variant or catalytically active fragment thereof and another domain interacting with the target nucleic acid of interest and thus representing a synthetic fusion of crRNA and tracrRNA ("single guide RNA" (sgRNA) or simply "gRNA"; Jinek et al., 2012, supra). The genomic target can be any ~20 nucleotide DNA sequence, provided that the target is present immediately upstream of a PAM. The PAM sequence is of outstanding importance for target binding and the exact sequence is dependent upon the species of Cas9 and, for example, reads 5' NGG 3' or 5' NAG 3' (Standard IUPAC nucleotide code) (Jinek et al., 2012, supra) for a *Streptococcus pyogenes* derived Cas9. Using modified Cas nucleases, targeted single-strand breaks can be introduced into a target sequence of interest. The combined use of such a Cas nickase with different recombinant gRNAs highly site specific DNA double-strand breaks can be introduced using a double nicking system. Using one or more gRNAs can further increase the overall specificity and reduce off-target effects.

Once expressed, the Cas9 protein and the gRNA form a ribonucleoprotein complex through interactions between the gRNA "scaffold" domain and surface-exposed positively-charged grooves on Cas9. Importantly, the "spacer" sequence of the gRNA remains free to interact with target DNA. The Cas9-gRNA complex will bind any genomic sequence with a PAM, but the extent to which the gRNA spacer matches the target DNA determines whether Cas9 will cut. Once the Cas9-gRNA complex binds a putative DNA target, a "seed" sequence at the 3' end of the gRNA targeting sequence begins to anneal to the target DNA. If the seed and target DNA sequences match, the gRNA will continue to anneal to the target DNA in a 3' to 5' direction (relative to the polarity of the gRNA).

Recently, engineered CRISPR/Cpf1 systems in addition to CRISPR/Cas9 systems become more and more important for targeted genome engineering (see Zetsche et al., supra and EP 3 009 511 A2). The Type V system together with the Type II system belongs to the Class 2 CRISPR systems (Makarova and Koonin Methods. Mol. Biol., 2015, 1311:47-753). The Cpf1 effector protein is a large protein (about 1,300 amino acids) that contains a RuvC like nuclease domain homologous to the corresponding domain of Cas9 along with a counterpart to the characteristic arginine-rich cluster of Cas9. However, Cpf1 lacks the HNH nuclease domain that is present in all Cas9 proteins, and the RuvC-like domain is contiguous in the Cpf1 sequence, in contrast to Cas9 where it contains long inserts including the HNH domain (Chylinski, 2014; Makarova, 2015). Cpf1 effectors possess certain differences over Cas9 effectors, namely no requirement of additional trans-activating crRNAs (tracrRNA) for CRISPR array processing, efficient cleavage of target DNA by short T-rich PAMs (in contrast to Cas9, where the PAM is followed by a G-rich sequence), and the introduction of staggered DNA double-strand breaks by Cpf1. Very recently, additional novel CRISPR-Cas systems based on CasX and CasY have been identified which due to the relatively small size of the effector protein are of specific interest for many gene editing or genome engineering approaches (Burstein et al., "New CRISPR-Cas systems from uncultivated microbes", Nature, December 2016). The specificity of CRISPR systems is determined in large part by how specific the gRNA targeting sequence is for the genomic target compared to the rest of the genome.

The kingdom of *Plantae* comprises species of high heterogeneity and diversity given the genomic and phenotypic differences of green algae, bryophytes, pteridophytes and land plants. Plant genomes and their complexity represent a challenge for high precision gene editing or genome engineering. *Zea mays* (maize or corn), for example, has the highest worldwide production of all grain crops, yielding 875 million tons in 2012. It has a large genome of about 2.4 gigabases (Gb) with a haploid chromosome number of 10 (Schnable et al, 2009; Zhang et al, 2009). *Triticum aestivum* (bread wheat), for instance, is hexaploid, with a genome size estimated at ~17 Gb. *Beta vulgaris* ssp. *vulgaris* (sugar beet) has a genome size ranging from about 470 megabases (Mb) to about 569 Mb. The specific architecture and composition of plant cells and the peculiar development of plants demands a specific adaption for genome engineering tools when intended for use to modify a target sequence within a plant cell. Therefore, genome engineering tools and principles associated therewith established for animal, particularly mammalian, systems will not necessarily work in a plant cell of interest and there is a need for specific strategies for establishing the technology to achieve a broad application in plants.

Likewise, animal, and especially mammalian genomes are complex, for example comprising 2.7 Gb for the genome of *Mus musculus* or 3.2 Gb for the genome of *Homo sapiens.* Especially, when CRISPR based gene editing or genome engineering approaches are intended to be used for precision gene editing or genome engineering of targets within the human genome, there is thus an urgent need to provide high specificity, as any kind of off-target effect could be highly detrimental.

Another aspect to be critically considered for genome engineering is the repair mechanism necessary after the cleavage of a genomic target site of interest, as double-strand breaks (DSBs) or DNA lesions in general are detrimental for the integrity of a genome. DSBs in genomic material can be caused by ionizing radiation, chemicals, oxidation, enzymes, and single-strand breaks during replication and represent a serious form of DNA damage which can result in gene loss, stalled DNA replication, and cell death. It is thus of outstanding importance that the cellular machinery provides mechanisms of double-strand break (DSB) repair. Cells possess intrinsic mechanisms to attempt to repair any double- or single-stranded DNA damage. DSB repair mechanisms have been divided into two major basic types, non-homologous end joining (NHEJ) and homologous recombination (HR). Homology based repair mechanisms in general are usually called homology-directed repair (HOR).

NHEJ is the dominant nuclear response in animals and plants which does not require homologous sequences, but is often error-prone and thus potentially mutagenic (Wyman C., Kanaar R. "DNA double-strand break repair: all's well that ends well", Annu. Rev. Genet. 2006; 40, 363-83). Repair by HOR requires homology, but those HOR pathways that use an intact chromosome to repair the broken one, i.e., double-strand break repair and synthesis-dependent strand annealing, are highly accurate. In the classical DSB repair pathway, the 3' ends invade an intact homologous template then serve as a primer for DNA repair synthesis, ultimately leading to the formation of double Holliday junctions (dHJs). dHJs are four-stranded branched structures that form when elongation of the invasive strand "captures" and synthesizes DNA from the second DSB end. The individual HJs are resolved via cleavage in one of two ways. Synthesis-dependent strand annealing is conservative, and results exclusively in non-crossover events. This means that all newly synthesized sequences are present on the same molecule. Unlike the NHEJ repair pathway, following strand invasion and D loop formation in synthesis-dependent strand annealing, the newly synthesized portion of the invasive strand is displaced from the template and returned to the processed end of the non-invading strand at the other DSB end. The 3' end of the non-invasive strand is elongated and ligated to fill the gap. There is a further pathway of HOR, called break-induced repair pathway not yet fully characterized. A central feature of this pathway is the presence of only one invasive end at a DSB that can be used for repair.

A further HOR pathway is single-strand annealing (SSA). SSA is non-conservative and occurs between direct repeats >30 bp and results in deletions. In recent years, microhomo-logy-mediated end joining (MMEJ) has been recognized as a distinct type of DSB repair in eukaryotes. Only very short (2-14 bp) regions of homology are needed for this pathway, and it typically leaves deletions like SSA. It has also been distinguished genetically from the HR and NHEJ pathways and in mammalian cells acts as a backup to NHEJ (Kwon, T., Huq, E., & Herrin, D. L. (2010). "Microhomology-mediated and nonhomologous repair of a double-strand break in the chloroplast genome of Arabidopsis." Proceedings of the National Academy of Sciences of the United States of America, 107(31), 13954-13959). In sum, HR/HOR employs a homologous stretch of DNA on a sister chromatid as a template. It thus provides high fidelity, however, less efficiency. NHEJ in contrast is highly efficient and a straightforward pathway that can rejoin the two ends independently of significant homology, whereas this efficiency is accompanied by the drawback that this process is error-prone and can be associated with insertions or deletions.

For gene editing or genome engineering approaches seeking to influence the natural repair pathways thus require physical design of a repair template (RT), which is an important parameter. It can be possible to provide the RT as either ssDNA or as partially dsDNA. Current protocols relying on CRISPR tools for genome editing in combination with a repair template (RT) exclusively rely on the separate provision of the nucleic acid RT, either double- or single-stranded, which in turn recognizes the break in the DNA to be repaired solely by base pairing and hybridization. The physical and temporal availability of the RT at the site where a DNA break is induced, can, however, not be controlled by the methods presently available, as those methods do not provide for the precise spatial and temporal provision of the RT in the right configuration, concentration and thus stochiometry at the compartment, where repair has to take place, preferably immediately after induction of a targeted DNA break to specifically control not only the break, but also the repair event.

Like CRISPR/Cas nucleases, Argonaute endonucleases ("Argonautes") are involved in defense against foreign nucleic acids by using nucleic acid guides to specify a target sequence, which is then cleaved by the Argonaute protein component. Specifically, an Argonaute can bind and cleave a target nucleic acid by forming a complex with a designed or synthetic nucleic acid-targeting nucleic acid, where cleavage of the target nucleic acid can introduce double-stranded breaks in the target nucleic acid. Also like the Cas9 system, the Argonautes nucleic acid guides provide a facile method for programming endonuclease sequence specificity. However, short ssRNA molecules are used as guides by many eukaryotic Argonautes without any secondary structure recognition constraints, such as those present in the Cas9-short guide RNA (sgRNA, gRNA) interaction. The abundance of ssRNA in most eukaryotic cells therefore makes specific targeting of RNA-guided eukaryotic Argonautes a potential challenge. In contrast, some prokaryotic Argonautes are guided by short 5'-phosphorylated ssDNA molecules (Swarts, D.C. et al. DNA-guided DNA interference by a prokaryotic Argonaute. Nature 507, 258-261, 2014; Swarts, D.C. et al. Argonaute of the archaeon Pyrococcus furiosus is a DNA-guided nuclease that targets cognate DNA Nucleic Acids Res. 43, 5120-5129 2015), and therefore inherently have lower potential for misguiding by host cell-derived nucleic acids due to the scarcity of short ssDNA molecules present in eukaryotic cells. Thus, DNA-guided Argonaute endonucleases have potential for application in eukaryotic genome editing. Use of the *Natronobacterium gregoryi* Argonaute (NgAgo) system in plants has, however, not been previously demonstrated.

In the literature, it has been documented that homologous recombination between two sequences occurs more frequently if the sequences are in close proximity within the nucleus rather than with a significant amount of separation. For example, analysis in *Arabidopsis* of the gene editing rate obtained between chromosomally located donor molecules and targets was higher in both cases where the donor existed on the same chromosome as the target than in the other cases where the two loci were located on distinct chromosomes (Fauser et al., 2012). However, these findings have never been exploited in a rational way to optimize site-specific endonuclease based gene editing or genome engineering approaches in eukaryotic cells.

EP 2 958 996 A1 seeks to overcome the problem of specific DSB repair by providing an inhibitor of NHEJ mechanisms in cell to increase gene disruption mediated by a nuclease (e.g., ZFN or TALEN) or nuclease system (e.g., CRISPR/Cas). By inhibiting the critical enzymatic activities of these NHEJ DNA repair pathways, using small molecule inhibitors of DNA-dependent-protein kinase catalytic subunit (DNA-PKcs) and/or Poly-(ADP-ribose) polymerase 1/2 (PARP1/2), the level of gene disruption by nucleases is increased by forcing cells to resort to more error prone repair pathways than classic NHEJ, such as alternate NHEJ and/or microhomology mediated end-joining. Therefore, an additional chemical is added in the course of genome editing, which might, however, be disadvantageous for several cell types and assays. This could also affect the genome integrity of the treated cells and/or the regenerative potential.

Ma et al. (2016, JCB, 214(5):529, "CRISPR-Cas9 nuclear dynamics and target recognition in living cells") used a 3'-modified sgRNA that allowed for aptamer-based binding of a fluorescent reporter to study the dynamics of Cas9 and sgRNA dynamics towards a telomeric target. Notably, the modification within the tracrRNA sequence did not have an effect on targeting. Only the subsequent truncation of the tracrRNA sequence led to destabilized sgRNA independent of the aptamer-modification.

Therefore, there exists an ongoing need in providing suitable CRISPR tools, particularly tools optimized for the precision editing of plants, especially major crop plants, which combine high precision genome cleavage, for example by providing gRNAs optimized for the target site in a cell of interest and simultaneously providing the possibility for mediating highly precise and accurate HOR and thus targeted repair of a DSB, which is imperative to control a gene editing or genome engineering intervention.

It is thus an aim to present novel strategies to provide repair templates for precision genome editing, especially suitable for eukaryotic cells, including yeast, animal and plant cells, but also being suitable for prokaryotic cells, e.g., for metabolic engineering and various other purposes, or for the modification of viral genomes, e.g., to attenuate a virus, or to reduce the virulence of a virus. Despite the tremendous advancements of genome editing in biotechnology, e.g., for therapeutic approaches, gene therapy or plant or microbe genome engineering for targeted trait development, there are still major problems and concerns with respect to the specificity of a targeted genome modification to be introduced or off-target effects. This problem is *inter alia* associated with the degree of precision which can be obtained when inducing a break and the associated repair of a genomic target nucleic acid of interest.

As any kind of gene editing or genome engineering approach inducing a DSB introduces a potentially harmful DNA break and possibly an undesired DNA repair mechanism leading to unwanted nucleic acid exchanges, there is an ongoing need in developing more efficient methods and tools to achieve highly precise and controlled gene editing or genome engineering which also implies the use of targeted DNA repair templates (RTs).

Another problem frequently associated with the provision of successful genome engineering without mediating off-target effects is the physical availability of a repair template at the site of the DSB exactly at the time the break is made and thus has to be repaired. Usually the desired editing event is outcompeted by repair through the non-homologous end-joining (NHEJ) pathway orthrough recombination with endogenous homologous sequence as detailed above. Depending on the target organism to be modified, this demands a concerted strategy for introducing a gene editing or genome engineering tool along with a repair template of interest so that all tools can, with the appropriate timing, reach the compartment within a cell comprising the genome, i.e., preferably the nucleus, or any other genome carrying compartment, like the mitochondria. One method to partially overcome this limitation is by amplifying the repair template and thus increasing the abundance of the template in the nucleus and presumably making it more available to use for repair of the DSB by help of a geminivirus vector (see e.g., Mach, Plant Cell. 2014, doi:10.1105/tpc.114.122606; and Baltes et al., Plant Cell. 2014, doi:10.1105/tpc.113.119792). The repair template, however, is delivered as separate physical entity and thus there is no mechanism of control ascertaining that the repair template will indeed be present at the place DNA repair is needed exactly at the time point, when a DSB is introduced by an endonuclease.

Concerning CRISPR applications, there is the frequent suggestion to use free ssDNA nucleotides as repair templates or plasmid borne repair templates, yet no strategy is disclosed or suggested, which would guarantee that the repair template is indeed brought into physical contact with the DSB to be repaired *in situ* when a DSB is generated.

Biotin-streptavidin and biotin-avidin interactions are amongst the most stable in nature, with a dissociation constant $K_d$ of $10^{-15}$ M. The association is based on a homotetrameric structure between avidin or streptavidin protein (~16.5 and 13.2 kDa per subunit, respectively) and the universally present, but low abundant, vitamin biotin. The homotetrameric streptavidin or avidin complexes form spontaneously and are capable of binding four biotin molecules with low dissociation constants. In at least two attempts, the spontaneous tetramerization could be overcome with a decrease in binding affinity (Laitinen et al. 2003, "Rational Design of an Active Avidin Monomer." Journal of Biological Chemistry 278(6): 4010-4014; Mann et al. 2016, "Cell labeling and proximity dependent biotinylation with engineered monomeric streptavidin." TECHNOLOGY 4(3): 1-7). Likewise, biotinylation of a nuclease was demonstrated to be possible by including a biotinylation signal in the sequence (Kay et al. 2009, "High-throughput Biotinylation of Proteins." Methods in molecular biology (Clifton, N.J.) 498: 185-196). BirA is a possible biotinylating enzyme for bacterial protein expression, but biotinylation also occurs in higher plants (Tissot et al. 1996, "Protein biotinylation in higher plants: characterization of biotin holocarboxylase synthetase activity from pea (Pisum sativum) leaves.", Biochemical Journal 314(Pt 2): 391-395).

Single-chain variable fragments (scFvs) represent fusion proteins of the variable regions of the heavy ($V_H$) and light chains ($V_L$) of immunoglobulins, connected with a short linker peptide of ten to about 25 amino acids and are known as versatile high affinity binding molecules. Divalent (or bivalent) single-chain variable fragments (di-scFvs, bi-scFvs) can be engineered by linking two scFvs. This can be achieved by producing a single peptide chain with two $V_H$ and two $V_L$ regions, yielding tandem scFvs (Kufer et al., 2004, Trends in Biotechnology, 22(5), 238-244; Xiong et al., 2006, Protein Engineering Design and Selection, 19(8), 359-367).

So far, these findings about the capacities of biotinylated molecules and their cognate binding partners, or about other high-affinity molecular binding pairs, like for example antibodies or single-chain variable fragments and their cognate partners, have, however, not yet been exploited for targeted genome engineering using site-specific nucleases and a repair template.

WO 2015/191693 A2 discloses delivery systems for mammalian cells in which a repair template can be bound to a guide RNA. WO 2016/065364 A1 discloses a similar system in T-cells and further describes tethering of a donor nucleotide to an endonuclease.

At this point, the peculiar differences of the delivery of gene editing or genome engineering and/or repair template tools as necessary for different target cells become evident. In this regard, plant cells have certain distinguishing features, including cell walls, making gene editing or genome engineering in plant cells a completely different task than gene editing or genome engineering as established for animal/mammalian cells, as the delivery of genome editing and/or repair tools is mediated by different transformation, transfection and/or transduction methods than for other eukaryotic cells. These peculiarities, however, have to be taken into consideration for achieving highly precise plant genome editing. Therefore, it was an object of the present invention to overcome the pronounced need in providing new tools and methods suitable for high precision genome editing in eukaryotic cells, including plant cells, particularly in the field of CRISPR and Argonaute mediated genome editing to overcome the ongoing limitation in the field of gene editing regarding the physical availability of the repair template at the site and time the DSB is repaired and thus the competition by DNA repair mechanisms through the non-homologous end-joining pathway (NHEJ) or through recombination with (endogenous) homologous sequence (HR/HOR). The present disclosure provides a simplified site-directed nuclease toolkit suitable for any site-specific nuclease and not being restricted to nucleic acid guided CRISPR or Argonaute nucleases, which can be utilized for site-directed genome editing in eukaryotic or prokaryotic cells or to any prokaryotic, eukaryotic or viral genome by providing a molecule or a molecular complex which unifies DNA recognition, cleavage and repair template properties and simultaneously can be easily delivered to the target site, i.e., a prokaryotic cell, a eukaryotic or viral genome, particularly the genome of an animal cell, particularly a mammalian cell, or of a plant cell, as the degree of precision to be achieved during genome editing of animal or plant cells still has to be improved to comply with necessarily high regulatory requirements as set by medical and food administration authorities. The risk for off-target integrations of the artificial molecular complexes as disclosed herein is lower than for a ss- or ds-DNA repair template introduced as free molecules into the cell. In addition, it was an object to provide a delivery tool that is specifically optimized for transferring a plant specific genome editing construct with the help of a plant specific delivery method. In addition, it was an object to provide an approach which can rely on transient editing activity using transiently provided RNA and site-specific nucleases, if desired, because of the sensitivity in certain jurisdictions towards any form of genetic modification that integrates foreign DNA as an intermediate in the production process. It was an object of the present invention to provide a gene editing or genome engineering method, which is superior to recent methods in that it is time saving regarding the testing of new targets as it should not require cumbersome cloning and pre-testing.

**Summary**

The present invention is as defined in the claims.

The above identified objects have been achieved according to the present invention by solving the problem of repair template availability by delivering the repair template to the site of the DSB by directly harnessing it as "cargo" to the nuclease complex, whereas the spectrum of nucleases suitable for this approach has been dramatically increased by providing artificial molecular complexes, which rely on any site-specific nuclease (SSN) of interest. Directing the repair template to the double-strand break at the time the break is made *in situ* by providing at least one repair template docking domain (RTDD) together with at least one SSN, wherein the repair template docking domain is configured to directly interact with at least one repair template nucleic acid sequence (RT) increasing the local availability of the repair template (RT) for exploitation in repair of the break. Thereby, the artificial molecular complexes according to the present invention do not only assist in providing custom-made repair templates, but furthermore can help to increase the frequency and/or specificity of gene editing events. This idea thus combines the functionalities of site-specific nuclease and repair templates into a single molecular complex for simultaneous genome cleavage and targeted repair combined with specific delivery tools and methods for delivering the genome editing tool(s) and/or the repair template into a compartment of interest into a target cell. This system thus allows a higher specificity and thus reduced off-target effects of present editing approaches, which is needed to minimize off-target cleavage in large animal, particularly mammalian, or sometimes even more complex plant genomes.

Specifically, the above objects have been achieved by providing, in a first aspect of the present disclosure, an artificial molecular complex, comprising (a) at least one site-specific nuclease (SSN) or a catalytically active fragment thereof, or a nucleic acid sequence encoding the same, and directly interacting therewith (b) at least one repair template docking domain (RTDD), or a nucleic acid sequence encoding the same, wherein the repair template docking domain is configured to directly interact with at least one repair template nucleic acid sequence (RT); (c) optionally comprising at least one interaction domain (IA), or a nucleic acid sequence encoding the same, wherein the at least one interaction domain is directly interacting with the at least one site-specific nuclease or the catalytically active fragment thereof, and wherein the at least one interaction domain is configured to provides at least one of the functionalities selected from the group consisting of (i) interaction with the at least one repairtemplate docking domain; and/or (ii) interaction with the at least one repair template nucleic acid sequence; and/or (iii) sequence-specific interaction with genomic DNA; wherein the at least one repair template nucleic acid sequence comprises at least one portion being complementary to at least one genomic complementarity sequence, and wherein the at least one repair template nucleic acid sequence is configured to mediate repair of a DNA target sequence.

In one embodiment according to the various aspects of the present disclosure, there is provided an artificial molecular complex, wherein the site-specific nuclease, or the nucleic acid sequence encoding the same, is selected from at least one of a CRISPR nuclease, including Cas or Cpf1 nucleases, a TALEN, a ZFN, a meganuclease, a restriction endonuclease, including a class IIS restriction endonuclease, including FokI or a variant thereof, or two site-specific nicking endonucleases, or a variant or a catalytically active fragment thereof.

In another embodiment of the present disclosure, there is provided an artificial molecular complex, wherein the at least one repair template docking domain, or the nucleic acid sequence encoding the same, is selected from at least one of biotin, an aptamer, a DNA, RNA or protein dye, comprising fluorophores, comprising fluorescein, or a variant thereof, maleimides, or Tetraxolium (XTT), a guide nucleic acid sequence specifically configured to interact with a at least one repair template nucleic acid sequence, a streptavidin, or a variant thereof, such as a monomeric steptavidin, an avidin, or a variant thereof, an affinity-tag, such as a streptavidin-tag, an antibody, a single-chain variable fragment (scFv), a single-domain antibody (nanobody), an anticalin, an Agrobacterium VirD2 protein or a domain thereof, a Picornavirus VPg, a topoisomerase or a domain thereof, a PhiX174 phage A protein, a PhiX A* protein, a VirE2 protein or a domain thereof, or digoxigenin. Another well-known system for interaction is SNAP-tag for instance fused to a dCas9 as offered by New England Biolabs Inc. (www.neb.com). The SNAP-tag is able to bind a series of fluorophores, biotin, and other conjugates. The main purpose is to allow visualization, but it would be useful for tethering the repair template as well.

In yet another embodiment of the above first aspect according to the present disclosure, there is provided an artificial molecular complex, wherein the at least one interaction domain, or the nucleic acid sequence encoding the same, is selected from at least one of a DNA-binding domain, a streptavidin, or a variant thereof, such as a monomeric steptavidin, avidin, or a variant thereof, an affinity tag, a biotinylation signal, a biotin acceptor site, a streptavidin-tag, an antibody, a single-chain variable fragment (scFv), a single-domain antibody (nanobody), an anticalin, biotin, an aptamer, a DNA, RNA or protein dye, comprising fluorophores, comprising fluorescein, or a variant thereof, maleimides, or Tetraxolium (XTT), a guide nucleic acid sequence specifically configured to interact with a at least one repair template nucleic acid sequence, an Agrobacterium VirD2 protein or a domain thereof, a Picornavirus VPg, a topoisomerase or a domain thereof, a PhiX174 phage A protein, a PhiX A* protein, a VirE2 protein or a domain thereof, or digoxigenin.

In yet a further embodiment of the present disclosure, there is provided an artificial molecular complex, wherein the at least one site-specific nuclease and/or the at least one interaction domain comprises at least one nuclear localization sequence, a plastid localization sequence, such as a mitochondrion localization sequence or a chloroplast localization sequence.

In another embodiment according to the various aspects of the present disclosure, there is provided an artificial molecular complex, wherein the at least one repair template nucleic acid sequence comprises at least one end portion, such as the 3' end, wherein this end portion does not interact with any other component of the artificial molecular complex and is thus configured to hybridize to at least one genomic complementarity sequence to mediate repair of the DNA target sequence, and/or wherein the at least one repair template nucleic acid sequence is provided as plasmid.

In still another embodiment of the present disclosure, there is provided an artificial molecular complex, wherein the at least one site-specific nuclease or the catalytically active fragment thereof, or the sequence encoding the same, is selected from a CRISPR nuclease, such as from a Cas or a Cpf1 nuclease, or a FokI nuclease, or a catalytically fragment thereof, and the at least one interaction domain, or the sequence encoding the same, is selected from a single-chain variable fragment or a monomeric streptavidin.

Furthermore, there is provided, in another embodiment of the present disclosure, an artificial molecular complex, wherein the complex comprises at least one guide nucleic acid sequence representing the at least one repair template docking domain, wherein each of the at least one guide nucleic acid sequences comprises (i) a first sequence portion that is complementary to a recognition DNA target sequence, and (ii) a second sequence portion, wherein the second sequence portion is configured to interact with the at least one site-specific nuclease, and (iii) wherein the at least one guide nucleic acid sequence is physically associated with the at least one repair template nucleic acid sequence and thus forms a hybrid nucleic acid sequence comprising or consisting of at least one RNA or DNA and at least one further DNA nucleic acid sequence, and (iv) optionally comprising a linker region between the at least one guide nucleic acid sequence and the at least one repair template nucleic acid sequence, for instance wherein the repair template nucleic acid sequence is associated with the guide nucleic acid sequence at the 3' end of the guide nucleic acid sequence, and/or wherein the repair template nucleic acid sequence is associated with the 5'-end of the guide nucleic acid sequence, and/or wherein the repair template nucleic acid sequence is located within the guide nucleic acid sequence.

In another embodiment of the present disclosure, there is provided an artificial molecular complex, wherein the at least one repair template nucleic acid sequence and/or the at least one guide nucleic acid sequence comprise a nucleotide sequence selected from a naturally or non-naturally occurring nucleotide sequence, including a synthetic nucleotide sequence, optionally comprising backbone and/or base modifications, wherein the guide nucleic acid sequence comprises a single-stranded or partially single-stranded RNA or DNA nucleotide sequence, and wherein the at least one repair template nucleic acid sequence comprises a single-stranded or a double-stranded DNA nucleotide sequence.

In yet a further embodiment according to the various aspects of the present disclosure, there is provided an artificial molecular complex, wherein the at least one site-specific nuclease, or the sequence encoding the same, and the at least one interaction domain, or the sequence encoding the same, and/or the at least one repair template docking domain, or the sequence encoding the same, are connected by at least one linker domain.

In one of the embodiments provided according the present disclosure, the at least one site-specific nuclease or the catalytically active fragment thereof, or the sequence encoding the same, is independently selected from the group consisting of a Cas polypeptide from *Streptococcus spp.,* including *Streptococcus pyogenes, Streptococcus thermophilus, Staphylococcus aureus,* or *Neisseria spp.,* including *Neisseria meningitides, Corynebacter, Sutterella, Legionella, Treponema, Filifactor, Eubacterium, Lactobacillus, Mycoplasma, Bacteroides, Flaviivola, Flavobacterium, Sphaerochaeta, Azospirillum, Gluconacetobacter, Roseburia, Parvibaculum, Nitratifractor, Mycoplasma, Campylobacter, Candidates* Micrarchaeum acidiphilum ARMAN-1, *Parcubacteria* (GenBank: APG80656.1), *Sulfolobus* spp., including *Sulfolobus islandicus* HVE10/4 (GenBank: ADX81770.1) or REY15A (GenBank: ADX84852.1), and *Candidates* Parvarchaeum acidiphilum ARMAN-4, a Cpf1 polypeptide from an archaea or a bacterium, including a Cpf1 polypeptide of *Acidaminococcus spp.,* including *Acidaminococcus sp.* BV3L6, *Lachnospiraceae spp.,* including *Lachnospiraceae bacterium* ND2006, *Lachnospiraceae* bacterium MC2017, *Lachnospiraceae* bacterium MA2020, *Butyrivibrio proteoclasticus,* Candidatus spp., *Methanoplasma termitum, Leptospira inadai, Moraxella bovoculi* 237, Peregrinibacteria bacterium GW2011_GWA2_33_10, Parcubacteria bacterium GW2011_GWC2_44_17, Smithella sp. SCADC, Smithella sp. SC_K08D17, *Francisella spp.,* including *Francisella novicida* U112, *Eubacterium eligens, Prevotella spp.,* or *Porphyromonas spp.,* or an Argonaute nuclease from *Natronobacterium gregoryi* (GenBank: AFZ73749.1), *Microcystis aeruginosa* (NCBI Reference Sequence: WP_012265209.1 or NCBI Reference Sequence: WP_002747795.1 or NCBI Reference Sequence: WP_012265209.1), *Halogeometricum pallidum* (GenBank: ELZ29017.1), *Natrialaba asiatica* (NCBI Reference Sequence: WP_006111085.1), *Natronorubrum tibetense* (NCBI Reference Sequence: WP_006090832.1), *Natrinema pellirubrum* (NCBI Reference Sequence: WP_006183335.1), or *Synechococcus spp.* (NCBI Reference Sequence: WP_011378069.1) or variants and/or functional fragments and/or combinations thereof, including nickases, or nucleases lacking endonucleolytic activity.

In a second aspect according to the present disclosure there is provided an artificial molecular complex according to any one of the preceding embodiments for use in a method of treatment of a disease, wherein the disease is characterized by at least one genomic mutation and the artificial molecular complex is configured to target and repair the at least one genomic mutation. There is thus provided a method of treating a disease using the artificial molecular complex according to any one of the preceding claims, wherein the disease is characterized by at least one genomic mutation and the artificial molecular complex is configured to target and repair the at least one genomic mutation.

In one aspect, there is provided a plant, plant cell, a plant material, or a derivative, or a progeny thereof comprising or edited by at least one artificial molecular complex according to any one of the preceding aspects and/or embodiments.

In a further aspect, there is provided a method of modifying at least one DNA target sequence comprising the following steps: (i) providing at least one prokaryotic, eukaryotic, or viral cell and/or genome (embodiments of the present invention being related to at least one plant cell and/or genome) comprising at least one genomic complementarity sequence and at least one DNA target sequence in a genomic region of interest; (ii) providing at least one artificial molecular complex as defined in any one preceding aspects and/or embodiments; (iii) contacting the at least one artificial molecular complex with the at least one DNA target sequence under suitable conditions to achieve (a) interaction of the at least one site-specific nuclease with the at least one DNA target sequence; and (b) complementary base pairing of the at least one repair template nucleic acid sequence with the at least one genomic complementarity sequence to achieve recognition of the at least one complementarity sequence and induction of at least one DNA break by the at least one site-specific nuclease, wherein the at least one repair template nucleic acid sequence directs homology directed repair at the site of the at least one DNA target sequence; and (iv) obtaining at least one prokaryotic, eukaryotic, or viral cell and/or genome comprising a modification in the at least one DNA target sequence; wherein, in embodiments of the present invention, the at least one repair template docking domain, or the nucleic acid sequence encoding the same, is selected from an Agrobacterium VirD2 protein.

In one embodiment of the above aspect, there is provided a method of modifying at least one DNA target sequence, wherein the at least one repair template nucleic acid sequence and/or the at least one repair template docking domain of the artificial molecular complex is/are provided to the at least one prokaryotic, eukaryotic, or viral cell and/or genome (at least one plant cell and/or genome in embodiments of the invention) independently of the at least one site-specific nuclease of the at least one molecular complex and the at least one artificial molecular complex is assembled, or partially assembled, within the at least one prokaryotic or eukaryotic cell.

In a further embodiment of the above aspect, there is provided a method of modifying at least one DNA target sequence, wherein the at least one artificial molecular complex is an ex *vivo* assembled artificial molecular complex.

In a further embodiment of the above aspect, there is provided a method of modifying at least one DNA target sequence, wherein the at least one eukaryotic cell is a plant cell, preferably a plant cell from a plant selected from the group consisting of *Hordeum vulgare, Hordeum bulbusom, Sorghum bicolor, Saccharum officinarium, Zea spp.,* including *Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and *Allium tuberosum,* or any variety or subspecies belonging to one of the aforementioned plants.

In a further embodiment, there is provided a method of modifying at least one DNA target sequence, wherein the modification of the at least one DNA target sequence causes a trait editing selected from the group consisting of yield improvement, tolerance to abiotic stress, including drought stress, osmotic stress, heat stress, cold stress, oxidative stress, heavy metal stress, salt stress or waterlogging, tolerance to biotic stress including tolerance to insects, tolerance to bacteria, tolerance to viruses, tolerance to fungi or tolerance to nematodes, resistance to herbicides, including glyphosate, glufosinate, ALS inhibitors, and Dicamba, lodging resistance, flowering time, shattering resistance, seed color, endosperm composition, nutritional content, or metabolic engineering, including genome editing to allow a molecular pharming approach in at least one plant cell.

Further provided is a method of modifying at least one DNA target sequence additionally comprising the following step: (v) identifying and/or selecting at least one prokaryotic, eukaryotic, or viral genome and/or cell comprising the modification in the at least one DNA target sequence.

In yet another aspect, there is provided a method for manufacturing a plant or plant cell comprising the following steps: (i) performing a method according to any one of the above aspects and/or embodiments, wherein the at least one eukaryotic cell is a plant cell; (ii) obtaining at least one plant or, according to the present disclosure, a progeny thereof from the at least one plant cell from step (i); (iii) optionally: determining the modification in the at least one DNA target sequence in the at least one cell of the at least one plant or a progeny thereof.

In one embodiment, there is provided a method for manufacturing a plant or plant cell, wherein the at least one plant or plant cell is selected from a monocotyledonous or a dicotyledonous plant, preferably, wherein the plant is selected from the group consisting of *Hordeum vulgare, Hordeum bulbusom, Sorghum bicolor, Saccharum officinarium, Zea spp.,* including *Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and *Allium tuberosum,* or any variety or subspecies belonging to one of the aforementioned plants.

In a further aspect, there is provided the use of at least one artificial molecular complex according to any one of the above aspects and/or embodiments for genome engineering in a prokaryotic, eukaryotic, or viral cell, genome or organism, for instance in a plant cell or organism, wherein embodiments of the invention relate to genome engineering in a plant cell or organism.

Further aspects of the present disclosure can be derived from the subsequent detailed description, the drawings, the sequence listing as well as the attached set of claims.

**Brief Description of the Drawings**

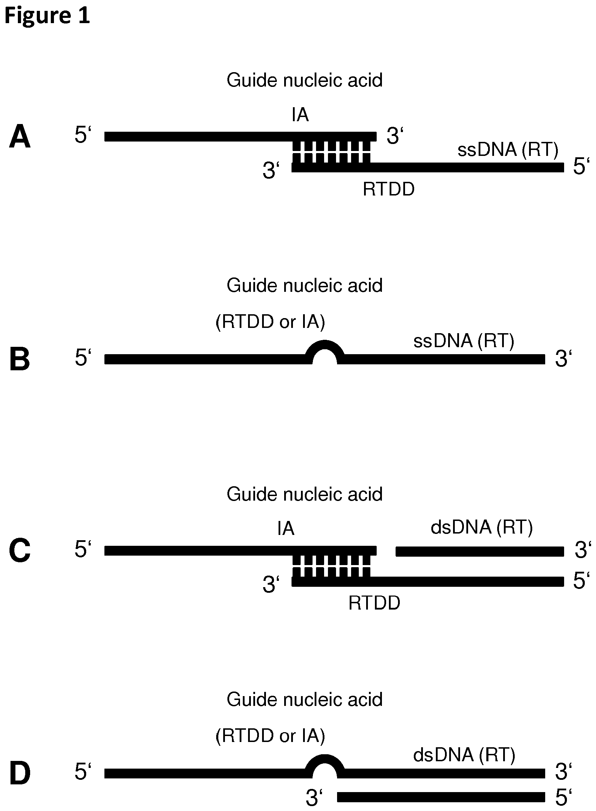

Figure 1 A to D (Fig. 1 A to D) show examples of possible configurations and different ways of association for different RNA-DNA hybrid or DNA-DNA nucleic acid sequences the guide nucleic acid portion representing the at least one repair template docking domain (RTDD) and/or the at least one interaction domain (IA) according to the present disclosure. (A) Non-covalent association by Watson-Crick base pairing of a single-stranded repair template (RT) (ssDNA) to a guide nucleic acid molecule containing the sequence functioning as a sgRNA or a tracrRNA or as a gDNA. (B) Covalent association of a single-stranded RT (ssDNA) to the guide nucleic acid molecule. This form can be manufactured by sequential synthesis of the RTDD guide nucleic acid molecule and RT portions as a single molecule, or by ligation of separate portions to form a single molecule. (C) Non-covalent association of a double-stranded RT (dsDNA) to the guide nucleic acid molecule. (D) Covalent association of a double-stranded RT (dsDNA) to the guide nucleic acid molecule.

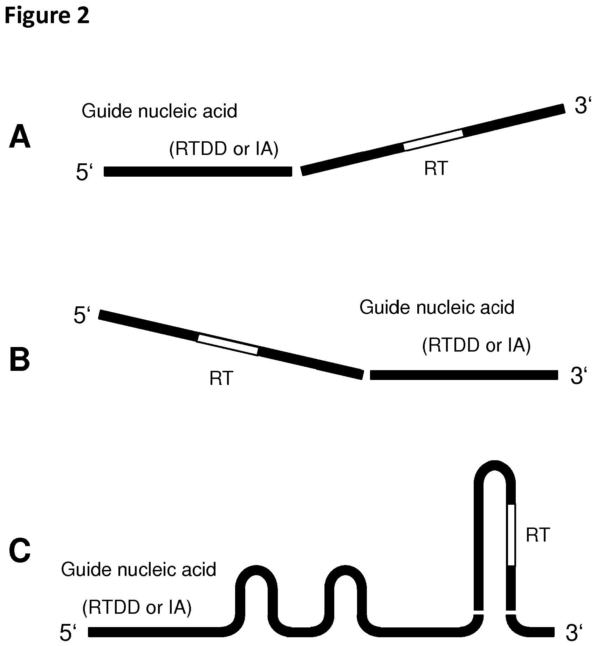

Figure 2 A to C (Fig. 2 A to C) show examples of possible locations at which the RT can be attached to or associated with a guide nucleic acid molecule as the at least one RTDD and/or the at least one IA according to the present disclosure. (A) Covalent or non-covalent association of the single- or double-stranded RT to the 3'-end of the guide nucleic acid molecule. (B) Covalent or non-covalent association of the single- or double-stranded RT to the 5'-end of the guide nucleic acid molecule. (D) Covalent or non-covalent association of the single- or double-stranded RT internal to the guide nucleic acid molecule. The repair template (RT) portion is shown in white in this and all further Figures.

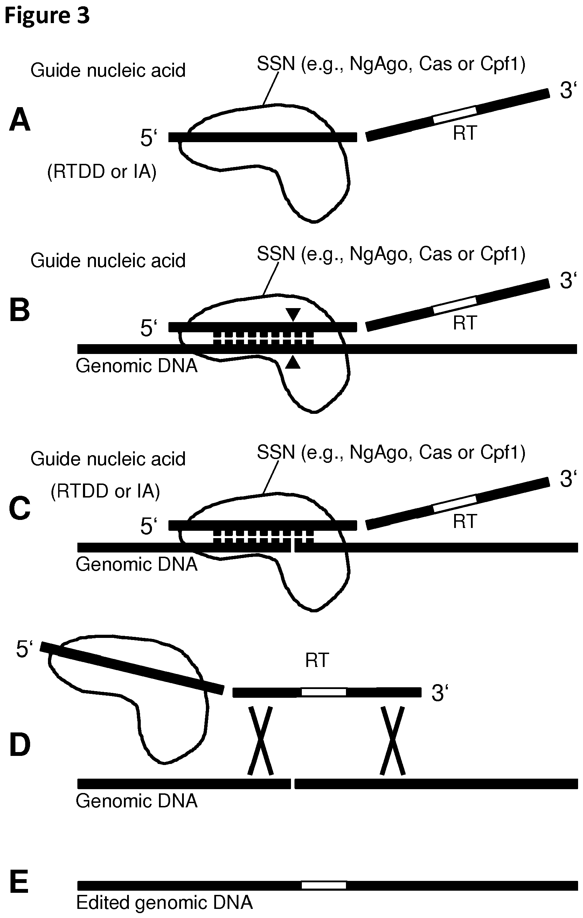

Figure 3 A to E (Fig. 3 A to E) show an example for the stepwise introduction of an edit into a genomic sequence of interest with the site specific nuclease (SSN) nuclease complex disclosed herein, using one embodiment of the covalent association of the RT with the 3'-end of the guide nucleic acid molecule as an example. (A) Schematic of the guide nucleic acid molecule in complex with a SSN, e.g., NgAgo, Cas, including Cas9, CasX or CasY, or Cpf1. (B) Schematic of the complex bound to the target DNA (genomic DNA (gDNA)) and indication of the cutting sites (black triangles). (C) Schematic of the cleaved target DNA. (D) Schematic of the cleaved target DNA released by the SSN and interacting with the repair template (RT) by complementary Watson-Crick base pairing. (E) Schematic of the repaired target site (gDNA) including the edits copied from the RT during homologous recombination. The repair template (RT) portion is shown in white in all Figures.

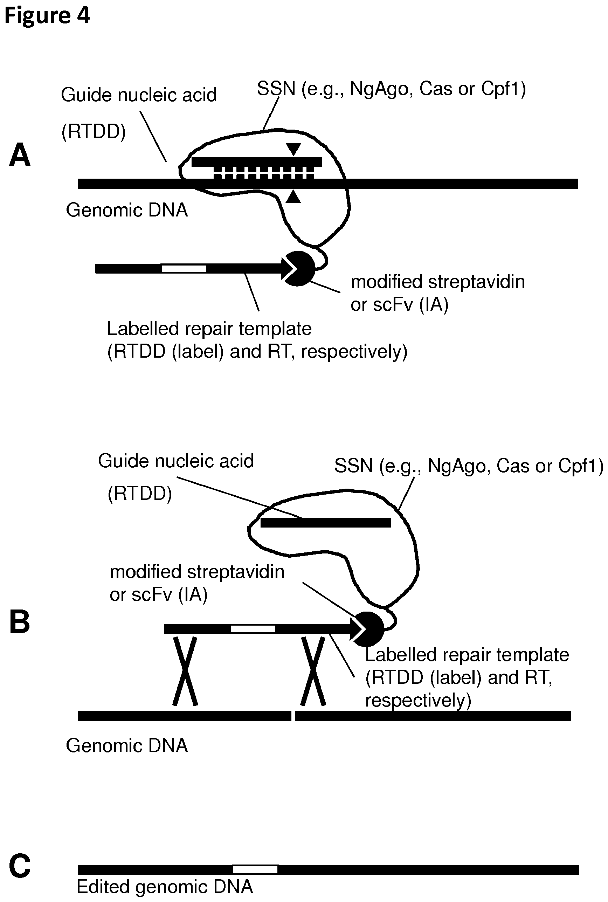

Figure 4 A to C (Fig. 4 A to C) show an example for the design of a fusion protein of a nucleic acid guided endonuclease as SSN and a protein or protein domain as interaction domain (IA) with capacity to directly or indirectly bind a repair template (RT). (A) Schematic of said fusion protein as a complex with the target DNA. (B) Schematic of the complex after the double-strand break was introduced. The nucleic acid guided endonuclease detaches from the target DNA. The fused nucleic acid repair template forms a complex with the target region in a homology based manner. (C) Schematic of the target DNA after the homology-directed repair occurred. Notably, the presented approach uses more than one RTDD to add more precision to the genome engineering complex.

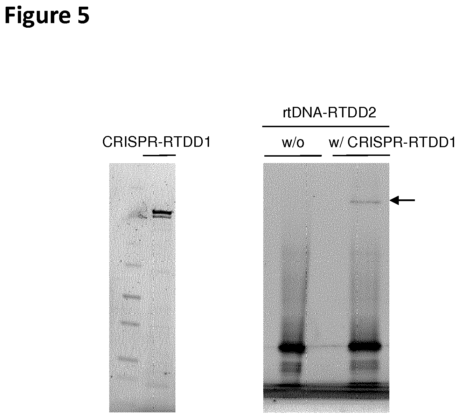

Figure 5 shows in the left panel a purified nuclease (in this case a CRISPR nuclease) that was fused with a RTDD1 and expressed in *E. coli.* It ran on a denaturing, continuous gradient (4-10%) SDS gel and shows the quantity and purity of the protein. The protein was stained in this gel. The right panel shows the tethering. This is a 4% non-denaturing acrylamide gel (Blue Native PAGE) and here the DNA is stained using GelRed. The FAM-labeled (RTDD2-) repair template was either incubated in the nuclease buffer without or with the nuclease-RTDD1 shown on the left. If the protein was present, tethering occurred as seen by DNA being detected at a higher molecular weight level (arrow).

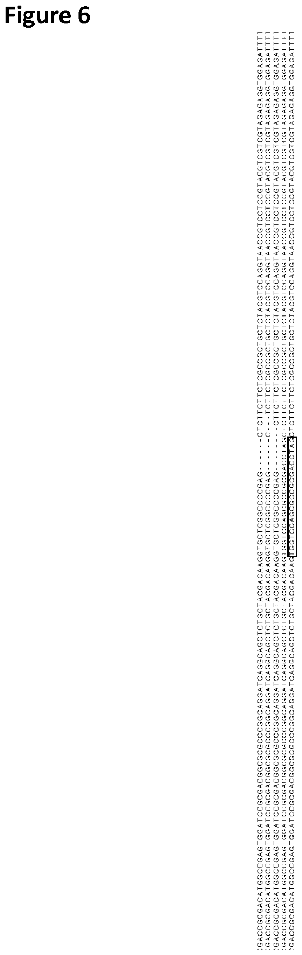

Figure 6 shows in line 1 a part of the wild-type sequence of the target site (full length sequence represents SEQ ID NO: 47), in line 2 and line 3 examples for INDEL occurrence (full length sequences represent SEQ ID NO: 48 and 49), in line 4 the correct HDR event (full length sequence represents SEQ ID NO: 50) and in line 5 shows the repair template (full length sequence represents SEQ ID NO: 51).

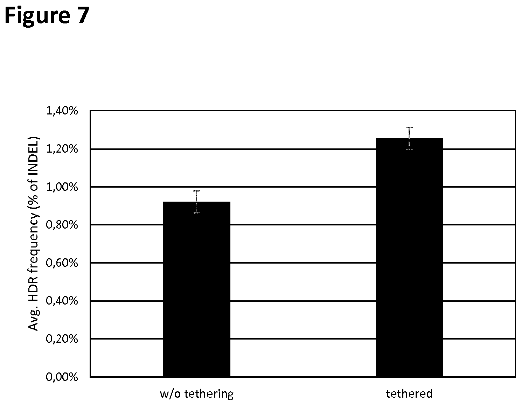

Figure 7 shows comparison of normalized HDR efficiency when the repair template is not (left column) and is tethered to the nuclease (right column).

## Definitions

It must be noted that, as used herein, the singular forms "a" "an" and "the" include plural references unless the context clearly dictates otherwise. For example, reference to a component is intended also to include composition of a plurality of components. References to a composition containing "a" constituent is intended to include other constituents in addition to the one named. In other words, the terms "a" "an" and "the" do not denote a limitation of quantity, but rather denote the presence of "at least one" of the referenced item. It is intended that each term contemplates its broadest meaning as understood by those skilled in the art and includes all technical equivalents which operate in a similar manner to accomplish a similar purpose.

Ranges may be expressed herein as from "about" or "approximately" or "substantially" one particular value and/or to "about" or "approximately" or "substantially" another particular value. When such a range is expressed, other exemplary embodiments include from the one particular value and/or to the other particular value. Further, the term "about" means within an acceptable error range for the particular value as determined by one of ordinary skill in the art, which will depend in part on how the value is measured or determined, i.e., the limitations of the measurement system. For example, "about" can mean within an acceptable standard deviation, per the practice in the art. Alternatively, "about" can mean a range of up to $\pm20\%$, preferably up to $\pm10\%$, more preferably up to $\pm5\%$, and more preferably still up to $\pm1\%$ of a given value. Alternatively, particularly with respect to biological systems or processes, the term can mean within an order of magnitude, preferably within 2-fold, of a value. Where particular values are described in the application and claims, unless otherwise stated, the term "about" is implicit and in this context means within an acceptable error range for the particular value.

By "comprising" or "containing" or "including" is meant that at least the named compound, element, particle, or method step is present in the composition or article or method, but does not exclude the presence of other compounds, materials, particles, method steps, even if the other such compounds, material, particles, method steps have the same function as what is named.

As used herein, "nucleic acid" means a polynucleotide and includes a single- or a double-stranded polymer of deoxyribonucleotide or ribonucleotide bases. Nucleic acids may also include fragments and modified nucleotides. Thus, the terms "polynucleotide", "nucleic acid sequence", "nucleotide sequence" and "nucleic acid fragment" are used interchangeably to denote a polymer of RNA and/or DNA that is single- or double-stranded, optionally containing synthetic, non-natural, or altered nucleotide bases. Nucleotides (usually found in their 5' monophosphate form) are referred to by their single letter designation as follows: "A" for adenosine or deoxyadenosine (for RNA or DNA, respectively), "C" for cytosine or deoxycytosine, "G" for guanosine or deoxyguanosine, "U" for uridine, "T" for deoxythymidine, "R" for purines (A or G), "Y" for pyrimidines (C or T), "K" for G or T, "H" for A or C or T, "I" for inosine, and "N" for any nucleotide. A nucleic acid can comprise nucleotides. A nucleic acid can be exogenous or endogenous to a cell. A nucleic acid can exist in a cell-free environment. A nucleic acid can be a gene or fragment thereof, but the nucleic acid does not necessarily have to encode a gene. A nucleic acid can be DNA A nucleic acid can be RNA. A nucleic acid can comprise one or more analogs (e.g., altered backbone, sugar, or nucleobase). Some nonlimiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, florophores (e.g., rhodamine or flurescein linked to the sugar), thiol containing nucleotides, biotin linked nucleotides, fluorescent base analogs, CpG islands, methyl-7-guanosine, methylated nucleotides, inosine, thiouridine, 20 pseudourdine, dihydrouridine, queuosine, and wyosine. A nucleic acid according to the present invention can be connected by phosphidiester linkages, e.g., as naturally occurring, or by phosphorothioate linkages, or a mixture of both.

The terms "guide RNA", "gRNA" or "single guide RNA" or "sgRNA" are used interchangeably herein and either refer to a synthetic fusion of a CRISPR RNA (crRNA) and a trans-activating crRNA (tracrRNA), or the term refers to a single RNA molecule consisting only of a crRNA and/or a tracrRNA, or the term refers to a gRNA individually comprising a crRNA or a tracrRNA moiety. The tracr and the crRNA moiety thus do not necessarily have to be present on one covalently attached RNA molecule, yet they can also be comprised by two individual RNA molecules, which can associate or can be associated by non-covalent or covalent interaction to provide a gRNA according to the present disclosure. The terms "gDNA" or "sgDNA" or "guide DNA" are used interchangeably herein and either refer to a nucleic acid molecule interacting with an Argonaute nuclease. Both, the gRNAs and gDNAs as disclosed herein are termed "guding nucleic acid(s)" or "guide nucleic acid(s)" due to their capacity to interacting with a site-specific nuclease and to assist in targeting said site-specific nuclease to a genomic target site.

The terms "gene editing", "genome editing" and "genome engineering" are used interchangeably herein and refer to strategies and techniques for the targeted, specific modification of any genetic information or genome of a living organism. As such, the terms comprise gene editing, but also the editing of regions other than gene encoding regions of a genome. It further comprises the editing or engineering of the nuclear (if present) as well as other genetic information of a cell. Furthermore, the terms "genome editing" and "genome engineering" also comprise an epigenetic editing or engineering, i.e., the targeted modification of, e.g., methylation, histone modification or of non-coding RNAs possibly causing heritable changes in gene expression.

The terms "nucleotide" and "nucleic acid" with reference to a sequence or a molecule are used interchangeably herein and refer to a single- or double-stranded DNA or RNA of natural or synthetic origin. The term nucleotide sequence is thus used for any DNA or RNA sequence independent of its length, so that the term comprises any nucleotide sequence comprising at least one nucleotide, but also any kind of larger oligonucleotide or polynucleotide. The term(s) thus refer to natural and/or synthetic deoxyribonucleic acids (DNA) and/or ribonucleic acid (RNA) sequences, which can optionally comprise synthetic nucleic acid analoga. A nucleic acid according to the present disclosure can optionally be codon optimized. Codon optimization implies that the codon usage of a DNA or RNA is adapted to that of a cell or organism of interest to improve the transcription rate of said recombinant nucleic acid in the cell or organism of interest. The skilled person is well aware of the fact that a target nucleic acid can be modified at one position due to the codon degeneracy, whereas this modification will still lead to the same amino acid sequence at that position after translation, which is achieved by codon optimization to take into consideration the species-specific codon usage of a target cell or organism. Nucleic acid sequences according to the present application can carry specific codon optimization for the following non limiting list of organisms: *Hordeum vulgare, Sorghum bicolor, Secale cereale,* Triticale, *Saccharum officinarium, Zea mays, Setaria italic, Oryza sativa, Oryza minuta, Oryza australiensis, Oryza alta, Triticum aestivum, Triticum durum,* Triticale, *Hordeum bulbosum, Brachypodium distachyon, Hordeum marinum, Aegilops tauschii, Malus domestica, Beta vulgaris, Helianthus annuus, Daucus glochidiatus, Daucus pusillus, Daucus muricatus, Daucus carota, Eucalyptus grandis, Erythranthe guttata, Genlisea aurea, Nicotiana sylvestris, Nicotiana tabacum, Nicotiana tomentosiformis, Nicotiana benthamiana, Solanum lycopersicum, Solanum tuberosum, Coffea canephora, Vitis vinifera, Cucumis sativus, Marus notabilis, Arabidopsis thaliana, Arabidopsis lyrata, Arabidopsis arenosa, Crucihimalaya himalaica, Crucihimalaya wallichii, Cardamine flexuosa, Lepidium virginicum, Capsella bursa-pastoris, Olmarabidopsis pumila, Arabis hirsuta, Brassica napus, Brassica oleracea, Brassica rapa, Brassica juncacea, Brassica nigra, Raphanus sativus, Eruca vesicaria sativa, Citrus sinensis, Jatropha curcas, Glycine max, Gossypium ssp., Populus trichocarpa, Mus musculus, Rattus norvegicus* or *Homo sapiens.*

As used herein, "non-native" or "non-naturally occurring" or "artificial" can refer to a nucleic acid or polypeptide sequence, or any other biomolecule like biotin or fluorescein that is not found in a native nucleic acid or protein. Non-native can refer to affinity tags. Non-native can refer to fusions. Non-native can refer to a naturally occurring nucleic acid or polypeptide sequence that comprises mutations, insertions and/or deletions. A non-native sequence may exhibit and/or encode for an activity (e.g., enzymatic activity, methyltransferase activity, acetyltransferase activity, kinase activity, ubiquitinating activity, etc.) that can also be exhibited by the nucleic acid and/or polypeptide sequence to which the non-native sequence is fused. A non-native nucleic acid or polypeptide sequence may be linked to a naturally-occurring nucleic acid or polypeptide sequence (or a variant thereof) by genetic engineering to generate a chimeric nucleic acid and/or polypeptide sequence encoding a chimeric nucleic acid and/or polypeptide. A non-native sequence can refer to a 3' hybridizing extension sequence.

As used herein, "nucleotide" can generally refer to a base-sugar-phosphate combination. A nucleotide can comprise a synthetic nucleotide. A nucleotide can comprise a synthetic nucleotide analog. Nucleotides can be monomeric units of a nucleic acid sequence (e.g., deoxyribonucleic acid (DNA) and ribonucleic acid (RNA)). The term nucleotide can include ribonucleoside triphosphates adenosine triphosphate (ATP), uridine triphosphate (UTP), cytosine triphosphate (CTP), guanosine triphosphate (GTP), inosine triphosphate (ITP) and deoxyribonucleoside triphosphates such as dATP, dCTP, dITP, dUTP, dGTP, dTTP, or derivatives thereof. Such derivatives can include, for example and not limitation, [$\alpha$S]dATP, 7-deaza-dGTP and 7-deaza-dATP, and nucleotide derivatives that confer nuclease resistance on the nucleic acid molecule containing them. The term nucleotide as used herein can refer to dideoxyribonucleoside triphosphates (ddNTPs) and their derivatives. Illustrative examples of dideoxyribonucleoside triphosphates can include, but are not limited to, ddATP, ddCTP, ddGTP, ddITP, and ddTTP. A nucleotide may be unlabeled or detectably labeled by well-known techniques. Labeling can also be carried out with quantum dots. Detectable labels can include, for example, radioactive isotopes, fluorescent labels, chemiluminescent labels, bioluminescent labels and enzyme labels. Fluorescent labels of nucleotides may include but are not limited to fluorescein, 5-carboxyfluorescein (FAM), 2'7'-5 dimethoxy-4'5-dichloro-6-carboxyfluorescein (JOE), rhodamine, 6-carboxyrhodamine (R6G), N,N,N',N'-tetramethyl-6-carboxyrhodamine (TAMRA), 6-carboxy-X-rhodamine (ROX), 4-(4'-dimethylaminophenylazo) benzoic acid (DABCYL), Cascade Blue, Oregon Green, Texas Red, Cyanine and 5-(2'-aminoethyl)aminonaphthalene-I-sulfonic acid (EDANS).

As used herein, "fusion" can refer to a protein and/or nucleic acid comprising one or more non-native sequences (e.g., moieties). A fusion can be at the N-terminal or C-terminal end of the modified protein, or both, or within the molecule as separate domain. For nucleic acid molecules, the fusion molecule can be attached at the 5'- or 3'-end, or at any suitable position in between. A fusion can be a transcriptional and/or translational fusion. A fusion can comprise one or more of the same non-native sequences. A fusion can comprise one or more of different non-native sequences. A fusion can be a chimera. A fusion can comprise a nucleic acid affinity tag. A fusion can comprise a barcode. A fusion can comprise a peptide affinity tag. A fusion can provide for subcellular localization of the Argonaute (e.g., a nuclear localization signal (NLS) for targeting to the nucleus, a mitochondrial localization signal for targeting to the mitochondria, a chloroplast localization signal for targeting to a chloroplast, an 15 endoplasmic reticulum (ER) retention signal, and the like). A fusion can provide a non-native sequence (e.g., affinity tag) that can be used to track or purify. A fusion can be a small molecule such as biotin or a dye such as alexa fluor dyes, Cyanine3 dye, Cyanine5 dye. The fusion can provide for increased or decreased stability. In some embodiments, a fusion can comprise a detectable label, including a moiety that can provide a detectable signal. Suitable detectable labels and/or moieties that can provide a detectable signal can include, but are not limited to, an enzyme, a radioisotope, a member of a specific binding pair; a fluorophore; a fluorescent reporter or fluorescent protein; a quantum dot; and the like. A fusion can comprise a member of a FRET pair, or a fluorophore/quantum dot donor/acceptor pair. A fusion can comprise an enzyme. Suitable enzymes can include, but are not limited to, horse radish peroxidase, luciferase, beta-25 galactosidase, and the like. A fusion can comprise a fluorescent protein. Suitable fluorescent proteins can include, but are not limited to, a green fluorescent protein (GFP), (e.g., a GFP from Aequoria victoria, fluorescent proteins from Anguilla japonica, or a mutant or derivative thereof), a red fluorescent protein, a yellow fluorescent protein, a yellow-green fluorescent protein (e.g., mNeonGreen derived from a tetrameric fluorescent protein from the cephalochordate Branchiostoma lanceolatum) any of a variety of fluorescent and colored proteins. A fusion can comprise a nanoparticle. Suitable nanoparticles can include fluorescent or luminescent nanoparticles, and magnetic nanoparticles, or nanodiamonds, optionally linked to a nanoparticle. Any optical or magnetic property or characteristic of the nanoparticle(s) can be detected. A fusion can comprise a helicase, a nuclease (e.g., FokI), an endonuclease, an exonuclease (e.g., a 5'-exonuclease and/or 3'-exonuclease), a ligase, a nickase, a nuclease-helicase (e.g., Cas3), a DNA methyltransferase (e.g., Dam), or DNA demethylase, a histone methyltransferase, a histone demethylase, an acetylase (including for example and not limitation, a histone acetylase), a deacetylase (including for example and not limitation, a histone deacetylase), a phosphatase, a kinase, a transcription (co-)activator, a transcription (co-)factor, an RNA polymerase subunit, a transcription repressor, a DNA binding protein, a DNA structuring protein, a long noncoding RNA, a DNA repair protein (e.g., a protein involved in repair of either single- and/or double-stranded breaks, e.g., proteins involved in base excision repair, nucleotide excision repair, mismatch repair, NHEJ, HR, microhomology-mediated end joining (MMEJ), and/or alternative non-homologous end-joining (ANHEJ), such as for example and not limitation, HR regulators and HR complex assembly signals), a marker protein, a reporter protein, a fluorescent protein, a ligand binding protein (e.g., mCherry or a heavy metal binding protein), a signal peptide (e.g., Tat-signal sequence), a targeting protein or peptide, a subcellular localization sequence (e.g., nuclear localization sequence, a chloroplast localization sequence), and/or an antibody epitope, or any combination thereof.

The term "catalytically active fragment" as used herein referring to amino acid sequences denotes the core sequence derived from a given template amino acid sequence, or a nucleic acid sequence encoding the same, comprising all or part of the active site of the template sequence with the proviso that the resulting catalytically active fragment still possesses the activity characterizing the template sequence, for which the active site of the native enzyme or a variant thereof is responsible. Said modifications are suitable to generate less bulky amino acid sequences still having the same activity as a template sequence making the catalytically active fragment a more versatile or more stable tool being sterically less demanding.

A "variant" of any site-specific nuclease disclosed herein represents a molecule comprising at least one mutation, deletion or insertion in comparison to the wild-type site-specific nuclease to alter the activity of the wild-type nuclease as naturally occurring. A "variant" can, as nonlimiting example, be a catalytically inactive Cas9 (dCas9), or a site-specific nuclease, which has been modified to function as nickase.

The term "delivery construct" or "delivery vector" as used herein refers to any biological or chemical means used as a cargo for transporting a nucleic acid, including a hybrid nucleic acid comprising RNA and DNA, and/or an amino acid sequence of interest into a target cell, such as a eukaryotic cell. The term delivery construct or vector as used herein thus refers to a means of transport to deliver a genetic or a recombinant construct according to the present disclosure into a target cell, tissue, organ or an organism. A vector can thus comprise nucleic acid sequences, optionally comprising sequences like regulatory sequences or localization sequences for delivery, either directly or indirectly, into a target cell of interest or into a plant target structure in the desired cellular compartment of a plant. A vector can also be used to introduce an amino acid sequence or a ribonucleo-molecular complex into a target cell or target structure. Usually, a vector as used herein can be a plasmid vector. Furthermore, according to certain preferred embodiments according to the present disclosure, a direct introduction of a construct or sequence or complex of interest is conducted. The term direct introduction implies that the desired target cell or target structure containing a DNA target sequence to be modified according to the present disclosure is directly transformed or transduced or transfected into the specific target cell of interest, where the material delivered with the delivery vector will exert its effect. The term indirect introduction implies that the introduction is achieved into a structure, for example, cells of leaves or cells of organs or tissues, which do not themselves represent the actual target cell or structure of interest to be transformed, but those structures serve as basis for the systemic spread and transfer of the vector, preferably comprising a genetic construct according to the present disclosure to the actual target structure, for example, a meristematic cell or tissue, or a stem cell or tissue. In case the term vector is used in the context of transfecting amino acid sequences and/or nucleic sequences, including hybrid nucleic acid sequences, into a target cell the term vector implies suitable agents for peptide or protein transfection, like for example ionic lipid mixtures, cell penetrating peptides (CPPs), or particle bombardment. In the context of the introduction of nucleic acid material, the term vector cannot only imply plasmid vectors but also suitable carrier materials which can serve as basis for the introduction of nucleic acid and/or amino acid sequence delivery into a target cell of interest, for example by means of particle bombardment. Said carrier material comprises, *inter alia,* gold or tungsten particles. Finally, the term vector also implies the use of viral vectors for the introduction of at least one genetic construct according to the present disclosure like, for example, modified viruses for example derived from the following virus strains: adenoviral or adeno-associated viral (AAV) vectors, lentiviral vectors, herpes simplex virus (HSV-1), vaccinia virus, Sendai virus, Sindbis virus, Semliki forest alphaviruses, Epstein-Barr-Virus (EBV), Maize Streak Virus (MSV), Barley Stripe Mosaic Virus (BSMV), Brome Mosaic virus (BMV, accession numbers: RNA 1: X58456; RNA2: X58457; RNA3: X58458), Maize stripe virus (MSpV), Maize rayado fino virus (MYDV), Maize yellow dwarf virus (MYDV), Maize dwarf mosaic virus (MDMV), positive strand RNA viruses of the family *Benyviridae,* e.g., *Beet necrotic yellow vein virus* (accession numbers: RNA 1: NC_003514; RNA2: NC_003515; RNA3: NC_003516; RNA4: NC_003517) or of the family *Bromoviridae,* e.g., viruses of the genus *Alfalfa mosaic virus* (accession numbers: RNA1: NC_001495; RNA2: NC_002024; RNA3: NC_002025) or of the genus *Bromovirus,* e.g., BMV (supra), or of the genus *Cucumovirus,* e.g., *Cucumber mosaic virus* (accession numbers: RNA1: NC_002034; RNA2: NC_002035; RNA3: NC_001440), or of the genus *Oleavirus,* dsDNA viruses of the family *Caulimoviridae,* particularly of the family *Badnavirus* or *Caulimovirus,* e.g., different *Banana streak* viruses (e.g., accession numbers: NC_007002, NC_015507, NC_006955 or NC_003381) or *Cauliflower mosaic virus* (accession number: NC_001497), or viruses of the genus *Cavemovirus, Petuvirus, Rosadnavirus, Solendovirus, Soymovirus* or *Tungrovirus,* positive strand RNA viruses of the family *Closteroviridae,* e.g., of the genus *Ampelovirus, Crinivirus,* e.g., *Lettuce infectious yellows virus* (accession numbers: RNA 1: NC_003617; RNA2: NC_003618) or *Tomato chlorosis virus* (accession numbers: RNA 1: NC_007340; RNA2: NC_007341), *Closterovirus,* e.g., *Beet yellows virus* (accession number: NC_001598), or *Velarivirus,* single-stranded DNA (+/-) viruses of the family *Geminiviridae,* e.g., viruses of the family *Becurtovirus, Begomovirus,* e.g., *Bean golden yellow mosaic virus, Tobacco curly shoot virus, Tobacco mottle leaf curl virus, Tomato chlorotic mottle virus, Tomato dwarf leaf virus, Tomato golden mosaic virus, Tomato leaf curl virus, Tomato mottle virus,* or *Tomato yellow spot virus,* or *Geminiviridae* of the genus *Curtovirus,* e.g., *Beet curly top virus,* or *Geminiviridae* of the genus *Topocuvirus, Turncurtvirus* or *Mastrevirus,* e.g., *Maize streak virus (supra), Tobacco yellow dwarf virus, Wheat dwarf virus,* positive strand RNA viruses of the family *Luteoviridae,* e.g., of the genus *Luteovirus,* e.g., *Barley yellow dwarf virus-PAV* (accession number: NC_004750), or of the genus *Polerovirus,* e.g., *Potato leafroll virus* (accession number: NC_001747), single-stranded DNA viruses of the family *Nanoviridae,* comprising the genus *Nanovirus* or *Babuvirus,* double-stranded RNA viruses of the family *Partiviridae,* comprising *inter alia* the families *Alphapartitivirus, Betapartitivirus* or *Deltapartitivirus,* viroids of the family *Pospiviroidae,* positive strand RNA viruses of the family *Potyviridae,* e.g., comprising the genus *Brambyvirus, Bymovirus, Ipomovirus, Macluravirus, Poacevirus,* e.g., *Triticum mosaic virus* (accession number: NC_012799), or *Potyviridae* of the genus *Potyvirus,* e.g., *Beet mosaic virus* (accession number: NC_005304), *Maize dwarf mosaic virus* (accession number: NC_003377), *Potato virus Y* (accession number: NC_001616), or *Zea mosaic virus* (accession number: NC_018833), or *Potyviridae* of the genus *Tritimovirus,* e.g., *Brome streak mosaic virus* (accession number: NC_003501) or *Wheat streak mosaic virus* (accession number: NC_001886), single-stranded RNA viruses of the family *Pseudoviridae,* e.g., of the genus *Pseudovirus,* or *Sirevirus,* double-stranded RNA viruses of the family *Reoviridae,* e.g., Rice dwarf virus (accession numbers: RNA1: NC_003773; RNA2: NC_003774; RNA3: NC_003772; RNA4: NC_003761; RNAS: NC_003762; RNA6: NC_003763; RNA7: NC_003760; RNAB: NC_003764; RNA9: NC_003765; RNA10: NC_003766; RNA11: NC_003767; RNA 12: NC_003768), positive strand RNA viruses of the family *Tombusviridae,* e.g., comprising the genus *Alphanecrovirus, Aureusvirus, Betanecrovirus, Carmovirus, Dianthovirus, Gallantivirus, Macanavirus, Machlomovirus, Panicovirus, Tombusvirus, Umbravirus* oder *Zeavirus,* e.g., *Maize necrotic streak virus* (accession number: NC_007729), or positive strand RNA viruses of the family *Virgaviridae,* e.g., viruses of the genus *Furovirus, Hordeivirus,* e.g., *Barley stripe mosaic virus* (accession numbers: RNA1: NC_003469; RNA2: NC_003481; RNA3: NC_003478), or of the genus *Pecluvirus, Pomovirus, Tobamovirus* or *Tobravirus,* e.g., *Tobacco rattle virus* (accession numbers: RNA1: NC_003805; RNA2: NC_003811), as well as negative strand RNA viruses of the order *Mononegavirales,* particularly of the family *Rhabdoviridae,* e.g., *Barley yellow striate mosaic virus* (accession number: KM213865) or *Lettuce necrotic yellows virus* (accession number/specimen: NC_007642/ AJ867584), positive strand RNA viruses of the order *Picornavirales,* particularly of the family *Secoviridae,* e.g., of the genus *Comovirus, Fabavirus, Nepovirus, Cheravirus, Sadwavirus, Sequivirus, Torradovirus,* or *Waikavirus,* positive strand RNA viruses of the order *Tymovirales,* particularly of the family *Alphaflexiviridae,* e.g., viruses of the genus *Allexivirus, Lolavirus, Mandarivirus,* or *Potexvirus, Tymovirales,* particularly of the family *Betaflexiviridae,* e.g., viruses of the genus *Capillovirus, Carlavirus, Citrivirus, Foveavirus, Tepovirus,* or *Vitivirus,* positive strand RNA viruses of the order *Tymovirales,* particularly of the family *Tymoviridae,* e.g., viruses of the order *Maculavirus, Marafivirus,* or *Tymovirus,* and bacterial vectors, like for example *Agrobacterium spp.,* like for example *Agrobacterium tumefaciens.* Finally, the term vector also implies suitable chemical transport agents for introducing linear nucleic acid sequences (single- or double-stranded) into a target cell combined with a physical introduction method, including polymeric or lipid-based delivery constructs.

Suitable delivery constructs or vectors thus comprise biological means for delivering nucleotide sequences into a target cell, including viral vectors, *Agrobacterium spp.,* or chemical delivery constructs, including nanoparticles, e.g., mesoporous silica nanoparticles (MSNPs), cationic polymers, including PEI (polyethylenimine) polymer based approaches or polymers like DEAE-dextran, or non-covalent surface attachment of PEI to generate cationic surfaces, lipid or polymeric vesicles, or combinations thereof. Lipid or polymeric vesicles may be selected, for example, from lipids, liposomes, lipid encapsulation systems, nanoparticles, small nucleic acid-lipid particle formulations, polymers, and polymersomes.

The terms "genetic construct" or "recombinant construct" are used herein to refer to a construct comprising, *inter alia,* plasmids or plasmid vectors, cosmids, artificial yeast or bacterial artificial chromosomes (YACs and BACs), phagemides, bacterial phage based vectors, an expression cassette, isolated single-stranded or double-stranded nucleic acid sequences, comprising DNA and RNA sequences, or amino acid sequences, viral vectors, including modified viruses, and a combination or a mixture thereof, for introduction or transformation, transfection or transduction into any prokaryotic or eukaryotic target cell, including a plant, plant cell, tissue, organ or material according to the present disclosure. A recombinant construct according to the present disclosure can comprise an effector domain, either in the form of a nucleic acid or an amino acid sequence, wherein an effector domain represents a molecule, which can exert an effect in a target cell and includes a transgene, an single-stranded or double-stranded RNA molecule, including a guide RNA ((s)gRNA), a miRNA or an siRNA, or an amino acid sequences, including, *inter alia,* an enzyme or a catalytically active fragment thereof, a binding protein, an antibody, a transcription factor, a nuclease, such as a site specific nuclease, and the like. Furthermore, the recombinant construct can comprise regulatory sequences and/or localization sequences. The recombinant construct can be integrated into a vector, including a plasmid vector, and/or it can be present isolated from a vector structure, for example, in the form of a polypeptide sequence or as a non-vector connected single-stranded or double-stranded nucleic acid. After its introduction, e.g., by transformation, the genetic construct can either persist extrachromosomally, i.e., non integrated into the genome of the target cell, for example in the form of a double-stranded or single-stranded DNA, a double-stranded or single-stranded RNA or as an amino acid sequence. Alternatively, the genetic construct, or parts thereof, according to the present disclosure can be stably integrated into the genome of a target cell, including the nuclear genome or further genetic elements of a target cell, including the genome of plastids like mitochondria or chloroplasts. The term plasmid vector as used in this connection refers to a genetic construct originally obtained from a plasmid. A plasmid usually refers to a circular autonomously replicating extrachromosomal element in the form of a double-stranded nucleic acid sequence. In the field of genetic engineering these plasmids are routinely subjected to targeted modifications by inserting, for example, genes encoding a resistance against an antibiotic or an herbicide, a gene encoding a target nucleic acid sequence, a localization sequence, a regulatory sequence, a tag sequence, a marker gene, including an antibiotic marker or a fluorescent marker, and the like. The structural components of the original plasmid, like the origin of replication, are maintained. According to certain embodiments of the present invention, the localization sequence can comprise a nuclear localization sequence, a plastid localization sequence, preferably a mitochondrion localization sequence or a chloroplast localization sequence. Said localization sequences are available to the skilled person in the field of plant biotechnology. A variety of plasmid vectors for use in different target cells of interest is commercially available and the modification thereof is known to the skilled person in the respective field.

The term "genetic(ally) modified" or "genetic manipulation" or "genetic(ally) manipulated" is used in a broad sense herein and means any modification of a nucleic acid sequence or an amino acid sequence, a target cell, tissue, organ or organism, which is accomplished by human intervention, either directly or indirectly, to influence the endogenous genetic material or the transcriptome or the proteome of a target cell, tissue, organ or organism to modify it in a purposive way so that it differs from its state as found without human intervention, whereas the term genome editing specifically refers to a targeted manipulation of the genome of a target cell. The human intervention can either take place *in vitro* or *in vivo,* or both. Further modifications can be included, for example, one or more point mutation(s), e.g., for targeted protein engineering or for codon optimization, deletion(s), and one or more insertion(s) or deletion(s) of at least one nucleic acid or amino acid molecule (including also homologous recombination), modification of a nucleic acid or an amino acid sequence, or a combination thereof. The terms shall also comprise a nucleic acid molecule or an amino acid molecule or a host cell or an organism, including a plant or a plant material thereof which is/are similar to a comparable sequence, organism or material as occurring in nature, but which have been constructed by at least one step of purposive manipulation.

A "targeted genetic manipulation" or "targeted" or "site-directed" gene editing or genome editing as used herein is thus the result of a "genetic manipulation", which is effected in a targeted way, i.e., at least one specific position in a target cell and under the specific suitable circumstances to achieve a desired effect in at least one cell, such as a plant cell, to be manipulated.

The term "transgenic" as used according to the present disclosure refers to an animal, an animal cell, tissue or organ, a plant, plant cell, tissue, organ or material which comprises a gene or a genetic construct, comprising a transgene that has been transferred into the plant, the plant cell, tissue organ or material by natural means or by means of genetic engineering techniques from another organism. The term "transgene" comprises a nucleic acid sequence, including DNA or RNA or a combination or mixture thereof. Therefore, the term "transgene" is not restricted to a sequence commonly identified as gene, i.e., a sequence encoding protein. It can also refer, for example, to a non-protein encoding DNA or RNA sequence. Therefore, the term transgenic generally implies that the respective nucleic acid introduced into a cell of interest is not naturally present in the respective target prokaryotic or eukaryotic cell, including a bacterial cell, a yeast cell, a fungal cell, an animal or animal cell, a plant, plant cell, tissue, organ or material. The terms transgene or transgenic as used herein thus refer to a nucleic acid sequence or an amino acid sequence that is taken from the genome of one organism, or produced synthetically, and which is then introduced into another organism, in a transient or a stable way, by artificial techniques of molecular biology, genetics and the like.

The term "plant" or "plant cell" as used herein refers to a plant organism, a plant organ, differentiated and undifferentiated plant tissues, plant cells, seeds, and derivatives and progeny thereof. Plant cells include without limitation, for example, cells from seeds, from mature and immature embryos, meristematic tissues, seedlings, callus tissues in different differentiation states, leaves, flowers, roots, shoots, gametophytes, sporophytes, pollen, pollen tubes and microspores, protoplasts, macroalgae and microalgae. The different plant cells can either be haploid, diploid, tetraploid, hexaploid or polyploid.

"Subject", as used herein, may mean either a human or non-human animal. The term includes, but is not limited to, mammals (e.g., humans, other primates, pigs, rodents (e.g., mice and rats or hamsters), rabbits, guinea pigs, cows, horses, cats, dogs, sheep, and goats). In an embodiment, the subject is a human being.

"Treat", "treating" and "treatment", as used herein, generally mean obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment" as used herein covers any treatment of a disease or symptom in a mammal, and includes: (a) preventing the disease or symptom from occurring in a subject which may be predisposed to acquiring the disease or symptom but has not yet been diagnosed as having it; (b) inhibiting the disease or symptom, i.e., arresting its development; or (c) relieving the disease, i.e., causing regression of the disease. The therapeutic agent may be administered before, during or after the onset of disease or injury. The treatment of ongoing disease, where the treatment stabilizes or reduces the undesirable clinical symptoms of the patient, is of particular interest. Such treatment is desirably performed prior to complete loss of function in the affected tissues. The subject therapy will desirably be administered during the symptomatic stage of the disease, and in some cases after the symptomatic stage of the disease.

A "plant material" as used herein refers to any material which can be obtained from a plant during any developmental stage. The plant material can be obtained either *in planta* or from an *in vitro* culture of the plant or a plant tissue or organ thereof. The term thus comprises plant cells, tissues and organs as well as developed plant structures as well as sub-cellular components like nucleic acids, polypeptides and all chemical plant substances or metabolites which can be found within a plant cell or compartment and/or which can be produced by the plant, or which can be obtained from an extract of any plant cell, tissue or a plant in any developmental stage. The term also comprises a derivative of the plant material, e.g., a protoplast, derived from at least one plant cell comprised by the plant material. The term therefore also comprises meristematic cells or a meristematic tissue of a plant.

As used herein, the terms "mutation" and "modification" are used interchangeably to refer to a deletion, insertion, addition, substitution, edit, strand break, and/or introduction of an adduct in the context of nucleic acid manipulation *in vivo* or *in vitro*. A deletion is defined as a change in a nucleic acid sequence in which one or more nucleotides is absent. An insertion or addition is that change in a nucleic acid sequence which has resulted in the addition of one or more nucleotides. A "substitution" or edit results from the replacement of one or more nucleotides by a molecule which is a different molecule from the replaced one or more nucleotide(s). For example, a nucleic acid may be replaced by a different nucleic acid as exemplified by replacement of a thymine by a cytosine, adenine, guanine, or uridine. Pyrimidine to pyrimidine (e.g., C to Tor T to C nucleotide substitutions) or purine to purine (e.g., G to A or A to G nucleotide substitutions) are termed transitions, whereas pyrimidine to purine or purine to pyrimidine (e.g., G to T or G to C or A to T or A to C) are termed transversions. Alternatively, a nucleic acid may be replaced by a modified nucleic acid as exemplified by replacement of a thymine by thymine glycol. Mutations may result in a mismatch. The term mismatch refers to a non-covalent interaction between two nucleic acids, each nucleic acid residing on a different nucleotide sequence or nucleic acid molecule, which does not follow the base-pairing rules. For example, for the partially complementary sequences 5'-AGT-3' and 5'-AAT-3', a G-A mismatch (a transition) is present.

The term "strand break" when made in reference to a double-stranded nucleic acid sequence, e.g., a genomic sequence as DNA target sequence, includes a single-strand break and/or a double-strand break. A single-strand break (a nick) refers to an interruption in one of the two strands of the double-stranded nucleic acid sequence. This is in contrast to a double-strand break which refers to an interruption in both strands of the double-stranded nucleic acid sequence. Strand breaks according to the present disclosure may be introduced into a double-stranded nucleic acid sequence by enzymatic incision at a nucleic acid base position of interest using a suitable endonuclease, including a CRISPR endonuclease or a variant thereof, where the variant can be a mutated or truncated version of the wild-type protein or endonuclease, which still can exert the enzymatic function of the wild-type protein.

"Complementary" or "complementarity" as used herein describes the relationship between two DNA, two RNA, or, regarding hybrid sequences according to the present disclosure, between an RNA and a DNA nucleic acid region. Defined by the nucleobases of the DNA or RNA, two nucleic acid regions can hybridize to each other in accordance with the lock-and-key model. To this end the principles of Watson-Crick base pairing have the basis adenine and thymine/uracil as well as guanine and cytosine, respectively, as complementary bases apply. Furthermore, also non-Watson-Crick pairing, like reverse-Watson-Crick, Hoogsteen, reverse-Hoogsteen and Wobble pairing are comprised by the term "complementary" as used herein as long as the respective base pairs can build hydrogen bonding to each other, i.e., two different nucleic acid strands can hybridize to each other based on said complementarity. Perfect complementarity in the sense of two sequence stretches aligning 100% to each other over a given length is not required, as the skilled person is aware of the fact that nucleic acid hybridization is impacted by such factors as the degree and length of complementarity between the nucleic acids, stringency of the conditions involved, the Tm of the formed hybrid, and the G:C ratio within the nucleic acids, and so on. Furthermore, sterical factors can influence the fact whether two sequences, even though not 100% complementary to each other, will hybridize. Therefore, two complementary nucleic acid sequences according to the present disclosure can have at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence homology or complementarity to each other and can still hybridize to each other at about medium stringency conditions. "Medium" stringency conditions refer to 0.165-0.330 M NaCl in a temperature range from 20 to 29°C below Tm, wherein Tm is defined as the Tm for a DNA sequence can be estimated via the commonly-used calculation:

$$Tm = 81.5 + 16.6\log_{10}([Na+]/1.0 + 0.7[Na+]) + 0.41(\%[G+C]) - (500/n) - P - F,$$

wherein Tm = melting temperature in °C, $[Na^+]$ = molar concentration of sodium ions, %[G+C] = percent of G+C bases in DNA sequence, n = length of DNA sequence in bases P = temperature correction for % mismatched base pairs (~1°C per 1% mismatch), and F = correction for formamide concentration (= 0.63°C per 1% [formamide]).

The term "transient introduction" as used herein refers to the transient introduction of at least one nucleic acid sequence according to the present disclosure, preferably incorporated into a delivery vector or into a recombinant construct, with or without the help of a delivery vector, into a target structure, for example, a plant cell, wherein the at least one nucleic acid sequence is introduced under suitable reaction conditions so that no integration of the at least one nucleic acid sequence into the endogenous nucleic acid material of a target structure, the genome as a whole, occurs, so that the at least one nucleic acid sequence will not be integrated into the endogenous DNA of the target cell. As a consequence, in the case of transient introduction, the introduced genetic construct will not be inherited to a progeny of the target structure, for example a prokaryotic, an animal or a plant cell. The at least one nucleic acid sequence or the products resulting from transcription or translation thereof are only present temporarily, i.e., in a transient way, in constitutive or inducible form, and thus can only be active in the target cell for exerting their effect for a limited time. Therefore, the at least one nucleic acid sequence introduced via transient introduction will not be heritable to the progeny of a cell. The effect which a nucleic acid sequence introduced in a transient way can, however, potentially be inherited to the progeny of the target cell.

The term "stable integration" or "stably integrated" as used herein, refers to the stable integration of at least one nucleic acid sequence according to the present disclosure, preferably incorporated into a delivery vector or into a recombinant construct. The integration can either take place into the nuclear genome of a target cell or any other genomic extra-nuclear material within a eukaryotic cell compartment of interest, e.g., a mitochondrium or a plant cell plastid. A stably integrated at least one recombinant construct will thus be heritable to the progeny of a thus modified target cell. Depending on the nature of the genetic construct, all or part of the genetic construct will be stably integrated, as the genetic construct may comprise several regions of interest comprising a target region to be stably integrated as well as further regions, *inter alia,* needed for the transport, delivery, maintenance, and the correct localization of the genetic construct within a plant cell, which regions, however, will not themselves be integrated, but serve as cargo for the region of interest to be stably integrated as it is known to the skilled person. The stable integration of at least one genetic construct according to the present disclosure into at least one hematopoietic or meristematic cell or tissue will consequently lead to the inheritance of the thus modified genomic region of the target structure, i.e., a DNA target region, to the progeny of the modified cell through all developmental stages of said at least one hematopoietic or meristematic cell, which can be favorable for approaches, where a targeted genetic modification in and the yield of the final cell type resulting from the differentiation and development of the at least one hematopoietic meristematic cell is desired. Achieving, for example, a stable integration into at least one meristematic cell of the immature inflorescence of a plant can thus lead to the stable inheritance of the introduced genetic feature into the gamete of the pollen or of the ovule developmentally resulting from the at least one meristematic cell of the immature inflorescence. Stable integration into at least one pluripotent hematopoietic cell or any pluripotent or multipotent cell will likewise lead to stable inheritance of the introduced genetic feature.

The term "particle bombardment" as used herein, also named biolistic transfection or microparticle-mediated gene transfer, refers to a physical delivery method for transferring a coated microparticle or nanoparticle comprising a nucleic acid or a genetic construct of interest into a target cell or tissue. The micro- or nanoparticle functions as projectile and is fired on the target structure of interest under high pressure using a suitable device, often called "gene-gun". The transformation via particle bombardment uses a microprojectile of metal covered with the gene of interest, which is then shot onto the target cells using an equipment known as "gene-gun" (Sandford et al. 1987) at high velocity fast enough to penetrate the cell wall of a target tissue, but not harsh enough to cause cell death. For protoplasts, which have their cell wall entirely removed, the conditions are different logically. The precipitated nucleic acid or the genetic construct on the at least one microprojectile is released into the cell after bombardment, and integrated into the genome or expressed transiently according to the definition given above. The acceleration of microprojectiles is accomplished by a high voltage electrical discharge or compressed gas (helium). Concerning the metal particles used it is mandatory that they are non-toxic, non-reactive, and that they have a smaller diameter than the target cell. The most commonly used are gold or tungsten. There is plenty of information publicly available from the manufacturers and providers of gene-guns and associated system concerning their general use.

The term "derivative" or "descendant" or "progeny" as used herein in the context of a prokaryotic or a eukaryotic cell, such as an animal cell and/or a plant or plant cell or plant material according to the present disclosure relates to the descendants of such a cell or material which result from natural reproductive propagation including sexual and asexual propagation. It is well known to the person having skill in the art that said propagation can lead to the introduction of mutations into the genome of an organism resulting from natural phenomena which results in a descendant or progeny, which is genomically different to the parental organism or cell, however, still belongs to the same genus/species and possesses mostly the same characteristics as the parental recombinant host cell. Such derivatives or descendants or progeny resulting from natural phenomena during reproduction or regeneration are thus comprised by the term of the present disclosure. Furthermore, the term "derivative" can imply, in the context of a substance or molecule rather than referring to a cell or organism, directly or by means of modification indirectly obtained from another. This might imply a nucleic acid sequence derived from a cell or a plant metabolite obtained from a cell or material. These terms, therefore, do not refer to any arbitrary derivative, descendant or progenitor, but rather to a derivative, or descendant or progenitor phylogenetically associated with, i.e., based on, a parent cell or virus or a molecule thereof, whereas this relationship between the derivative, descendant or progenitor and the "parent" is clearly inferable by a person skilled in the art.

Furthermore, the terms "derived", "derived from", or "derivative" as used herein in the context of a biological sequence (nucleic acid or amino acid) or molecule or complex imply that the respective sequence is based on a reference sequence, for example from the sequence listing, or a database accession number, or the respective scaffold structure, i.e., originating from said sequence, whereas the reference sequence can comprise more sequences, e.g., the whole genome or a full polyprotein encoding sequence, of a virus, whereas the sequence "derived from" the native sequence may only comprise one isolated fragment thereof, or a coherent fragment thereof. In this context, a cDNA molecule or an RNA can be said to be "derived from" a DNA sequence serving as molecular template. The skilled person can thus easily define a sequence "derived from" a reference sequence, which will, by sequence alignment on DNA or amino acid level, have a high identity to the respective reference sequence and which will have coherent stretches of DNA/amino acids in common with the respective reference sequence (>75% query identity for a given length of the molecule aligned provided that the derived sequence is the query and the reference sequence represents the subject during a sequence alignment). The skilled person can thus clone the respective sequences based on the disclosure provided herein by means of polymerase chain reactions and the like into a suitable vector system of interest, or use a sequence as vector scaffold. The term "derived from" is thus no arbitrary sequence, but a sequence corresponding to a reference sequence it is derived from, whereas certain differences, e.g., certain mutations naturally occurring during replication of a recombinant construct within a host cell, cannot be excluded and are thus comprised by the term "derived from". Furthermore, several sequence stretches from a parent sequence can be concatenated in a sequence derived from the parent. The different stretches will have high (preferably more than 90%) or even 100% homology to the parent sequence. The skilled person is well aware of the fact that a sequence of the artificial molecular complexes according to the present disclosure when provided or partially provided as nucleic acid sequence will then be transcribed and optionally translated *in two* and will possibly be further digested and/or processed within a host cell (cleavage of signal peptides, endogenous biotinylation etc.) so that the term "derived from" indicates a correlation to the sequence originally used according to the disclosure of the present invention.

The term "target region", "target site", "target structure", "target construct", "target nucleic acid" or "target cell/tissue/organism", or "DNA target region" as used herein refers to a target which can be any genomic region within any compartment of a target cell.

The term "regulatory sequence" as used herein refers to a nucleic acid or an amino acid sequence, which can direct the transcription and/or translation and/or modification of a nucleic acid sequence of interest.

The terms "protein", "amino acid" or "polypeptide" are used interchangeably herein and refer to an amino acid sequence having a catalytic enzymatic function or a structural or a functional effect. The term "amino acid" or "amino acid sequence" or "amino acid molecule" comprises any natural or chemically synthesized protein, peptide, polypeptide and enzyme or a modified protein, peptide, polypeptide and enzyme, wherein the term "modified" comprises any chemical or enzymatic modification of the protein, peptide, polypeptide and enzyme, including truncations of a wild-type sequence to a shorter, yet still active portion.

In accordance with the present disclosure there may be employed conventional molecular biology, microbiology, and recombinant DNA techniques within the skill of the art. Such techniques are explained fully in the literature. See, e.g., Sambrook, Fritsch & Maniatis, Molecular Cloning: A Laboratory Manual, Second Edition (1989) Cold Spring Harbor Laboratory Press, Cold Spring Harbor, New York (herein "Sambrook *et al.,* 1989"); DNA Cloning: A Practical Approach, Volumes I and II (D.N. Glover ed. 1985); Oligonucleotide Synthesis (M.J. Gait ed. 1984); Nucleic Acid Hybridization (B.D. Hames & SJ. Higgins eds. (1985); Transcription and Translation (B.D. Hames & S.J. Higgins, eds. (1984); Animal Cell Culture (RI. Freshney, ed. (1986); Immobilized Cells and Enzymes (IRL Press, (1986); B. Perbal, A Practical Guide To Molecular Cloning (1984); F.M. Ausubel et al. (eds.), Current Protocols in Molecular Biology, John Wiley & Sons, Inc. (1994); among others.

Whenever the present disclosure relates to the percentage of the homology or identity of nucleic acid or amino acid sequences these values define those as obtained by using the EMBOSS Water Pairwise Sequence Alignments (nucleotide) programme (www.ebi.ac.uk/Tools/psa/ emboss_water/nucleotide.html) nucleic acids or the EMBOSS Water Pairwise Sequence Alignments (protein) programme (www.ebi.ac.uk/Tools/psa/emboss_water/) for amino acid sequences. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5.

## Detailed Description

The present invention is defined by the subject-matter of the attached set of claims.

According to the first aspect of the present disclosure, there is provided an artificial molecular complex, comprising (a) at least one site-specific nuclease (SSN) or a catalytically active fragment thereof, or a nucleic acid sequence encoding the same, and directly interacting therewith (b) at least one repair template docking domain (RTDD), or a nucleic acid sequence encoding the same, wherein the repair template docking domain is configured to directly interact with at least one repair template nucleic acid sequence (RT); (c) optionally comprising at least one interaction domain (IA), or a nucleic acid sequence encoding the same, wherein the at least one interaction domain is directly interacting with the at least one site-specific nuclease or the catalytically active fragment thereof, and wherein the at least one interaction domain is configured to provides at least one of the functionalities selected from the group consisting of (i) interaction with the at least one repair template docking domain; and/or (ii) interaction with the at least one repair template nucleic acid sequence; and/or (iii) sequence-specific interaction with genomic DNA; wherein the at least one repair template nucleic acid sequence comprises at least one portion being complementary to at least one genomic complementarity sequence, and wherein the at least one repair template nucleic acid sequence is configured to mediate repair of a DNA target sequence.

The present invention relies on a site-specific nuclease (SSN). This nuclease is characterized in having nuclease function and a DNA-recognition function. The DNA-recognition function may be intrinsic to the nuclease in the form of a domain mediating DNA recognition or binding, or it may be assisted by additional guiding molecules, e.g., for nucleic acid guided CRISPR (RNA guided) or Argonaute (DNA guided) nucleases, but the present invention is not restricted to the use of the aforementioned nucleic acid guided nucleases and thus increases the scope of application of targeted genome engineering to any non-CRISPR or Argonaute site-specific nucleases. Another part of the artificial system according to the present disclosure is at least one repair template nucleic acid sequence (RT), as the products and methods of the present disclosure mainly focus on making a RT physically available at a site of a double-strand break induced by a SSN. Furthermore, the present invention relies on a repair template docking domain (RTDD) as part of an optimized molecular system. This RTDD fulfils the function of directly or indirectly bringing the SSN and at least one RT into close contact to allow efficient and targeted genome engineering.

The RTDD of the present disclosure is thus covalently or non-covalently associated with the RT, i.e., it is directly interacting with the RT on a molecular level. Simultaneously, the RTDD is directly interacting with the at least one SSN and thus represents the linking molecule or domain between the SSN and the RT. For the RTDD, there are several possible configurations. In one embodiment the RTDD is directly associated with the SSN. For example, if the SSN is a CRISPR nuclease, the RTDD can be a gRNA, or if the SSN is an Argonaute nuclease, the RTDD can be a gDNA. In another embodiment, the RTDD can be part of the SSN itself, or it can be part of the RT, the RTDD representing a specific portion of the RT or the SSN. In these embodiments, the SSN can comprise a domain as part of its amino acid sequence, which can interact with an aptamer carrying a RT. Therefore, in certain embodiments, there is no separate interaction domain, as the site-specific nuclease itself comprises a domain for interaction with a RTDD. The RTDD can thus be an aptamer associated with the RT nucleic acid sequence, wherein the aptamer is recognized and can thus specifically interact with either the SSN and/or an additional interaction domain.

Furthermore, covalent and non-covalent interactions between the components of the artificial molecular complex are envisaged according to the present disclosure.

In a further embodiment of the present disclosure, the artificial molecular complex comprises an additional interaction domain (IA). In this configuration, the RTDD can be associated with the additional interaction domain for certain embodiments. The interaction domain is directly interacting, i.e., physically associated, either covalently as fusion molecule or non-covalently, with the SSN and provides additional functionality to the molecular complex. The interaction domain can be a protein domain comprising DNA recognition/binding functions, i.e., it may be a domain which is capable of interacting with a genomic DNA target site in a site-specific manner, or the interaction domain can be specifically configured to interact with a RTDD and/or the RT. For example, the interaction domain can comprise intrinsic DNA recognition and binding function without having nuclease function itself to specifically interact with a genomic DNA. In another embodiment, the interaction domain can function as highly specific interaction partner for a RTDD associated with a RT as further detailed below. By adding this additional DNA recognition or RTDD interaction functionality to the molecular complex by adding an interaction domain, there is provided another level of specificity to genome engineering in addition to the mere functionality of a SSN alone.

Ultimately, particularly the RTDD directly interacting with a RT and further the interaction domains detailed herein below provide a versatile toolkit to (i) bring a RT into close contact with a SSN of interest and thus into close proximity of the double-strand break induced by the at least one SSN to provide a molecular system in the form of an artificial molecular complex having (ii) a superior targeting range and higher precision suitable for a variety of custom-made genome engineering approaches in eukaryotes and prokaryotes to achieve optimized results for genome engineering, metabolic engineering, trait development in plants and for therapeutic applications.

The various aspects and embodiments of the present disclosure thus all rely on the provision of a suitable double-strand break inducing enzyme, or two nickases, as SSN as well as a suitably designed repair template nucleic acid sequence (RT), wherein the gist of the present disclosure is the fact that the SSN and the RT are brought into close proximity to direct a genome engineering event in a targeted way.

In one embodiment of the present disclosure, at least one RTDD is a CRISPR gRNA or gDNA and it is directly interacting with or associated with a repair template to build a hybrid nucleic acid sequence of RNA-DNA or DNA from the RTDD and DNA from the RT.

An "artificial molecular complex" according to the present disclosure thus represents a complex comprising at least one amino acid component, i.e., a SSN and optionally an interaction domain, a RTDD and a nucleic acid-base repair template (RT). In the assembled state, the complex will usually comprise at least one amino acid (protein) comprising component, i.e., at least one SSN, and a nucleic acid comprising component, i.e., the RT. The at least one RTDD and optionally the at least one interaction domain may also comprise amino acids and/or nucleic acids as building blocks, yet due to the functions of said components within the molecular complex, a greater spectrum of molecules, including synthetic building blocks or combinations of different biomolecules and/or synthetic molecules is possible.

The artificial molecular complex according to the present disclosure thus overcomes the disadvantage of oligonucleotide (RT)-enzyme (SSN) conjugates that they cannot self-assemble *in vivo,* thereby severely limiting their usefulness for genome editing *in vivo* by adding at least one further interaction mediating domain, i.e., a RTDD and optionally an IA guaranteeing a tight association of the RT and the SSN and a perfect assembly of the molecular complex *in vivo,* or in general under physiological conditions, *in vivo* and *in vitro* when working with at least one intact cell carrying a genomic target DNA (genomic, including coding and non-coding regions, including nuclear, plastid and episomal target DNA and epigenetic target sites) of interest to be modified.

In one embodiment of the present disclosure, the artificial molecular complex can be provided and assembled fully *in vivo,* e.g., by providing the necessary constructs to synthesize and subsequently assemble the complex within a host cell. In another embodiment, the artificial molecular complex can be provided as *ex vivo* assembled molecular complex, which is subsequently introduced into a host cell of interest *in vivo,* or which is brought into contact with a genomic target molecule of interest *in vitro.* In yet a further embodiment, parts of the artificial molecular complex can be produced *ex vivo* and parts can be produced *in vivo,* e.g., after introduction of a suitable delivery vector carrying a plasmid for the transcription and/or expression of a component of the artificial molecular complex, and the final artificial molecular complex exerting its function will then assemble *in vivo* based on the intrinsic recognition function mediated by the RTDD.

An "interaction" or "direct interaction" between any components of the artificial molecular complex according to the present disclosure thus implies any covalent or non-covalent interaction or linkage between two components of the artificial molecular complex. A covalent linkage, on nucleic acid level, might thus imply a phosphodiester or a phosphorothioate linkage between nucleotides of a nucleic acid molecule. Furthermore, a covalent linkage can be a disulfide bridge between an amino acid and another amino acid and/or a modified nucleic acid molecule, yet any naturally occurring or artificial covalent linkage can be envisaged according to the present disclosure. Non-covalent interactions comprise electrostatic interactions, including ionic, hydrogen bonding or halogen bonding, van-der-Waals forces, including dipole-dipole, dipole-induced dipole, London dispersion forces, $\pi$-effects and hydrophobic effects. Notably, more than one interaction type can be present within the components of the artificial molecular complex according to the present disclosure. For example, the SSN, e.g., a CRISPR nuclease, might interact via non-covalent interaction(s) with a gRNA as RTDD. The RTDD might be covalently linked to a repair template RT. In another embodiment, an Argonaute fusion protein as SSN can be covalently fused to a single-chain variable antibody fragment as interaction domain (IA). The IA can, *inter alia,* be specific for fluorescein and can thus non-covalently interact with the RTDD fluorescein. Fluorescein and such labeled repair template nucleic acid RT can be provided as synthetic covalent fusion. In another embodiment, the association of the different components is mediated by non-covalent interactions, e.g., by a leucin-zipper recognition of a DNA target sequence and/or an aptamer (nucleic acid or amino acid based) interacting with either the SSN or the IA. In one embodiment, the RTDD can be an aptamer, for example a sequence providing the aptamer function in the repair template. In another embodiment, an extension of a guide nucleic acid allowing hybridization with the repair template can function as the at least one RTDD. If defining a guide nucleic acid as such as RTDD, such an embodiment uses more than one RTDD. In yet a further embodiment, the 3'- or 5'-end of the guide nucleic acid used for ligation with the repair template can be specifically configured to function as RTDD.

According to the present disclosure, the different components of the artificial molecular complex can comprise naturally occurring and/or synthetic artificial building blocks.

A site-specific nuclease (SSN) according to the various embodiments of the present disclosure, or the nucleic acid sequence encoding the same, can thus be any naturally occurring or engineered nuclease which is able to recognize and cleave DNA in a site-specific manner. As many SSNs will have a high number of potential cleavage sites within a genome of an organism or virus, such SSNs with a defined cleavage pattern, or designer SSNs with custom-made cleavage patterns are desirable. SSNs thus include site-specific nucleases for genome-editing techniques such as designer zinc fingers, transcription activator-like effectors (TALEs), (homing) meganucleases, CRISPR system derived nucleases, including Cas or Cpf1 nucleases, or Argonaute nucleases as well as rare cutting endonucleases, or two site-specific nicking endonucleases, including a class IIS restriction endonuclease, including FokI or a variant thereof, or two site-specific nicking endonucleases, or a variant or a catalytically active fragment thereof, or any variant or a catalytically active fragment of the aforementioned SSNs. Therefore, according to the present disclosure, more than one SSN, or a nucleic acid sequence encoding the same, can be present whereas the molecules in sum are able to induce a targeted DNA double-strand break, or two consecutive single-strand breaks at a DNA target sequence.

The "DNA target sequence" according to the present invention/disclosure can be any region within a double-stranded DNA, genomic or plasmid-based, where a targeted DNA break is induced and is subsequently repaired with the help of the repair template (RT) according to the present invention/disclosure. Even though a "DNA target sequence" originates from an endogenous sequence, the editing or engineering of said sequence can be performed *in vitro* by presenting the relevant sequence on a molecule comprising the genomic DNA, preferably on a plasmid. In such embodiments, the target locus of interest may be comprised in a DNA molecule within a cell. The cell may be a prokaryotic cell or a eukaryotic cell, or a viral genome on a plasmid within a prokaryotic cell or a eukaryotic host cell used for propagation of the virus. The cell may be a mammalian cell. The mammalian cell may be a non-human primate, bovine, porcine, rodent or mouse cell. The cell may be a non-mammalian eukaryotic cell such as poultry, fish or shrimp. The cell may also be a plant cell. The plant cell may be of a crop plant such as cassava, corn, sorghum, wheat, soybean, cotton, sugar beet or rice. The plant cell may also be of an algae, tree or vegetable. The modification introduced to the cell by the present invention may be such that the cell and progeny of the cell are altered for improved production of biologic products such as an antibody, starch, alcohol or other desired cellular output. The modification introduced to the cell by the present invention may be such that the cell and progeny of the cell include an alteration that changes the biologic product produced. In another embodiment, the "DNA target sequence" may be an epigenomic locus of interest.

A "genomic complementarity sequence" according to the present invention refers to that sequence portion of a RT according to the present disclosure can align to by means of complementary base pairing. The "DNA target sequence" and the "genomic complementarity sequence" can thus be overlapping or even the same, but for certain embodiments, said sequences can be different, for example in case that the at least one SSN will have a cutting site upstream or downstream of the "genomic complementarity sequence" portion of the RT.

In any of the described embodiments, the strand break may be a double-strand break, or it may be two single-strand breaks.

In certain embodiments, the SSN component and optionally the IA component of the artificial molecular complex will be delivered to a host cell or to an assay system comprising a genomic region of interest to be modified via a protein co-delivery with the RTDD tagged or associated repair template oligonucleotide in one embodiment, or in another embodiment as a plasmid-based expression of the fusion protein and the subsequent exposure to the RTDD tagged repair template. An additional RTDD can be co-delivered in case more than one RTDD is envisaged, for example, one RTDD being a guide nucleic acid molecule and another RTDD being a molecule, e.g., biotin or a marker, for example, fluorescein, associated with the RT. Plasmid- or vector based approaches according to the present invention also include those of a stable expressor line of the SSN and/or the IA and/or a fusion protein thereof.

In one embodiment of the present disclosure, the artificial molecular complex comprises two SSN molecules, one SSN being an active nuclease, and the other SSN being a catalytically inactive nuclease-deficient molecule, wherein the inactive SSN will function as interaction partner for a RTDD/RT. Said configuration of the artificial molecular complex can enhance specificity for certain DNA target sequences of interest.

In another embodiment of the present disclosure, a fusion protein or a non-covalently associated active Cpf1 and an inactive dCas9 as interaction domain can be provided as SSN. The gRNA for Cas9 as RTDD can target the repair template or an extension thereof, forming a Cpf1-dCas9-RT complex. The crRNA (Cpf1) targets the genomic locus defined for the double-strand cut to initiate HDR.

Likewise, a highly active zinc finger protein, a megaTAL or an inactive meganuclease can be used as interaction domain.

In one embodiment according to the various aspects of the present disclosure, the at least one repair template docking domain (RTDD), or the nucleic acid sequence encoding the same, or the at least one artificial molecular complex is selected from at least one of biotin, an aptamer, a DNA, RNA or protein dye, comprising fluorophores, comprising fluorescein, or a variant thereof, maleimides, or Tetraxolium (XTT), a guide nucleic acid sequence specifically configured to interact with at least one repair template nucleic acid sequence, a streptavidin, or a variant thereof, such as a monomeric steptavidin, an avidin, or a variant thereof, an affinity tag, such as a streptavidin-tag, an antibody, a single-chain variable fragment (scFv), a single-domain antibody (nanobody), an anticalin, an Agrobacterium VirD2 protein or a domain thereof (see e.g., SEQ ID NO: 33), a Picornavirus VPg, a topoisomerase or a domain thereof, a PhiX174 phage A protein, a PhiX A* protein, a VirE2 protein or a domain thereof, or digoxigenin. Therefore, the RTDD can be a naturally occurring or a synthetic molecule not being restricted to a nucleic acid or an amino acid molecule. The RTDD is thus rather a specific interaction mediator of the artificial molecular complex of the present disclosure, which can be designed in a versatile way to couple at least one SSN of interest and at least one repair template specific for a genomic complementarity region of interest and optionally carrying an insert of interest to be introduced at a DNA target sequence of interest cleaved by the at least one SSN. For embodiments using CRISPR or Argonaute based SSNs the RTDD can be a guide nucleic acid sequence. An RTDD according to the present disclosure can thus be a molecule belonging to various classes of artificial or natural molecules. The RTDD is thus defined by its capacity to directly interact with at least one repair template nucleic acid sequence (RT) and additionally by directly interacting with the at least one SSN. The RTDD is thus the molecular linker within the artificial molecular complex providing a close physical proximity of the RT and the SSN and - due to its dual interaction with the RT and the SSN - guaranteeing the association of the artificial molecular complex *in vitro* and *in vivo* by means of highly specific molecular interactions. For certain embodiments, more than one RTDD carrying more than one RT can be present.

In another embodiment of the present disclosure, the artificial molecular complex comprises an interaction domain, wherein the at least one interaction domain, or the nucleic acid sequence encoding the same, is selected from at least one of a DNA-binding domain, a streptavidin, or a variant thereof, such as a monomeric steptavidin, avidin, or a variant thereof, an affinity tag, a biotinylation signal, a biotin acceptor site, a streptavidin-tag, an antibody, a single-chain variable fragment (scFv), a single-domain antibody (nanobody), an anticalin, biotin, an aptamer, a DNA, RNA or protein dye, comprising fluorophores, comprising fluorescein, or a variant thereof, maleimides, or Tetraxolium (XTT), a guide nucleic acid sequence specifically configured to interact with a at least one repair template nucleic acid sequence, an Agrobacterium VirD2 protein or a domain thereof, a Picornavirus VPg, a topoisomerase or a domain thereof, a PhiX174 phage A protein, a PhiX A* protein, a VirE2 protein or a domain thereof, or digoxigenin.

Notably, a RTDD and an interaction domain can be selected from a comparable and overlapping class of molecules due to the fact that the interaction domain is an optional component, which can additionally optimize the specificity or efficiency of an artificial molecular complex according to the present disclosure. The presence of an interaction domain can be of importance for embodiments using artificial molecular complexes, wherein no nucleic acid guided nuclease is used as SSN, or wherein the SSN carries one or more mutation(s) modifying the intrinsic DNA recognition, binding or cleavage activity of the SSN.

In yet another embodiment, the presence of an interaction domain can be favorable to be used in combination with any kind of SSN to further increase the targeting range, the efficiency of binding and/or cleavage, the cleavage rate, or the precision of targeting to a DNA target sequence of interest, as the interaction domain as further component within the artificial molecular complex can add an expanded functionality to the complex and can thus broaded the scope of applicability thereof. Particularly for genome engineering in higher eukaryots comprising complex genomes, the presence of an additional component, i.e., the interaction domain, can thus be of outstanding importance to achieve an improved precision of DNA cleavage and - mediated by the RT according to the present disclosure - targeted repair. In certain embodiments of the present disclosure, the IA can represent a highly specific binding partner for a molecule partner not involved in genome engineering itself, wherein the molecule partner or cognate binding partner represents a RTDD being associated with a RT Therefore, the additional level of adding an IA domain as well as a cognate partner RTDD can add significantly more binding specificity and RT availability to the artificial molecular complex to improve the outcome of a targeted genome engineering approach.

The interaction domain (IA) according to the present invention/disclosure has several functionalities selected from the group consisting of (i) interaction with the at least one repair template docking domain; and/or (ii) interaction with the at least one repair template nucleic acid sequence; and/or (iii) sequence-specific interaction with genomic DNA. More than one of these functionalities can be unified within one specific IA.

It might be considered to use an IA which represents a protein or polypeptide having intrinsic high specificity and high affinity binding capacities for a cognate ligand, e.g., a synthetic ligand, including fluorescein for a biomolecule, including biotin or digoxigenin and variants thereof, for an aptamer or an antigen/epitope. The term "antigen" as used herein and as commonly used in the field of immunology refers to an "antibody generating" molecule, i.e., a substance, which can elicit an adaptive immune response. An antigen is thus a molecule binding to an antigen-specific receptor, either a T-cell or a B-cell receptor or a variant thereof, e.g., a nanobody or a single-chain variable fragment antibody, bispecific antibodies a tandem di-scFv, a diabody, a tandem tri-scFv (trivalent) or a triabody (trivalent). An antigen is usually a (poly)peptide, but it can also be a polysaccharide or a lipid, possibly combined with a protein or polysaccharide carrier molecule. Mediated by this intrinsic binding/recognition property of the IA, an IA of interest can be chosen which will specifically recognize a RTDD in a highly specific manner and the IA can be connected to or fused to, covalently or non-covalently, to a SSN. The inclusion of such an IA thus adds an additional level of specificity to the artificial molecular complex of the present disclosure and guarantees that the RT directly interacting with the RTDD, will be specifically associated with the SSN-IA complex as mediated by the highly specific IA-RTDD association. Most desirably, the IA and the cognate RTDD have a high affinity constant or bonding affinity and thus a low dissociation constant ($K_d$) for each other under physiological conditions, i.e. a $K_d$ value in the low $\mu$M, or nM range, and below. The IA can be a monovalent, a divalent, a trivalent or a multivalent molecule having one or more specificities (trivalent antibody derived fragment), respectively, or having more than one binding site (tetrameric streptavidin). In this embodiments, more than one RTDD and/or RT can be present and presented to the at least one SSN with the artificial molecular complex. IAs are desirable which have low dissociation constants ($K_d$), i.e., in turn which have a high affinity for their cognate ligand. Usually, sub-picomolar dissociation constants as a result of non-covalent binding interactions between two molecules, i.e., the typical interaction form between a protein and a ligand, are rare. Nevertheless, there are some important exceptions. Biotin and naturally occurring avidin bind with a dissociation constant of roughly $10^{-15}$ M, which represents an affinity that high not being suitable for applications, where a reversible binding is intended. Commercial antibodies or scFvs can have $K_d$ values in the range of $10^{-14}$ M $10^{-6}$ M. For the purpose of the present disclosure, an IA-RTDD pair should thus have a low dissociation constant, i.e., a high affinity.

Additionally, in certain embodiments of the present disclosure, the IA can directly interact with the RT. When the RT nucleic acid sequence comprises a stretch, e.g., a nucleic acid based aptamer this sequence can be recognized by a cognate binding partner, the IA, which can then interact with the RT in a highly specific manner. Furthermore, the IA can be a divalent or trivalent or multivalent molecule having more than one binding specificity. One portion of the IA can be configured to interact with the RTDD, and one portion can be configured to interact with the RT, whilst the IA is associated with the SSN, so that an even tighter association of the RT and the SSN during genome engineering can be achieved.

In another embodiment of the present disclosure, the IA can be a binding molecule having the capacity of sequence-specific interaction with a genomic DNA. This will add more specificity during targeting of an artificial molecular complex to a DNA target sequence. Furthermore, this allows the use of modified SSNs so that an SSN with optimized cleavage activity can be provided, whereas the IA mediates the function of targeting the artificial molecular complex to a DNA target sequence with high precision, whereas SSN and/or IA can interact with an RTDD interacting with and thus presenting the RT to the site, where a double-strand break will be induced. In one embodiment the IA can thus be DNA-binding domain or DNA-binding motif designed to be part of a fusion protein on either the N- or C-terminus of the at least one SSN nuclease or a variant thereof. An amino-acid based linker will allow for flexibility and avoid steric hindrance for DNA binding or nuclease activity. Potential DNA binding domains could also be Zinc fingers (Roy et al. 2012), such as a $Cys_2/His_2$ Zn finger (Kubo et al. 1998), TALENs (Hubbard et al. 2015) or inactivated Argonaute or Cas9 proteins capable of highly specific DNA binding. Either of these DNA-binding domains would ideally target a sequence outside the homology-arm flanked sequence of interest to avoid steric hindrance of interaction and can thus add another level of specificity to the artificial molecular complex of the present disclosure.

In a further embodiment of the present disclosure, more than one IA domain can be used within the artificial molecular complex, i.e., one IA used as high specificity and affinity binder for a RTDD, and one IA used as additional DNA-binding domain, both IAs being directly in interaction with, i.e., being covalently or non-covalently associated with, the at least one SSN of the artificial molecular complex.

In one embodiment of the present disclosure, the at least one SSN and/or the at least one IA comprise a biotinylation signal or biotinylation acceptor site or a strep-tag. The relevant signal/site can be biotinylated *in vitro* or *in vivo* by endogenous (BirA) or exogenous biotinylating enzymes/agents, or in an *in vitro* biotinylation step, and the biotinylated signal/site and/or the strep-tag can then be recognized and bound by a streptavidin or avidin, or a modified variant thereof, or a monomeric variant thereof, wherein the streptavidin or avidin or the variant thereof will be associated with a RT of interest. As avidin is known to interact unspecifically with DNA (Morpurgo et al., 2004), modified variants of avidin or streptavidin or variants thereof might be desirable.

Particularly for SSNs not relying on guiding RNAs/DNAs the additional binding capacitiy and thus RT targeting capability of the monomeric streptaviding or scFv with a given binding specificity can dramatically increase the range of suitable SSNs for genome engineering if used in combination with the RTDDs and/or IAs according to the present disclosure.

In one embodiment, biotin can be fused to the repair template DNA by commercially available kits or as part of a third party synthesis process as RTDD. Using a modified streptavidin or avidin sequence ensures that no inter-protein complexing occurs and one biotinylated repair template DNA is bound per protein. The repair template is then linked to streptavidin or any variant thereof as interaction domain (Niemeyer et al. 1999, "Functionalization of covalent DNA-streptavidin conjugates by means of biotinylated modulator components." Bioconjug Chem 10(5): 708-719), wherein the interaction domain is directly interacting with the SSN, e.g., by providing the SSN and the streptavidin as fusion molecule. In another embodiment, the SSN can comprise a biotinylation signal or peptide and the biotinylation will proceed *in vivo* in a host cell. In this embodiment, streptaviding or avidin, or a variant thereof, can function as RTDD itself being linked to a RT. An exemplary sequence encoding a monomeric streptavidin (mSA) suitable as interaction domain or as RTDD according to the present disclosure is shown in SEQ ID NO: 34. mSA fused to a SSN could thus be understood either as a RTDD or as an interaction domain according to the present disclosure. In another embodiment, the SSN can carry a strep-tag, the tag being recognized by a streptavidin variant functioning as interaction domain or as RTDD, respectively. Suitable streptavidin or avidin enzymes, or variants thereof, or vectors encoding the same, are available to the skilled person, e.g., from IBA Lifesciences (Göttingen, Germany), addgene (Cambridge, MA, USA), Intregrated DNA Technologies (Coralville, IA, USA), or GeneArt (ThermoFisher; Waltham, MA, USA). Another exemplary sequence for a monomeric streptavidin construct encoding mSA suitable as IA or RTDD according to the present disclosure is shown in SEQ ID NO: 42.

In some embodiment of the present disclosure, the interaction or attachment or association between the RTDD and the SSN and/or the interaction domain thus results from an interaction of a binding-pair selected from non-covalent interaction of a binding-pair selected from, but not limited to: biotin-avidin; biotin-streptavidin; biotin-modified forms of avidin; protein-protein; protein-nucleic acid interactions; ligand-receptor interactions; ligand-substrate interactions; antibody-antigen; single-chain antibody-antigen; antibody or single-chain antibody-hapten; hormone-hormone binding protein; receptor-agonist; receptor-receptor antagonist; IgG-protein A; enzyme-enzyme cofactor; enzyme-enzyme inhibitor; single-strand DNA-VirE2; StickyC-dsDNA; RISC (RNA-induced silencing complex)-RNA; viral coat protein-nucleic acid; anti-Fluorescein single-chain variable fragment antibody (anti-FAM scFV)-fluorescein; anti-digoxigenin (DIG) single-chain variable fragment (scFv) immunoglobin (DIG-scFv)-digoxigenin (DIG) and *Agrobacterium* VirD2-binding protein or any combination or variation thereof. Notably, antibodies and antibody fragments or derivatives like scFvs, nanobodies ordiabodies having custom-made specificities and high affinities (in the pM or even fM range) are commercially available, particularly such antibodies or fragments or variants thereof binding classical dyes, like fluorescein, or derivatives thereof.

In one embodiment of the present disclosure, the interaction domain according to the present disclosure is selected from a leucine zipper, an aptamer sequence, dCas9, dCPF1, a meganuclease, a zinc finger, or a TALE construct. In this embodiment, the at least one SSN and the RT DNA can be brought into direct interaction through an intermediate DNA-binding domain or DNA-binding motif designed to be part of a fusion protein on either the N- and/or C-terminus of the SSN. An amino-acid based linker will allow for flexibility and avoid steric hindrance for DNA binding or nuclease activity. Potential DNA binding domains could also be Zinc fingers (Roy et al. 2012, "Prediction of DNA-binding specificity in zinc finger proteins." J Biosci 37(3): 483-491), such as a $Cys_2/His_2$ Zn finger (Kubo et al. 1998, "Cys2/His2 zinc-finger protein family of petunia: evolution and general mechanism of target-sequence recognition." Nucleic Acids Research 26(2): 608-615), TALENs (Hubbard et al. 2015, "Continuous directed evolution of DNA-binding proteins to improve TALEN specificity." Nat Methods 12(10): 939-942) or inactivated Argonaute or Cas proteins capable of highly specific DNA binding. Either of these DNA-binding domains as interaction domains can additionally help to target a sequence outside the homology-arm flanked sequence of interest to avoid steric hindrance of interaction. Said interaction domains can fulfil the function of increasing DNA-binding of the artificial molecular complex of the present disclosure and/or to allow the provision of additional docking sites for RTDD/RT linkage to provide a highly specific complex suitable for genome engineering.

According to certain embodiments of the present disclosure, the at least one SSN according to the present disclosure may be fused to a DNA binding domain, i.e., a protein or a fragment thereof, or a gene sequence encoding said protein or a fragment of the protein, that bind DNA molecules or bind other cellular molecules, including but not limited to maltose binding protein (MBP), S-tag, Lex A DNA binding domain (DBD) fusions, GAL4 DNA binding domain fusions, and herpes simplex virus (HSV) BP16 protein fusions.

In certain embodiments of the present disclosure, there may be more than one RTDD. The first RTDD could be a mSA or a single-chain variable fragment (scFv), whereas the second RTDD could be biotin or the cognate ligand of the scFv. In one embodiment, using a CRISPR or Argonaute based SSN system, the first RTDD is a guide nucleic acid sequence, and the second RTDD is a biotin or fluorescein or any other high affinity binding partner moiety linked to a RT, wherein a monomeric streptavidin or a scFv or another cognate protein binding partner represents an IA recognizing the the second RTDD and bind thereto with high affinity. This design of the artificial molecular complex according to the present disclosure allow maximum flexibility to bring a RT into close contact with an effector SSN by simultaneously providing high RT availability and no loss of the RT, as the RTDDs provide strong and reliable interactions with the RT and the SSN to achieve precision genome engineering events.

In one embodiment of the present disclosure, repair template linkage to the SSN can be achieved by a single-chain variable fragment (scFv) antibody against the dye Fluorescein. The scFv specifically binding fluorescein and fluorescein-derivatives is fused to the SSN in a hybrid-protein manner (Schenk et al. 2007, "Generation and application of a fluorescein-specific single-chain antibody." Biochimie 89(11): 1304-1311). In another embodiment, the SSN can comprise a fluorescein molecule interacting therewith and the cognate fluorescein specific scFv can be provided as a fusion with a RT and can bind the fluorescein associated with the SSN. The scFv thus can function as RTDD or as interaction domain according to the present disclosure.

In other embodiments of the present disclosure, any scFv with a different binding specificity can be used.

Suitable scFv-ligand pairs are selected from the group consisting of a scFv and fluorescein (FAM) or any FAM derivative or variant, a scFv recognizing digoxigenin (DIG), a custom-made scFv recognizing an epitope/antigen on a SSN of interest, and the like. One exemplary sequence encoding a scFv encoding sequence with binding capacity for fluorescein is shown in SEQ ID NO: 43.

In another embodiment of the present disclosure, an aptamer sequence is designed to specifically interact with the at least one SSN. In this embodiment, the aptamer sequence can be covalently or non-covalently linked to a repair template sequence of interest to allow a direct association between the SSN and the aptamer as RTDD without creating a fusion protein and/or utilizing an additional interaction domain. In embodiments, wherein no separate interaction domain is used, the RTDD interacting with the RT comprises a nucleotide motif capable of specifically interacting, i.e., attaching or binding to a domain of the at least one SSN protein, or a specific domain thereof configured to interact with the RTDD: In some embodiments, the interaction is selected from, but not limited to: Zinc finger protein-Zinc finger motif; restriction enzyme recognition domain-restriction enzyme recognition sequence; DNA binding domain of transcription factor-DNA motif; repressor-operator; Leucine zipper-promoter; Helix loop helix-E box domain; RNA binding motifs comprising Arginine-Rich Motif domains, $\alpha\beta$ protein domains, RNA Recognition Motif (RRM) domains, K-Homology Domains, Double-Stranded RNA Binding Motifs, RNA-binding Zinc Fingers, and RNA-Targeting Enzymes-cognate specific RNA sequence; HIV-rev protein-Stem IIB of the HIV rev response element (RRE); Bovine immunodeficiency virus (BIV) Tat main binding domain-loop 1 of the BIV trans-acting response element (TAR) sequence; Phage lambda, phi21, and P22 N proteins, the boxB loop hairpins in the N-utilization (nut) sites in their respective RNAs.

As far as the present invention relates to the use of an Argonaute as site-specific nuclease, in addition to the advantages of a guide-DNA molecule, delivery of the NgAgo endonuclease is facilitated by its small size. The wild-type (WT) protein (GenBank Accession Number AFZ73749) is 887 amino acids, or roughly 2/3 the size of *Streptococcus pyogenes* Cas9. This simplifies cloning and vector assembly, can increase expression levels of the nuclease in cells, and reduces the challenge in expressing the protein from highly size-sensitive platforms such as viruses, including either DNA or RNA viruses. Like other nucleic acid guided endonucleases, NgAgo SSNs usually require a minimum of two components for targeted mutagenesis in plant cells: a 5'-phosphorylated single-stranded guide-DNA and the NgAgo endonuclease protein. For targeted edits, insertions, or sequence replacements, a DNA template encoding the desired sequence changes can also be provided to the plant cell to introduce changes either via the NHEJ or HR repair pathways. Successful editing events are most commonly detected by phenotypic changes (such as by knockout or introduction of a gene that results in a visible phenotype), by PCR-based methods (such as by enrichment PCR, PCR-digest, or T7EI or Surveyor endonuclease assays), or by targeted Next Generation Sequencing (NGS; also known as deep sequencing). In one specific embodiment, the modified Argonaute endonuclease is active at a temperature from about 20°C to about 35°C. In one specific embodiment, the modified Argonaute endonuclease is active at a temperature from about 23°C to about 32°C. Argonaute proteins which can function as endonucleases can comprise three key functional domains: a PIWI endonuclease domain, a *PAZ* domain, and a MID domain. The PIWI domain may resemble a nuclease. The nuclease may be an RNase H or a DNA-guided ribonuclease. The PIWI domain may share a divalent cation-binding motif for catalysis exhibited by other nucleases that can cleave RNA and DNA. The divalent cation-binding motif may contain four negatively charged, evolutionary conserved amino acids. The four negatively charged evolutionary conserved amino acids may be aspartate-glutamate-aspartate-aspartate (DEDD). The four negatively charged evolutionary conserved amino acids may form a catalytic tetrad that binds two Mg2+ ions and cleaves a target nucleic acid into products bearing a 3' hydroxyl and 5' phosphate group. The PIWI domain may further comprise one or more amino acids selected from a basic residue. The PIWI domain may further comprise one or more amino acids selected from histidine, arginine, lysine and a combination thereof. The histidine, arginine and/or lysine may play an important role in catalysis and/or cleavage. Cleavage of the target nucleic acid by Argonaute can occur at a single phosphodiester bond. In some instances, one or more magnesium and/or manganese cations can facilitate target nucleic acid cleavage, wherein a first cation can nucleophilically attack and activate a water molecule and a second cation can stabilize the transition state and leaving group. For certain Argonaute nucleases, the length of the gDNA will provide for the affinity between Argonaute and guiding gDNA.

Suitable argonaute proteins according to the present disclosure are shown with SEQ ID NOs: 19 and 20, or may comprise a sequence having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence homology thereto provided that the homologous sequence still fulfills the function of the argonaute protein it is derived from, i.e., it originates from. Further suitable argonaute sequences are disclosed in the provisional U.S. application No. 62/345,448 which are incorporated herein by reference. Further suitable Argonaute sequences can be derived from a sequence according to SEQ ID NOs: 21 to 29 or a sequence having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence homology thereto.

An Argonaute can comprise a nucleic acid-binding domain. The nucleic acid-binding domain can comprise a region that contacts a nucleic acid. A nucleic acid-binding domain can comprise a nucleic acid. A nucleic acid-binding domain can comprise a proteinaceous material. A nucleic acid-binding domain can comprise nucleic acid and a proteinaceous material. A nucleic acid-binding domain can comprise DNA. A nucleic acid-binding domain can comprise single-stranded DNA. Examples of nucleic acid-binding domains can include, but are not limited to, a helix-turn-helix domain, a zinc finger domain, a leucine zipper (bZIP) domain, a winged helix domain, a winged helix-turn-helix domain, a helix-loop-helix domain, a HMG-box domain, a Wor3 domain, an immunoglobulin domain, a B3 domain, or a TALE domain. A nucleic acid-binding domain can be a domain of an Argonaute protein. An Argonaute protein can be a eukaryotic Argonaute or a prokaryotic Argonaute. An Argonaute protein can bind RNA or DNA, or both RNA and DNA. An Argonaute protein can cleave RNA, or DNA, or both RNA and DNA. In some instances, an Argonaute protein binds a DNA and cleaves the DNA. In some instances, the Argonaute protein binds a double-stranded DNA and cleaves a double-stranded DNA. In some instances, two or more nucleic acid-binding domains can be linked together. Linking a plurality of nucleic acid-binding domains together can provide increased polynucleotide targeting specificity. Two or more nucleic acid-binding domains can be linked via one or more linkers. The linker can be a flexible linker. Linkers can comprise 1, 2, 3, 4, 5, 6, 7, 8, 9, 10, 11, 12, 13, 14, 15, 16, 17, 18, 19, 20, 21, 22, 23, 24, 25, 30, 35, 40 or more amino acids in length. The linker domain may comprise glycine and/or serine, and in some embodiments may consist of or may consist essentially of glycine and/or serine. Linkers can be a nucleic acid linker which can comprise nucleotides. A nucleic acid linker can link two DNA binding domains together. A nucleic acid linker can be at most 5, 10, 15, 20, 25, 30, 35, 40, 45, or 50 or more nucleotides in length. A nucleic acid linker can be at least 5, 10, 15, 30, 35, 40, 45, or 50 or more nucleotides in length. Nucleic acid-binding domains can bind to nucleic acid sequences. Nucleic acid binding domains can bind to nucleic acids through hybridization. Nucleic acid-binding domains can be engineered (e.g., engineered to hybridize to a sequence in a genome). A nucleic acid-binding domain can be engineered by molecular cloning techniques (e.g., directed evolution, site-specific mutation, and rational mutagenesis).

In certain embodiments of the present disclosure, the SSN according to the present disclosure will be a CRISPR nuclease, including Cas or Cpf1, or an Argonaute nuclease, or a variant or a catalytically active fragment thereof. Suitable CRISPR nuclease sequences are selected from the group consisting of SEQ ID NOs: 19 to 29, or 35 to 41 or a sequence having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence homology thereto. Further suitable Cas or Cpf1 effectors can be derived from an organism from a genus comprising *Streptococcus, Campylobacter, Candidatus* Micrarchaeum acidiphilum ARMAN-1, *Parcubacteria* (GenBank: APG80656.1), *Sulfolobus* spp., including *Sulfolobus islandicus* HVE10/4 (GenBank: ADX81770.1) or REY15A (GenBank: ADX84852.1), *Nitratifractor, Staphylococcus, Parvibaculum, Roseburia, Neisseria, Gluconacetobacter, Azospirillum, Sphaerochaeta, Lactobacillus, Eubacterium, Corynebacter, Camobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Letospira, Desulfovibrio, Desulfonatronum, Opitutaceae, Tuberibacillus, Bacillus, Brevibacilus, Methylobacterium* or *Acidaminococcus.*, e.g., from *S. mutans, S. agalactiae, S. equisimilis, S. sanguinis, S. pneumonia; C. jejuni, C. coli; N. salsuginis, N. tergarcus; S. auricularis, S. carnosus; N. meningitides, N. gonorrhoeae; L. monocytogenes, L. ivanovii; C. botulinum, C. difficile, C. tetani, C. sordellii.*

In one embodiment of the present disclosure, the at least one site-specific nuclease or the catalytically active fragment thereof as part of the artificial molecular complex of the present disclosure, or the sequence encoding the same, is independently selected from the group consisting of a Cas polypeptide of *Streptococcus spp.,* including *Streptococcus pyogenes, Streptococcus thermophilus, Staphylococcus aureus,* or *Neisseria spp.,* including *Neisseria meningitides, Corynebacter, Sutterella, Legionella, Treponema, Filifactor, Eubacterium, Lactobacillus, Mycoplasma, Bacteroides, Flaviivola, Flavobacterium, Sphaerochaeta, Azospirillum, Gluconacetobacter, Roseburia, Parvibaculum, Nitratifractor, Mycoplasma* and *Campylobacter, Candidatus* Micrarchaeum acidiphilum ARMAN-1, *Parcubacteria* (GenBank: APG80656.1), *Sulfolobus* spp., including *Sulfolobus islandicus* HVE10/4 (GenBank: ADX81770.1) or REY15A (GenBank: ADX84852.1), a Cpf1 polypeptide from an archaea or a bacterium, including a Cpf1 polypeptide of *Acidaminococcus spp.,* including *Acidaminococcus* sp. BV3L6, *Lachnospiraceae spp.,* including *Lachnospiraceae bacterium* ND2006, *Lachnospiraceae* bacterium MC2017, *Lachnospiraceae* bacterium MA2020, *Butyrivibrio proteoclasticus,* Candidatus spp., *Methanoplasma termitum, Leptospira inadai, Moraxella bovoculi 237,* Peregrinibacteria bacterium GW2011_GWA2_33_10, Parcubacteria bacterium GW2011_GWC2_44_17, Smithella sp. SCADC, Smithella sp. SC_K08D17, *Francisella spp.,* including *Francisella novicida* U112, *Eubacterium eligens, Prevotella spp.,* or *Porphyromonas spp.,* or an Argonaute nuclease from *Natronobacterium gregoryi* (GenBank: AFZ73749.1), *Microcystis aeruginosa* (NCBI Reference Sequence: WP_012265209.1 or NCBI Reference Sequence: WP_002747795.1 or NCBI Reference Sequence: WP_012265209.1), *Halogeometricum pallidum* (GenBank: ELZ29017.1), *Natrialaba asiatica* (NCBI Reference Sequence: WP_006111085.1), *Natronorubrum tibetense* (NCBI Reference Sequence: WP_006090832.1), *Natrinema pellirubrum* (NCBI Reference Sequence: WP_006183335.1), or *Synechococcus spp.* (NCBI Reference Sequence: WP_011378069.1) or variants and/or functional fragments and/or combinations thereof, including nickases, or nucleases lacking endonucleolytic activity.

In further embodiments of the invention using at least one Cpf1 effector as SSN, a protospacer adjacent motif (PAM) or PAM-like motif directs binding of the effector protein complex to the target locus of interest. In an embodiment using at least one Cpf1 effector as SSN, the PAM is 5' TTN, where N is A/C/G or T. In another preferred embodiment of the invention, the PAM is 5' TTTV, where V is A/C or G. In certain embodiments, the PAM is 5' TTN, where N is A/C/G or T and the PAM is located upstream of the 5'-end of the protospacer. In certain embodiments of the invention, the PAM is 5' CTA, and the PAM is located upstream of the 5'-end of the protospacer or the target locus. In certain embodiments, there is provided an expanded targeting range for RNA guided genome editing nucleases wherein the T-rich PAMs of the Cpf1 family allow for targeting and editing of AT-rich genomes.

In certain embodiments, the CRISPR enzyme is engineered and can comprise one or more mutations that reduce or eliminate a nuclease activity. Likewise, the present invention contemplates methods of using two or more nickases, in particular a dual or double nickase approach to generate a targeted DNA double-strand break.

In embodiments using Cpf1 effector protein complexes within the artificial molecular complex according to the present disclosure, a Cpf1 effector having one or more non-naturally occurring or engineered or modified or optimized nucleic acid components, or the encoded protein, can be used. In a preferred embodiment the nucleic acid component of the complex may comprise a guide sequence linked to a direct repeat sequence, wherein the direct repeat sequence comprises one or more stem loops or optimized secondary structures. In a preferred embodiment, the direct repeat has a minimum length of 16 nucleotids and a single stem loop. In further embodiments the direct repeat has a length longer than 16 nucleotids, preferably more than 17 nucleotids, and has more than one stem loop or optimized secondary structures. In a preferred embodiment the direct repeat may be modified to comprise one or more protein-binding RNA aptamers. In a preferred embodiment, one or more aptamers may be included such as part of optimized secondary structure. Such aptamers may be capable of binding a bacteriophage coat protein. The bacteriophage coat protein may be selected from the group comprising Qβ, F2, GA, fr, JP501, MS2, M12, R17, BZ13, JP34, JP500, KU1, M11, MX1, TW18, VK, SP, FI, ID2, NL95, TW19, AP205, (pCb5, (pCb8r, φCb12r, φCb23r, 7s and PRR1. In a preferred embodiment the bacteriophage coat protein is MS2.

In certain embodiments, the disclosure also provides for the one or more mutations or the two or more mutations to be in a catalytically active fragment of the at least one SSN effector protein comprising a RuvC domain. In some embodiments of the present disclosure, the RuvC domain may comprise a RuvCI, RuvCII or RuvCIII domain, or a catalytically active domain which is homologous to a RuvCI, RuvCII or RuvCIII domain etc or to any relevant domain as described in any of the herein described methods. The effector protein SSN may comprise one or more heterologous functional domains. The one or more heterologous functional domains of the artificial molecular complex may comprise one or more nuclear localization signal (NLS) domains. The one or more heterologous functional domains may comprise at least two or more NLS domains. The one or more NLS domain(s) may be positioned at or near or in promixity to a terminus of the effector protein (e.g., Cpf1) and if two or more NLSs, each of the two may be positioned at or near or in promixity to a terminus of the effector protein (e.g., Cpf1) The one or more heterologous functional domains may comprise one or more transcriptional activation domains. In one embodiment of the present disclosure the transcriptional activation domain may comprise VP64. The one or more heterologous functional domains may comprise one or more transcriptional repression domains. In one embodiment of the present disclosure the transcriptional repression domain comprises a KRAB domain or a SID domain (e.g., SID4X). The one or more heterologous functional domains may comprise one or more nuclease domains as SSNs. In one embodiment, the SSN can comprise Fok1 or a catalytically active fragment or variant thereof.

In one preferred embodiment of the present invention, the at least one site-specific nuclease or the variant or catalytically active fragment thereof of the artificial molecular complex according to the present disclosure, or the sequence encoding the same, is selected from a CRISPR nuclease, preferably from a Cas or a Cpf1 nuclease, or a FokI nuclease, or a catalytically fragment thereof, and the at least one interaction domain, or the sequence encoding the same, is selected from a single-chain variable fragment or a monomeric streptavidin.

In one embodiment, the artificial molecular complex according to the present disclosure comprises at least one CRISPR or Argonaute derived SSN, or a variant or a catalytically active fragment thereof, and at least one guide nucleic acid sequence representing the at least one repair template docking domain, wherein each of the at least one guide nucleic acid sequences comprises (i) a first sequence portion that is complementary to a recognition DNA target sequence, and (ii) a second sequence portion, wherein the second sequence portion is configured to interact with the at least one site-specific nuclease, and (iii) wherein the at least one guide nucleic acid sequence is physically associated with the at least one repair template nucleic acid sequence and thus forms a hybrid nucleic acid sequence comprising or consisting of at least one RNA or DNA and at least one further DNA nucleic acid sequence, and (iv) optionally comprising a linker region between the at least one guide nucleic acid sequence and the at least one repair template nucleic acid sequence, for instance wherein the repair template nucleic acid sequence is associated with the guide nucleic acid sequence at the 3'-end of the guide nucleic acid sequence, and/or wherein the repair template nucleic acid sequence is associated with the 5'-end of the guide nucleic acid sequence, and/or wherein the repair template nucleic acid sequence is located within the guide nucleic acid sequence.

The at least one repair template nucleic acid sequence and/or the at least one guide nucleic acid sequence according to the various aspects and embodiments of the present invention comprises a nucleotide sequence selected from a naturally or non-naturally occurring nucleotide sequence, including a synthetic nucleotide sequence, optionally comprising backbone and/or base modifications, wherein the guide nucleic acid sequence comprises a single-stranded or partially single-stranded RNA or DNA nucleotide sequence, and wherein the at least one repair template nucleic acid sequence comprises a single-stranded or a double-stranded DNA nucleotide sequence.

In certain embodiments, the at least one repair template nucleic acid sequence (RT) of the artificial molecular complex according to the present disclosure comprises at least one end portion, preferably the 3'-end, wherein this end portion does not interact with any other component of the artificial molecular complex and is thus configured to hybridize to at least one genomic complementarity sequence to mediate repair of the DNA target sequence, and/or wherein the at least one repair template nucleic acid sequence is provided as plasmid. To be able to access the at least one genomic complementarity sequence, the RT should thus be provided in a configuration allowing optimum base pairing with the at least one genomic complementarity sequence. This configuration will vary depending on the nature of the RT and depending on the way of providing the RT. In certain embodiments, at least one RT is used which can be covalently or non-covalently attached to a RTDD, e.g., a gRNA or a gDNA.

In certain embodiments of the present disclosure using a molecule comprising at least one RNA stretch as RTDD, or using RNA encoding a protein of interest, the RNA can be presented together with a protecting or protector molecule or strand, which protector molecule will anneal at least partially to the RNA representing the actual effector molecule of the artificial molecular complex to protect the RNA effector molecule from degradation within the cell.

Suitable configurations for an artificial molecular complex according to the present disclosure are shown in **Figures 1** to **4.** Artificial molecular complexes using a "hybrid nucleic acid sequence" as RTDD and RT according to the present disclosure are shown in **Fig. 1 A** to **D** and **Fig. 2 A** to **C.** Depending on the SSN, the repair template (RT) can be in a ssDNA or dsDNA form and, in case a CRISPR or Argonaute protein is used as SSN, can be attached to the at least one guide nucleic acid (sgRNA or gRNA or gDNA) at the 3'-end, the 5'-end in a covalent or non-covalent way or it can lie within the gRNA, e.g., forming a hairpin secondary structure of a defined size and shape. This design allows that both the gRNA and the RT portion can both fulfill their functions without disturbing the interaction of at least one gRNA of interest with a CRISPR or Argonaute nuclease of interest and simultaneously positioning the RT in close proximity to the site of a DNA break induced by the at least one CRISPR/gRNA Argonaute/gDNA pair.

In certain embodiments using CRISPR nucleases, the artificial molecular complex will comprise a hybrid nucleic acid sequence comprising or consisting of at least one RNA and at least one DNA nucleic acid sequence or simply a hybrid RNA/DNA nucleic acid sequence according to the present disclosure thus represents a chimeric RNA and DNA comprising molecule, which comprises two functionalities. First, it comprises a guide nucleic acid (gRNA) moiety, comprising a ribonucleic acid. This gRNA comprises two nucleotide sequence portions, one nucleotide sequence being necessary for interaction with a CRISPR polypeptide of interest as well as another nucleotide sequence comprising a targeting domain, wherein the targeting domain is able to hybridize via base-pairing to a complementary DNA target sequence of interest adjacent to a PAM sequence in the opposite strand, this complementary DNA target sequence thus representing the first DNA target sequence according to the present disclosure. Secondly, the hybrid RNA/DNA nucleic acid sequence comprises a repair template nucleic acid sequence moiety which can comprise a desired edit to be introduced into a DNA target sequence of interest. Furthermore, the repair template nucleic acid sequence can comprise additional homologous sequence immediately upstream and downstream of the DNA target sequence, i.e., left and right homology arms. The length and binding position of each homology arm is dependent on the size of the change being introduced, and can be adjusted for optimal efficiency. For example, it is likely that a repair template with complementarity specific for the cleaved DNA strand first released by Cas9 (as described in Richardson, et al., Nature Biotechnology. 2016, doi:10.1038/nbt.3481) may produce the most efficient repair. The repair template can be a single-stranded or a double-stranded DNA nucleotide sequence depending on the specific application.

The repair template may contain polymorphisms relative to the genomic DNA to disrupt binding by the nuclease, otherwise the repair template becomes a suitable target for CRISPR polypeptide cleavage. For example, the PAM could be mutated such that it is no longer present, but the coding region of the gene is not affected, which corresponds to a silent mutation not changing the encoded amino acid sequence. In another embodiment, where a nuclease deficient CRISPR polypeptide is used within the artificial molecular complex as SSN, the presence of a PAM sequence within the repair template sequence is possible. In one embodiment, the RTDD/RTsequence comprises at least one guide nucleic acid sequence and at least one repair template nucleic acid sequence, but the RTDD/RT hybrid can also comprise further moieties attached thereto suitable for genome editing as further detailed below. In another embodiment the hybrid RTDD/RT sequence consists of at least one guide nucleic acid sequence and at least one repair template nucleic acid sequence.

It was found that an optimal RT size can exist depending on the SSN used that provides a balance of nuclease efficiency with homology arm size for efficiency of HR-mediated DSB repair.

In one embodiment of the present disclosure, the guide nucleic acid sequence or gRNA is provided as one RNA nucleic acid sequence unifying a tracrRNA and a crRNA element. In another embodiment, for example when working with a Type V CRISPR system using a Cpf1 polypeptide or a variant or catalytically active fragment thereof, the gRNA comprises a crRNA element. In yet a further embodiment, the gRNA can be provided as more than one RNA nucleic acid sequence mimicking the natural situation in many CRISPR systems that crRNA and tracrRNA, if both necessary, are provided on two separate RNA molecules. In certain embodiments, this arrangement thus allows for the possibility of having the two elements (tracrRNA and crRNA) in separate RNA strands like in nature. In one embodiment, there is provided a separate RNA nucleic acid molecule providing a crRNA and there is provided a separate RNA nucleic acid molecule, i.e., more than one RTDD is presented. Eitherthe crRNA moiety or the tracrRNA moiety can be associated with a repair template (RT) nucleic acid sequence. For example, providing a tracrRNA:RT hybrid or a crRNA:RT can be preferred when *ex vivo* chemical synthesis of the tracrRNA:RT or the crRNA:RT is chosen due to the shorter length of the respective molecule in comparison to a gRNA:RT hybrid, wherein the gRNA consists of one single RNA molecule unifying crRNA and tracrRNA function.

The RTDD/RT sequence according to the present disclosure is thus suitable for precision genome editing in any cell type of interest, including prokaryotic cells and eukaryotic cells, including fungal, animal and plant cells and to any genome of interest in an *in vitro* setting and represents a suitable physically connected tool to allow simultaneous spatiotemporal availability of a repair template and SSN during genome editing.

According to all aspects and embodiments of the present disclosure, the at least one RTDD and the at least one repair template nucleic acid sequence are associated with each other. The term "associated with" or "in association" according to the present disclosure is to be construed broadly and, therefore, according to the present disclosure it implies that a RTDD, for example a gRNA or a biotin molecule, FAM or a digoxigenin, is provided in physical association with a DNA repair template, the association being either of covalent or non-covalent nature, inherently increasing the availability of the repair template for homologous recombination. Instead of indiscriminate amplification of the repair template, or provision of the repair template in excess, yet physically unlinked to the RTDD, the repair template nucleotide sequence is thus presented at the DSB together with the SSN of the artificial molecular complex to a DNA target sequence of interest, which in turn significantly improves the predictability and the specificity of a genome editing approach.

In a further embodiment according to the present disclosure, at least one repair template nucleic acid sequence is attached to at least one RTDD sequence by way of both covalent and/or non-covalent bonds or attachments. According to this embodiment, the hybrid RTDD and RT complex can be provided as *in vitro* synthesized molecule which can then be associated with at least one SSN of interest, either *in vitro,* or *in vivo* in the target cell of interest, or within an *in vitro* assay of interest. The cell may be a eukaryotic cell, including a fugal, an animal or a plant cell. The cell can also be a prokaryotic cell. Furthermore, the cell can be a prokaryotic or eukaryotic host cell carrying, either on a plasmid or integrated into the genome, a heterologous target sequence of another organism or virus. In this embodiment, the cell functions as host to perform genome engineering on a heterologous sequence provided within said host cell.

In one embodiment according to the various aspects of the present disclosure at least one repair template nucleic acid sequence (RT) is covalently attached to at least one RTDD. A covalent attachment or covalent bond is a chemical bond that involves the sharing of electron pairs between atoms of the molecules or sequences covalently attached to each other.

In another embodiment according to the various aspects of the present disclosure at least one repair template nucleic acid sequence is non-covalently attached to at least one RTDD sequence. A non-covalent interaction differs from a covalent bond in that it does not involve the sharing of electrons, but rather involves more dispersed variations of electromagnetic interactions between molecules/sequences or within a molecule/sequence. Non-covalent interactions or attachments thus comprise electrostatic interactions, van der Waals forces, TT effects and hydrophobic effects. Of special importance in the context of nucleic acid molecules are hydrogen bonds as electrostatic interaction. A hydrogen bond (H-bond) is a specific type of dipole-dipole interaction that involves the interaction between a partially positive hydrogen atom and a highly electronegative, partially negative oxygen, nitrogen, sulfur, or fluorine atom not covalently bound to said hydrogen atom.

The term "hybridization" as used herein refers to the pairing of complementary nucleic acids, i.e., DNA and/or RNA, using any process by which a strand of nucleic acid joins with a complementary strand through base pairing to form a hybridized complex. Hybridization and the strength of hybridization (i.e., the strength of the association between the nucleic acids) is impacted by such factors as the degree and length of complementarity between the nucleic acids, stringency of the conditions involved, the Tm of the formed hybrid, and the G:C ratio within the nucleic acids. The term hybridized complex refers to a complex formed between two nucleic acid sequences by virtue of the formation of hydrogen bounds between complementary G and C bases and between complementary A and T/U bases. A hybridized complex or a corresponding hybrid construct can be formed between two DNA nucleic acid molecules, between two RNA nucleic acid molecules or between a DNA and an RNA nucleic acid molecule. For all constellations, the nucleic acid molecules can be naturally occurring nucleic acid molecules generated *in vitro* or *in vivo* and/or artificial or synthetic nucleic acid molecules. Hybridization as detailed above, e.g., Watson-Crick base pairs, which can form between DNA, RNA and DNA/RNA sequences, are dictated by a specific hydrogen bonding pattern, which thus represents a non-covalent attachment form according to the present disclosure.

Concerning non-covalent associations according to the present disclosure, the at least one RTDD of the artificial molecular complex of the present disclosure and the at least one repair template sequence of the present disclosure can associate with each other by RNA-DNA base pairing.

Another form of non-covalent interaction is the association of the at least one repair template sequence with at least one component, either RTDD or a RTDD comprised by the SSN, by electrical charges.

Concerning a covalent association or attachment, the at least one RTDD and the at least one repair template sequence are connected as contiguous molecule, either produced *in vivo* or *in vitro.* Covalent and non-covalent attachment can also be combined, e.g., by providing a covalently attached RTDD/repair template sequence, which can further comprise an additional repair template nucleic acid sequence non-covalently attached to covalently attached RTDD/repair template sequence. This approach is especially suitable, in case the covalently attached RTDD/repair template sequence is at least partially produced *in vivo* and a further repair template, either produced *in two* or *in vitro,* is to be added to the pre-existing RTDD/repair template complex.

As also evident from Nishimasu et al., supra, a gRNA is configured to interact with a CRISPR polypeptide or a variant or a catalytically active fragment thereof in accordance with the present disclosure, if the gRNA comprises at least one portion usually comprising a heteroduplex configuration, which is recognized by a CRISPR polypeptide either in a sequence dependent way, i.e., via interaction with the bases of a RNA, comprising A, U, G and C, or in a sequence-independent manner, i.e., via interaction of the backbone phosphate of a gRNA nucleotide sequence with a CRISPR polypeptide.

According to certain embodiments of the present disclosure, the DNA target sequence is located within the genome of a cell, such as a prokaryotic or eukaryotic cell, a fungal, an animal or a plant cell (with embodiments of the present invention relating to a plant cell), wherein the genome comprises the nuclear genome as well as other genome parts, including the genome of plastids.

A "DNA target sequence" defines the genomic region, where a targeted genome editing is to be made. Due to the fact that the RTDD and the repair template nucleic acid sequence intrinsically have different functionalities, there can be a more than one DNA target region, which can be different for the different components of the artificial molecular complex of the present disclosure. One DNA target sequence thus may define the region of a DNA target region of interest a sequence portion of the RTDD the RTDD being a gRNA, is complementary to, whilst another DNA target sequence defines the region of a DNA target region of interest a SSN and/or an interaction domain will bind to. The at least one portion of the repair template nucleic acid sequence is complementary to is defined as genomic complementarity sequence, said sequence also representing a further DNA target sequence. The DNA target regions can be the same, or preferably different, yet possibly overlapping regions, within the DNA target sequence of interest.

The spatial relation between the target site of a RTDD and/or SSN and/or an interaction domain and the site of homology for the repair template nucleic acid sequence (RT) can be variable. The two sites can be identical, can be completely or partially overlapping, or can be separated by any number of nucleotides within the genome of interest. The RT can have homology to both strands of genomic DNA, optionally presented as double-stranded construct, for example a plasmid, or either strand individually, independent of which strand is targeted. An efficient repair template may be configured to have complementarity specific to the cleaved DNA strand first released by a SSN, for example a Cas9 (as described in Richardson, et al., Nature Biotechnology. 2016, doi:10.1038/nbt.3481).

The interaction between the RTDD and the RT and thus the close proximity of the SSN and the RT according to artificial molecular complex of the present disclosure is predicted to overcome the generally low efficiency of homology-directed repair (HDR)/homologous recombination (HR) as it guarantees the physical availability of the repair template nucleotide sequence present in a stochiometric way in relation to the at least one SSN *in situ* at the place a targeted genomic strand break is introduced by the at least one SSN polypeptide in a DNA target sequence.

The term repair template nucleic acid sequence (RT) as part of the artificial molecular complex according to the present disclosure thus implies a nucleotide sequence, which can be a single-stranded or double-stranded DNA sequence, which is capable of providing a template for modification and/or repair of a DNA break.

In one embodiment according to the present disclosure, the artificial molecular complex is an *in vitro* pre-assembled complex, wherein the SSN, the RTDD and the RT and optionally the interaction domain component or portion are provided either covalently attached to each other or non-covalently associated. In one embodiment, the RTDD/RT sequence is pre-assembled and the SSN and optionally the interaction domain is separately delivered into a target cell, either as transcribable DNA or translatable RNA construct or directly as amino acid sequence and the RTDD/RT sequence and the SSN and optionally the interaction domain form a complex within the target cell. In another embodiment, the RTDD/RT sequence as well as the SSN and optionally the interaction domain are assembled *in vitro* and the nucleoprotein complex optionally comprising further molecules, e.g., biotin or FAM, or digoxigenin, is then introduced into a target cell of interest or into an *in vitro* system comprising at least one DNA target nucleotide sequence of interest to be modified.

Introduction of a functional pre-assembled artificial molecular complex into a target cell results in a targeted double-strand break and simultaneous repair and site-specific modification due to the fact that the activity of the at least one site-specific nuclease (SSN) is immediately accompanied by the subsequent homologous recombination at the site of the DNA target sequence according to the present disclosure with the DNA repair template nucleic acid sequence linked to the RTDD, the RTDD also directly interacting with a SSN. Therefore, the drawbacks of poor availability of a RT or of unspecific NHEJ events (see Background of the Invention above) hampering a highly-specific and controllable genome editing event can be simultaneously reduced, as the artificial molecular complex can reach a target site in a coordinated way in an adequate stochiometric composition of repair template and nuclease.

A further benefit is that the potential for off-target integration of the repair template is reduced due to its physical association with the protein as well as the RTDD of the complex, wherein SSN and/or RTDD cannot be integrated into the genome *per se.*

The term "targeted homology directed repair" according to the present disclosure comprises any type of alterations that can be introduced by the repair template sequence according to the present application, which can independently comprise sequence insertions, edits of at least one sequence position, deletions or rearrangements, the preferable strategy for genome editing approaches in higher eukaryotes presently being insertions, deletions or edits, as these strategies allow the targeted knock-in or knock-out of a sequence of interest within a DNA target sequence, or a site-specific modification of at least one sequence.

An example for targeted homology directed repair as mediated by an artificial molecular complex using a CRISPR nuclease as SSN formed ex *vivo* or *in two* in cooperating with the hybrid nucleic acid sequence according to the present disclosure can be found in **Fig. 3 A to E** illustrating the chronological sequence of DNA recognition, binding, cleavage and subsequent repair for an exemplary SSN/guide nucleic acid (RTDD)/repair template (RT) complex and for a given endogenous DNA target sequence.

In one embodiment according to the various aspects of the present disclosure, the repair template nucleic acid sequence and/or the at least one RTDD sequence comprise a nucleotide sequence selected from a naturally or non-naturally occurring nucleotide sequence, including a synthetic nucleotide sequence, optionally comprising backbone and/or base modifications, wherein the guide nucleic acid sequence comprises a single-stranded, or partially single-stranded RNA nucleotide sequence, and wherein the repair template nucleic acid sequence comprises a single-stranded or a double-stranded DNA nucleotide sequence.

A challenge for any CRISPR genome editing approach is the fact that the gRNA and the functional CRISPR polypeptide as SSN have to be transported to the nucleus or any other compartment comprising genomic DNA, i.e., the DNA target sequence, in a functional (not degraded) way. As RNA is less stable than a polypeptide or double-stranded DNA and has a higher turnover, especially as it can be easily degraded by nucleases, in some embodiments according to the first aspect of the present disclosure, the gRNA as RTDD and/or the DNA repair template nucleic acid sequence comprises at least one non-naturally occurring nucleotide. Backbone modifications according to the present disclosure increasing the stability of the gRNA and/or the DNA repair template nucleic acid sequence are selected from the group consisting of a phosphorothioate modification, a methyl phosphonate modification, a locked nucleic acid modification, an 2'O-(2-methoxyethyl) modification, a di phosphorothioate modification, and a peptide nucleic acid modification. Notably, all said backbone modifications still allow the formation of complementary base pairing between two nucleic acid strands, yet are more resistant to cleavage by endogenous nucleases. Depending on the nuclease utilized according to the present invention, it might be necessary not to modify those nucleotide positions of a gRNA, which are involved in sequence-independent interaction with the CRISPR polypeptide. Said information can be derived from the available structural information as available for CRISPR nuclease/gRNA complexes.

In certain embodiments according to the first aspect of the present disclosure, it is envisaged that the RTDD and/or the DNA repair template RT nucleic acid sequence and/or the interaction domain comprise/comprises a nucleotide and/or base modification, for instance at selected, not all, nucleotide sequence positions. These modifications are selected from the group consisting of addition of acridine, amine, biotin, cascade blue, cholesterol, Cy3, Cy5, Cy5.5, Daboyl, digoxigenin, dinitrophenyl, Edans, 6-FAM, fluorescein, 3'-glyceryl, HEX, IRD-700, IRD-800, JOE, phosphate psoralen, rhodamine, ROX, thiol (SH), spacers, TAMRA, TET, AMCA-S", SE, BODIPY®, Marina Blue®, Pacific Blue®, Oregon Green®, Rhodamine Green®, Rhodamine Red®, Rhodol Green® and Texas Red®. For instance, said additions are incorporated at the 3'- or the 5'-end of a nucleic acid sequence used as RT and/or RTDD and/or interaction domain as part of the artificial molecular complex of the present disclosure. This modification has the advantageous effects, that the cellular localization of the RTDD and/or the interaction domain and/or the DNA repair template nucleic acid sequence within a cell can be visualized to study the distribution, concentration and/or availability of the respective sequence. Furthermore, the interaction of the artificial molecular complex of the present disclosure with endogenous molecules can be studied. Methods of studying such interactions or for visualization of a nucleotide sequence modified or tagged as detailed above are available to the skilled person in the respective field.

For any embodiment according to the various aspects of the present invention, the at least one site-specific nuclease and/or the at least one repair template nucleic acid sequence and/or the at least one interaction domain and/or the at least one RTDD comprises at least one nuclear localization sequence (NLS), a plastid localization sequence (PLS), preferably a mitochondrion localization sequence or a chloroplast localization sequence. Therefore, at least one of the components of the artificial molecular complex comprises a sequence to target the complex to the nuclear genome. In certain embodiments, also the RTDD can carry at least one localization sequence. Preferably, the SSN and/or the interaction domain of the artificial molecular complex will comprise at least one NLS or at least one PLS, or it will comprise both at least one NLS and at least one PLS sequence. This at least one NLS or PLS sequence will transport the entire artificial molecular complex tio the nucleus. NLS- or PLS-tagged proteins can be generated as NLS- or PLS-tagged fusion molecules.

For embodiments, wherein the artificial molecular complex according to the present disclosure is used for *in vitro* purposes, e.g., to modify a genome or part of a genome, on a plasmid or any other vector *in vitro,* no localization sequence might be necessary. Localization sequences help to target the artificial molecular complex to the at least one DNA target sequence of interest in the relevant compartment within a target cell of interest. According to certain embodiments of the present invention, the localization sequence can comprise a nuclear localization sequence, a plastid localization sequence, preferably a mitochondrion localization sequence or a chloroplast localization sequence. Therefore, the at least one SSN and/or the at least one RTDD and/or the at least one interaction domain will comprise at least one corresponding localization sequence, preferably a nuclear localization sequence (NLS) for directing the complex to the nuclear genome of cell. In some embodiments, the SSN and/or RT and/or the at least one interaction domain and/or the RTDD may comprise about or more than about 1, 2, 3, 4, 5, 6, 7, 8, 9, 10, or more NLSs at or near the aminoterminus (for peptides and proteins), about or more than about 1, 2, 3, 4, 5, 6, 7, 8, 9, 10, or more NLSs at or near the carboxyterminus (for peptides and proteins), or a combination of these (e.g., one or more NLSs at the amino-terminus and one or more NLSs at the carboxy terminus for peptides and proteins). Non-amino acid based components of the artificial molecular complex will carry the localization sequence, for example, on the 5'- and/or 3'-end, as it is the case for nucleic acid sequences. Furthermore, a localization sequence, preferably a synthetic localization sequence, can also be added at any position within a molecule provided that it will not disturb the interactions within the molecular complex and/or the binding, cleavage and repair capacity of the artificial molecular complexes of the present disclosure. When more than one NLS is present, each may be selected independently of the others, such that a single NLS may be present in more than one copy and/or in combination with one or more other NLSs present in one or more copies.

In an embodiment of the disclosure, the at least one SSN and/or the interaction domain will comprise a localization sequence and may comprise at most 6 NLSs. In some embodiments, an NLS is considered near the N- or C-terminus of an amino acid component of the artificial molecular complex when the nearest amino acid of the NLS is within about 1, 2, 3, 4, 5, 10, 15, 20, 25, 30, 40, 50, or more amino acids along the polypeptide chain from the N- or C-terminus. Non-limiting examples of NLSs include an NLS sequence derived from: the NLS of the SV40 virus large T-antigen, having the amino acid sequence PKKKRKV (SEQ ID NO: 1); the NLS from nucleoplasmin (e.g., the nucleoplasmin bipartite NLS with the sequence KRPAATKKAGQAKKKK (SEQ ID NO: 2)); the c-myc NLS having the amino acid sequence PAAKRVKLD (SEQ ID NO: 3) or RQRRNELKRSP (SEQ ID NO: 4); the hRNPA1 M9 NLS having the sequence NQSSNFGPMKGGNFGGRSSGPYGGGGQYFAKPRNQGGY (SEQ ID NO: 5); the sequence RMRIZFKNKGKDTAELRRRRVEVSVELRKAKKDEQILKRRNV (SEQ ID NO: 6) of the 188 domain from importin-alpha; the sequences VSRKRPRP (SEQ ID NO: 7) and PPKKARED (SEQ ID NO: 8) of the myoma T protein; the sequence PXPKKKPL (SEQ ID NO: 9) of human p53, wherein the "L" at position 8 of SEQ ID NO: 9 is optional; the sequence SALIKKKKKMAP (SEQ ID NO: 10) of mouse c-abl IV; the sequences DRLRR (SEQ ID NO: 11) and PKQKKRK (SEQ ID NO: 12) of the influenza virus NS1; the sequence RKLKKKIKKL of the Hepatitis virus delta antigen (SEQ ID NO: 13); the sequence REKKKFLKRR (SEQ ID NO: 14) of the mouse Mx1 protein; the sequence KRKGDEVDGVDEVAKKKSKK (SEQ ID NO: 15) of the human poly(ADP-ribose) polymerase; and the sequence RKCLQAGMNLEARKTKK (SEQ ID NO: 16) of the steroid hormone receptors (human) glucocorticoid. In some embodiments, the localization signal can be a plastid localization signal, for example a plastid or a mitochondria localization signal. Suitable plastid localization signals are selected from the group consisting of chloroplast transit peptides or mitochondrial targeting peptides. Furthermore, peptides derived from the HIV Tat protein, or sequences encoding the same, can be suitable for targeting a construct or molecule of interest into a cell and/or subcellular compartment of interest. Suitable Tat peptides are derived from YGRKKRRQRRR (SEQ ID NO: 17) or comprise the motif GRKKR (SEQ ID NO: 18). In another exemplary embodiment, a sequence derived from the Yeast mitochondrial Cox4p (SEQ ID NO: 30) or a sequence derived from the human malate dehydrogenase mitochondrial leader sequence (MLS) (SEQ ID NO: 31) or derived from the Arabidopsis Lipoic acid synthase (NCBI Ref. Seq. ID: NP 179682.1 designated herein as SEQ ID NO: 32) may be used to localize the artificial molecular complex according to the present disclosure into a mitochondrial matrix to modify mitochondrial DNA.

In particular embodiments it can be of interest to target the artificial molecular complex to the chloroplast. In many cases, this targeting may be achieved by the presence of an N-terminal extension, called a chloroplast transit peptide (CTP) or plastid transit peptide. Chromosomal transgenes from bacterial sources must have a sequence encoding a CTP sequence fused to a sequence encoding an expressed polypeptide if the expressed polypeptide is to be compartmentalized in the plant plastid (e.g., chloroplast). Accordingly, localization of an exogenous polypeptide to a chloroplast is often accomplished by means of operably linking a polynucleotide sequence encoding a CTP sequence to the 5' region of a polynucleotide encoding the exogenous polypeptide, i.e., the at least one SSN according to the present disclosure. The CTP is removed in a processing step during translocation into the plastid. Processing efficiency may, however, be affected by the amino acid sequence of the CTP and nearby sequences at the NH2 terminus of the peptide. Other options for targeting to the chloroplast which have been described are the maize cab-m7 signal sequence (U.S. Patent 7,022,896, WO 97/41228) a pea glutathione reductase signal sequence (WO 97/41228) and the CTP described in US 2009/029861 A1.

The various localization sequences according to the present disclosure can be encoded on a plasmid or expression cassette encoding the at least one localization sequence to operably link the localization sequence to the respective molecule, or the localization sequences can be attached to a protein, a nucleic acid or another biomolecule forming the artificial molecular complexes of the present disclosure in a synthetic way.

In yet a further embodiment, at least one of nuclear export signal can be used in addition to or instead of at least one localization sequence.

In embodiments, wherein the artificial molecular complex is delivered to a cell with the help of at least one delivery vector in the form of a nucleic acid sequence, the localization signal can be covalently attached to the at least one SSN and/or interaction domain encoding sequence in a covalent way as nucleic acid sequence encoding a localization signal.

In one embodiment, the at least one SSN and/or a polypeptide interaction domain can be covalently or non-covalently associated with a fluorescent reporter gene or protein. This reporter can be delivered as DNA, as mRNA, as an independent protein, or as a fusion protein linked to the at least one SSN and/or the interaction domain polypeptide.

The RTDD/RT molecule according to the present disclosure can be produced by several ways. It can be made by chemical synthesis, adding RNA bases where appropriate in the synthesis process and DNA bases where appropriate in the synthesis process. Alternatively, RTDD and/or RT can be synthesized independently of each other and the molecules can then be associated with each other as described above. Another option is to use T4 RNA ligase or another enzyme capable of ligating nucleic acids to RNA, such as single-stranded RNA. Here, the RNA and DNA components are generated independently by any method, mixed, exposed to the enzyme according to the manufacturer's protocol, and they will be covalently linked by ligation, i.e., to generate a covalent attachment. Other strategies for covalent bonding of the RTDD to the RT include linking each of them to other linking chemical groups or complexes, such as to a peptide. This type of approach is especially suitable, when the hybrid RTDD/RT sequence has to be detected later on within the cell, or when a further function should be attributed to the hybrid nucleic acid sequence. Chemical modification of either the RTDD and/or the RT nucleic acid sequence can be of great importance to stabilize the RTDD/RT sequence and to avoid degradation by cellular enzymes to achieve a high simultaneous availability of the RTDD/RT sequence and the at least one site-specific nuclease at the DNA target site of interest.

In embodiments of the present disclosure, wherein the RTDD is a gRNA and the SSN is a CRISPR nuclease, such as a Cas or a Cpf1 nuclease, more than one RTDD can be present. It has been found that using multiple gRNAs simultaneously will augment CRISPR based gene activation or repression and can significantly reduce the emergence of alleles resistant to gene drives. Therefore, the gRNAs as RTDDs can be presented as single unprocessed transcript and the gRNAs will then be excised from the precursor in the nucleus by RNA polymerase II transcription simultaneously obviating the export of gRNA to the cytoplasm (Port and Bullock, Nat. Methods, 2016, vol. 13, no.10, 852-854). In those embodiments, the gRNAs can be presented as tRNA-gRNA plasmids so that the endogenous tRNA processing machinery will liberate multiple functional gRNAs.

According to certain embodiments of the various aspects of the present disclosure/invention, the at least one site-specific nuclease, or the sequence encoding the same, and the at least one interaction domain, or the sequence encoding the same, and/or the at least one repair template docking domain, or the sequence encoding the same, are connected by at least one linker domain. This linker sequence can serve as molecular spacer to achieve optimum geometry of the RTDD sequence and the repair template nucleic acid sequence as well as the SSN and optionally the interaction domain component of the artificial molecular complex so that all individual components can fully exert their function. The length and composition of the linker or tether regions may be an important design aspect, e.g., for certain RTDD and RT pairs. In one embodiment, especially the 5'-end of the left homology arm of the RT can comprise a linker region. The tether or linker can take a variety of forms. Starting from the left or right homology arm of the RT, allowing this portion of the RT to act as a tether or flexible linker to allow movement of the RT toward the chromosomal target, and as homology to mediate the HR reaction can be performed by the skilled person based on the present disclosure and having knowledge of usual design parameters for repair templates as presently widely used for genome editing.

In embodiments, wherein the artificial molecular complex comprises at least one SSN as well as at least one interaction domain (IA), the SSN and the IA can be connected by a suitable linker.

Design parameters to be considered include geometry of the repairtemplate homology relative to the cut site of a CRISPR polypeptide as SSN, the strand within a DNA target site of interest to which the repair template is homologous, size of the repair template, which can influence, whether a linker and in which length a linker will be introduced. A linker sequence can be used for both covalent and non-covalent associations of the gRNA and the repair template. Based on the present disclosure and based on the information provided in Nishimasu (supra), Tsai et al. (Nature Biotechnology, 32, 569-576, (2014)), or Shechner et al. (Nature Methods, 12(7), 664-670 (2015), doi:10.1038/nmeth.3433), the skilled person can thus define a suitable linker region for a hybrid nucleic acid sequence to define a specific sequence between the gRNA as RTDD and the RT or between different gRNAs and or RTs, in case several hybrid nucleic acids are used so that both the gRNA and the RT moiety can fully exert their function without any sterical constraints. The at least one linker region can comprise up to 10, 15, 20, 25, 30, 35, 40, 45, 50, 55, 60, 65, 70, 75, 80, 85, 90, 95 or 100 additional nucleotides to properly separate the at least one gRNA from the RT, or to optimize the positioning of the gRNA and/or the RT. In certain embodiments, the linker sequence can comprise up to 150, 200, 250, 500, 1,000, 1,500, 2,000, 2,500, 3,000, 3,500, 4,000, 4,500 or at least 4,700 or 5,000 nucleotides to achieve a better positioning of the gRNA and/or the RT.

For non-covalent association, according to the present disclosure, of any RTDD comprising nucleic acid sequences with the RT, one approach is to provide partially complementary sequences in the RTDD and RT so that the two molecules will naturally associate by nucleic acid base pairing.

Other methods of non-covalent association are conceivable, such as the use of electrical charges of molecules to cause a sufficient association of the RT with some component of the artificial molecular editing complex. In another embodiment, at least one component of the artificial molecular complex, can comprise a tag and the binding partner, i.e., the RTDD and the SSN, or the RTDD and the interaction domain, and/or the RT portion thereof, respectively, comprise the corresponding binding partner of the tag so that a non-covalent interaction, optionally in addition to the base pairing between RTDD or the interaction domain and RT and the association between RTDD and the SSN polypeptide is achieved to increase the interaction and thus stability of the artificial molecular complex.

In human cells, Cas9 loaded with a gRNA possessing 28 bp of additional sequence on the 3'-end plus an associated 187 amino acid (21.4 kD) Csy4 protein maintained at least 90% activity in DSB induction compared to standard gRNA controls (Tsai et al., Nature Biotech., 32, 2014). This suggests a fairly substantial tolerance by Cas9 as SSN for cargo tethered to the 3'-end of the sgRNA and of proper structure-function potential for the extended sgRNA molecule. Cas9 tolerance is enabled in part by the flexibility of the free 3'-end of the nucleic acid sequence, which in a standard gRNA terminates in a hairpin that is held outside the architecture of the Cas9 protein and on a surface roughly perpendicular to the surface holding the active site (Nishimasu et al., 2014; Anders et al., 2014). Furthermore, Shechner et al. ("Multiplexable, locus-specific targeting of long RNAs with CRISPR-Display", Nature Methods, 12(7), 664-670 (2015), doi:10.1038/nmeth.3433) show that long noncoding ssRNA molecules can be transcriptionally attached to the 5'- or 3'-ends of the sgRNA, or in an internal loop of the sgRNA without loss of sequence-specific targeting activity by a dCas9 protein in the human cell genome. ssRNAs of up to 4.8 kb were accommodated by the ribonucleoprotein complex with maintenance of sequence-specific targeting activity.

In one embodiment according to the various aspects of the present disclosure, the repair template nucleic acid sequence is associated with the RTDD, for example a guide nucleic acid sequence, at the 3'-end of the guide nucleic acid sequence, and/or the repair template nucleic acid sequence is associated with the 5'-end of the RTDD, for example a guide nucleic acid sequence, and/or the repair template nucleic acid sequence is located within the RTDD and thus forms a separate functional part of the RTDD.

Surprisingly, it was found by the inventors of the present invention that a gRNA as RTDD carrying a 3' located DNA repair template sequence (RT), either a single-stranded or a double-stranded RT, was free to interact with homologous sequence as it is delivered to the target by a CRISPR polypeptide, e.g., Cas9 from a CRISPR Type II system, or Cpf1 from a CRISPR Type V system or another CRISPR polypeptide effector. Similar observations were made when a gRNA as RTDD carrying a 5' located DNA repair template sequence, either a single-stranded or a double-stranded RT, or both, a 3' and 5' located RT, were used. 3' or 5' located thus implies that the RT is either covalently attached to the 3'- or 5'-end of a gRNA, or it can mean that the RT is hybridized to, i.e., non-covalently associated with, a region corresponding to sequence attached to the 3' and/or the 5' region of the gRNA. In addition, the RT could be covalently incorporated in the stem loops of a gRNA, or it could be non-covalently be associated with said gRNA stem loops to achieve a functional hybrid nucleic acid construct, wherein the RTDD and the RT are directly interacting. Thus, it was found that DNA associated with a gRNA at various positions of the gRNA as described above was well tolerated and this new form of hybrid complex, therefore, is suitable to bring together two key aspects of the gene editing principle: (1) precision of targeting mediated by the RTDD/gRNA and (2) efficient and site-directed repair as mediated by the RT. Furthermore, there is the synergistic effect that gRNA and RT are brought into close proximity to increase the stability and the availability of the hybrid construct together with a CRISPR polypeptide as SSN of interest at a DNA target site of interest.

There are nearly no limitations on the length of this extended repair template nucleotide sequence delivered as part of the artificial molecular complex according to the present disclosure, in case the RT is attached to the 3'- or the 5'-end of a nucleic acid based RTDD, e.g., a gRNA or a gDNA. The length of the RT, independent of the kind of RTDD, is rather dictated by the targeted modification to be introduced. Typical RT sequences can have a length from about 20 to 8,000 bp or even more, e.g., of 20 to 5,000 bp, of 30 to 8,000 bp, of 30 to 5,000 bp, of 40 to 8,000 bp, of 40 to 5,000 bp, of 50 to 8,000 bp, of 50 to 5,000 bp, of 60 to 8,000 bp, of 60 to 5,000 bp, of 70 to 8,000 bp, of 70 to 5,000 bp, of 80 to 8,000 bp, of 80 to 5,000 bp, of 90 to 8,000 bp, of 90 to 5,000 bp, of 100 to 8,000 bp, of 100 to 5,000 bp of single-stranded and/or double-stranded DNA without a significant loss in cutting frequency of at least one SSN is observed. As it is known to the skilled person, the length of a RT template is strongly dictated by the kind of modification/insertion to be effected/introduced. In case a knock-in of a larger nucleic acid sequence encoding a protein of interest is intended, the length of the RT sequence will have the length: length of the nucleic acid construct encoding the protein of interest plus two sufficiently long homology arms located left and right of the sequence. Thus, there is in principle no upper limit of 1,500 bp, but the RT can have up to 5,000 or even more base pairs (bp). For example, larger inserts presently introduced using a plasmid DNA as repair template and producing the repair template within a target site use left and right homology arms of 800 bp and more so that the total length of a repair template can have several 1,000 bp. The length of the nucleic acid inserts should be designed not to inhibit the site-specific nuclease of interest which can be determined in preexperiments.

The different components of the molecular complex of the present disclosure, i.e., the at least one SSN, the at least one RTDD and the at least one RT, and optionally at least one interaction domain are associated in a functional way.

The term "associated in a functional way" implies that the components of the artificial complex are brought into contact so that the SSN and the RTDD can interact with each other, for instance by a form of non-covalent association as detailed above. The at least one RTDD sequence interacting with at least one RT sequence are independently assembled, either before, after, or simultaneously with contacting the at least RTDD sequence with the at least one corresponding SSN, or a variant or a catalytically active fragment thereof of interest. In one embodiment, the whole complex, optionally comprising at least one interaction domain, is associated *in vitro* before it is introduced into a target cell comprising at least one DNA target region of interest to be edited. In another embodiment, the at least one SSN and optionally an interaction domain is introduced into the at least one target cell before or after the at least one interacting RTDD/RT sequence. The SSN polypeptide can be introduced into a target cell by means of transfecting the polypeptide sequence or by transfecting or transforming at least one target cell with RNA encoding the at least one SSN polypeptide or by introducing a delivery construct encoding at least one SSN polypeptide which can be transcribed and translated in a target cell. Likewise, in certain embodiments, the RTDD sequence(s) and the repair template nucleic acid sequence(s) can be provided simultaneously as *in vitro* provided and assembled construct. Alternatively, either the RTDD sequence and/or the repair template nucleic acid sequence can be transfected or transformed into a target cell with the help of a suitable delivery vector. In a preferred embodiment, the whole artificial molecular complex is assembled *in vitro* and then introduced into a target cell of interest to allow best spatial and stochiometric control of the genome editing construct. In another preferred embodiment, the at least one SSN and optionally an interaction domain polypeptide is introduced into a target cell before the RTDD/RT sequences and the at least one RTDD/RT sequences are then introduced into a target cell of interest afterwards. The sequential order might be desirable for certain approaches using, for example a gRNA as RTDD due to the intrinsically low stability of RNA in comparison to a polypeptide, so that the introduced gRNA will be immediately bound and stabilized by the SSN, i.e., for certain embodiments a CRISPR polypeptide already present in the cell. Without wishing to be bound by theory, the ex *vivo* assembly of a guide nucleic acid sequence and a repair template nucleic acid sequence can also enhance the stability of the construct in comparison to the guide RNA alone.

Currently, there exists a variety of plant transformation methods to introduce genetic material in the form of a genetic construct into a plant cell of interest, comprising biological and physical means known to the skilled person on the field of plant biotechnology. A common biological means is transformation with *Agrobacterium spp.* which has been used for decades for a variety of different plant materials. Viral vector mediated plant transformation represents a further strategy for introducing genetic material into a cell of interest. Physical means finding application in plant biology are particle bombardment, also named biolistic transfection or microparticle-mediated gene transfer, which refers to a physical delivery method for transferring a coated microparticle or nanoparticle comprising a nucleic acid or a genetic construct of interest into a target cell or tissue. Physical introduction means are suitable to introduce nucleic acids, i.e., RNA and/or DNA, and proteins. Likewise, specific transformation or transfection methods exist for specifically introducing a nucleic acid or an amino acid construct of interest into a plant cell, including electroporation, microinjection, nanoparticles, and cell-penetrating peptides (CPPs). Furthermore, chemical-based transfection methods exist to introduce genetic constructs and/or nucleic acids and/or proteins, comprising *inter alia* transfection with calcium phosphate, transfection using liposomes, .e.g., cationic liposomes, or transfection with cationic polymers, including DEAD-dextran or polyethylenimine, or combinations thereof. Said delivery methods and delivery vehicles or cargos thus inherently differ from delivery tools as used for other eukaryotic cells, including animal and mammalian cells and every delivery method has to be specifically fine-tuned and optimized so that a construct of interest for mediating genome editing can be introduced into a specific compartment of a target cell of interest in a fully functional and active way. The above delivery techniques, alone or in combination, can be used to insert the at least one artificial molecular complex, or at least one subcomponent thereof, i.e., at least one SSN, at least one RTDD, at least one RT and optionally at least one IA, or the sequences encoding the aforementioned subcomponents, according to the present disclosure into a target cell, *in vivo* or *in vitro*.

In certain embodiments, modes of delivery of the artificial molecular complex of the present disclosure can be selected from PEG mediated delivery of a SSN-(IA)-RTDD-RT complex, PEG mediated delivery of plasmid encoding SSN-(IA)-RTDD, the RTDD for example being a gRNA or gDNA and parallel delivery of RT, bombardment of a SSN-(IA)-RTDD-RT complex, bombardment of plasmid encoding protein (SSN and optionally IA)-RTDD, for example gRNA/gDNA, and parallel delivery of RT, cellpenetrating peptide (CPP) mediated delivery of a SSN-(IA)-RTDD-RT complex, lipofection of a SSN-(IA)-RTDD-RT complex, lipofection of plasmid encoding protein (SSN and optionally IA)-RTDD, for example gRNA/gDNA, and parallel delivery of RT, or stable expression of protein (SSN and optionally IA) and transient delivery of the RTDD or for certain RTDDs a plasmid encoding the RTDD and parallel delivery of rtDNA.

In certain embodiments, the crRNA portion of the gRNA comprises a stem loop or an optimized stem loop structure or an optimized secondary structure. In another embodiment the mature crRNA comprises a stem loop or an optimized stem loop structure in the direct repeat sequence, wherein the stem loop or optimized stem loop structure is important for cleavage activity. In certain embodiments, the mature crRNA preferably comprises a single stem loop. In certain embodiments, the direct repeat sequence preferably comprises a single stem loop. In certain embodiments, the cleavage activity of the effector protein complex is modified by introducing mutations that affect the stem loop RNA duplex structure. In preferred embodiments, mutations which maintain the RNA duplex of the stem loop may be introduced, whereby the cleavage activity of the effector protein complex is maintained. In other preferred embodiments, mutations which disrupt the RNA duplex structure of the stem loop may be introduced, whereby the cleavage activity of the effector protein complex is completely abolished.

The size of the at least one repair template nucleic acid sequence according to the present disclosure as part of the artificial molecular complex according to the present disclosure can vary. It can be in the range from about 20 bp to about 5,000 bp or even 8,000 bp depending on the DNA target sequence to be modified.

HOR templates used to create specific mutations or insertions into a DNA target region of interest require a certain amount of homology surrounding the target sequence that will be modified. It is best if the insertion sites of the modification are no more than 100 bp away from the DSB as effected by a SSN or a fusion partner, i.e., an interaction domain, in the case of a nuclease deficient SSN, for example a CRISPR polypeptide, ideally less than 10 bp away if possible, and the overall length of the homology arm is an important factor to consider when designing these. Longer distances will work, but the efficiency will likely be lower and the introduction of a selection marker might become necessary to ensure that the desired modification to be introduced into the DNA target sequence of interest is present.

According to the various aspects of the present invention, the at least one repair template nucleic acid sequence can be a single-stranded or double-stranded DNA nucleic acid molecule. The at least one repair template nucleic acid sequence can be provided in the form of one or more linear, ss- or ds-DNA molecules. However, it might be suitable to use at least one single-stranded or double-stranded repair template nucleic acid sequence, which is produced ex *vivo,* when a molecular complex is to be assembled ex *vivo,* which is especially suitable to increase the availability of the functional SSN-RTDD-RT complex, as all components can be introduced simultaneously in the correct stochiometry to increase the specificity of the genome editing approach.

The synthesis of larger nucleic acid sequences, either single- or double-stranded, can be accomplished using common prior art methods. It is noted that for certain embodiments, also partial single-stranded and/or partial double-stranded repair template nucleic acid sequences might be suitable. Any combination of a single-stranded and/or double-stranded nucleic acid sequence and any kind of introduction, either simultaneous with or before or after the introduction of the polypeptide components of the artificial molecular complex is possible. In one embodiment, it is envisaged to introduce a molecular complex according to the second aspect of the present disclosure into a target cell, wherein the target cell comprises an additional plasmid vector encoding a repair template or an additional repair template sequence, as the use of more than one repair template nucleic acid sequence is beneficial for certain genome editing approaches, wherein the artificial molecular complex can then assemble in *two* after the different components are provided. In general, high physical availability of the repair template nucleic acid sequence at that site within a target cell, where the DNA target region is located is of outstanding importance to allow for a highly precise genome editing event. In certain embodiments, especially single-stranded (ss) DNA repair templates are suitable to strike the right balance keeping the molecular weight as low as possible while providing sufficient length for homology interactions to achieve optimum homology directed repair.

In one embodiment according to any aspect of the present invention, the at least one SSN is a CRISPR polypeptide and is independently selected from the group consisting of a Cas polypeptide of *Streptococcus spp.,* including *Streptococcus pyogenes, Streptococcus thermophilus, Staphylococcus aureus,* or *Neisseria spp.,* including *Neisseria meningitides, Corynebacter, Sutterella, Legionella, Treponema, Filifactor, Eubacterium, Lactobacillus, Mycoplasma, Bacteroides, Flaviivola, Flavobacterium, Sphaerochaeta, Azospirillum, Gluconacetobacter, Roseburia, Parvibaculum, Nitratifractor, Mycoplasma* and *Campylobacter, Candidatus* Micrarchaeum acidiphilum ARMAN-1, *Parcubacteria* (GenBank: APG80656.1), *Sulfolobus* spp., including *Sulfolobus islandicus* HVE10/4 (GenBank: ADX81770.1) or REY15A (GenBank: ADX84852.1), or wherein the CRISPR polypeptide is selected from a Cpf1 polypeptide from an archaea or a bacterium, including a Cpf1 polypeptide of *Acidaminococcus spp.,* including *Acidaminococcus sp.* BV3L6, *Lachnospiraceae spp.,* including *Lachnospiraceae bacterium* ND2006, *Francisella spp.,* including *Francisella novicida* U112, *Eubacterium eligens, Prevotella spp.,* or *Porphyromonas spp.,* or variants and/or functional fragments and/or combinations thereof, including CRISPR polypeptide nickases, or a CRISPR polypeptide lacking endonucleolytic activity.

In one embodiment according to the present invention, the RTDD/RT sequences according to the present disclosure can be used with a SSN nickase, e.g., a Cas9 nickase, mutant to minimize off-target mutations, wherein paired guide RNAs are used, each of which is specific for a Cas9 derived nickase mutant.

In some embodiments, the at least one SSN and optionally the at least one interaction domain is provided as *in vitro* expressed, translated or synthesized polypeptide. In some embodiments, a delivery vector is used encoding at least one CRISPR polypeptide, wherein the delivery vector can additionally comprise regulatory sequences or localization signals. A SSN polypeptide that is mutated with respect to a corresponding wild-type enzyme such that the mutated SSN enzyme lacks the ability to cleave one or both strands of a target polynucleotide containing a target sequence also comprised by various embodiments according to the present disclosure. For example, an aspartate-to-alanine substitution (D10A) in the RuvC I catalytic domain of Cas9 from S. *pyogenes* can be used which converts Cas9 from an endonuclease that cleaves both strands of a DNA target region of interest to a nickase cleaving a single-strand. Other examples of mutations that render a Cas9 polypeptide a nickase include, without limitation, H840A, N854A, and N863A. As a further example, two or more catalytic domains of Cas9 (RuvC I, RuvC II, and RuvC III or the HNH domain) may be mutated to produce a mutated Cas9 substantially lacking all DNA cleavage activity. In some embodiments, a D10A mutation is combined with one or more of H840A, N854A, or N863A mutations to produce a Cas9 enzyme substantially lacking all DNA cleavage activity. In some embodiments, a SSN enzyme is considered to substantially lack all DNA cleavage activity when the DNA cleavage activity of the mutated enzyme is about no more than 25%, 10%, 5%, 1%, 0.1%, 0.01%, or less of the DNA cleavage activity of the non-mutated form of the enzyme; an example can be when the DNA cleavage activity of the mutated form is null or negligible as compared with the non-mutated wild-type form. Where the enzyme is not Cas9 from S. *pyogenes,* mutations may be made at any or all residues corresponding to positions 10, 762, 840, 854, 863 and/or 986 of *Sp*Cas9 (which may be ascertained for instance by standard sequence comparison tools). In particular, any or all of the following mutations are preferred in Cas9 from S. *pyogenes:* D10A, E762A, H840A, N854A, N863A and/or D986A; as well as a conservative substitution for any of the replacement amino acids is also envisaged according to the present disclosure. The same or conservative substitutions of these mutations at corresponding positions in other Cas9s are also possible for certain embodiments, particularly D10 and H840 in Cas9 from S. *pyogenes.* However, in other Cas9s, residues corresponding to D10 and H840 Cas9 from S. *pyogenes* are also possible. "Orthologs" or "orthologous" of given CRISPR proteins can also be used in the practice of the invention. Orthologs are genes in different species that evolved from a common ancestral gene by speciation. Normally, orthologs retain the same function in the course of evolution. Most preferably, the Cas9 enzyme as SSN is from, or is derived from, S. *pyogenes* Cas9, or S. *aureus* Cas9, or wild-type Cas9 from S. *thermophilus,* the protein sequence of which is give in the SwissProt database under accession number G3ECR1. Similarly, S. *pyogenes* Cas9 or S. *aureus* Cas9 is included in SwissProt under accession number Q99ZW2.

In one embodiment of the present disclosure, the guide RNA as RTDD sequence according to the present disclosure is designed for having optimal activity, i.e., recognition properties, towards a selected SSN enzyme or polypeptide of a specific length, the SSN enzyme can thus be truncated to a catalytically active fragment of the wild-type SSN making it smaller in length than the corresponding wild-type enzyme by truncating the nucleic acid molecules coding for the SSN enzyme which can be transcribed or translated *in vitro* or *in vivo,* or by providing a synthesized SSN polypeptide. Generating chimeric SSN enzymes, wherein different parts of the enzyme are swapped or exchanged between different orthologs to arrive at chimeric enzymes having tailored specificity is also possible.

A "variant" or "functional fragment" according to the present disclosure thus comprises any SSN and/or interaction domain and/or RTDD protein or a truncated version thereof derived from the wild-type SSN and/or interaction domain and/or RTDD protein, i.e., having a degree of sequence homology with, a wild-type enzyme, but that it has been mutated (modified) in some way as described herein. For example, enzymatic activity by Cas9 derived nuclease generates double-stranded breaks at target site sequences which hybridize to 20 nucleotides of the guide sequence and that have a protospacer-adjacent motif (PAM) sequence examples including NGG/NRG or a PAM that can be determined as described herein following the 20 nucleotides of the target sequence. This enzymatic function can be varied by generating SSN variants having nickase activity or nuclease dead variant. Furthermore, a SSN and/or interaction domain and/or RTDD polypeptide variant according to the present disclosure can be codon-optimized to adapt the SSN and/or interaction domain and/or RTDD polypeptide to the codon usage of a target cell, such as a eukaryotic cell, an animal or a plant cell.

In preferred embodiments according to the present invention, the components of the artificial molecular complex, particularly the SSN or IA components, or the catalytically active fragments thereof still exerting the catalytic function of the wild-type polypeptide, and/or the further components can be codon optimized, and/or the SSN polypeptide and/or the interaction domain and/or the RTDD and/or the RT can be linked to a tag sequence, to identify the location of a target sequence and/or the artificial molecular complex. The tag can be selected from the group consisting of a polyhistidin(His)-Tag, a glutathione-S-transferase (GST)-tag, a thioredoxin-tag, a FLAG-tag, a tag having fluoresecent properties, for example, selected from (E)GFP ((enhanced) green fluorescent protein) tag, a DsRed-tag, a mCherrytag, a (t)dtomato-tag, an mNeonGreen-tag and the like or, a streptavidin or strep-tag, a maltose-binding protein (MBP) tag, a transit peptide allowing the targeting to a subcellular compartment, including mitochondria or the nucleus, a snap-tag and/or a secretion tag allowing the secretion of an amino acid sequence attached thereto, a non-natural amino acid not normally occurring in nature, or a combination of the aforementioned tags. A protein component of the artificial molecular complex, for example the SSN and/or the interaction domain, may comprise any additional protein sequence, and optionally a linker sequence between any two domains. Examples of protein domains that may be fused to any component of the at least one artificial molecular complex include, without limitation, epitope tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. Non-limiting examples of epitope tags include histidine (His) tags, V5 tags, FLAG tags, influenza hemagglutinin (HA) tags, Myc tags, VSV-G tags, and thioredoxin (Trx) tags. Examples of reporter genes include, but are not limited to, glutathione-S-transferase (GST), horseradish peroxidase (HRP), chloramphenicol acetyltransferase (CAT) beta-galactosidase, beta-glucuronidase, luciferase, green fluorescent protein (GFP), HcRed, DsRed, cyan fluorescent protein (CFP), yellow fluorescent protein (YFP), and autofluorescent proteins including blue fluorescent protein (BFP). A CRISPR enzyme may be fused to a gene sequence encoding a protein or a fragment of a protein that bind DNA molecules or bind other cellular molecules, including but not limited to maltose binding protein (MBP), S-tag, Lex A DNA binding domain (DBD) fusions, GAL4 DNA binding domain fusions, and herpes simplex virus (HSV) BP16 protein fusions.

In one embodiment, at least one component of the artificial molecular complex can be a modified functioning as DNA nickase, and/or the SSN polypeptide, or the catalytically active fragment thereof, can be present in the form of a fusion molecule with another functional moiety, preferably a functional polypeptide moiety having enzymatic function, preferably a functional moiety having chromatin modeling function, and/or stimulating homologous recombination, and/or modifying transcription. When analyzing at least one modified cell within a tissue of a multicellular organism, such tags and marker proteins, especially fluorescent protein tags, are preferred which have a bright fluorescence so that they can be even be determined in deeper layers of complex tissues. Suitable fluorescent proteins are commercially available and can be easily selected for the specific purpose by the skilled person.

According to the various embodiments of the present invention, either the SSN and/or interaction domain and/or RTDD polypeptide(s) and/or the RTDD and/or RT sequence(s) comprises at least one nuclear localization sequence, and/or a plastid localization sequence, for example a mitochondria localization sequence or a chloroplast localization sequence, for efficient targeting of the SSN polypeptide to a cellular compartment comprising a genomic DNA sequence of interest to be modified. Sequence requirements for such localization sequences are known to the skilled person in the field of molecular biology. Not to hamper the function of the SSN polypeptide or of the RT nucleotide sequence, the localization sequence is fused, i.e., covalently linked, to the N-terminal or C-terminal part, or correspondingly the 5'-or 3'-end of the respective molecule.

In one embodiment, the at least one SSN polypeptide and optionally the at least one interaction domain, if representing a polypeptide sequence, is provided as polypeptide sequence produced *ex vivo,* either using recombinant technologies for protein production or via synthesis of the corresponding amino acid sequence. In another embodiment, the SSN polypeptide and optionally the at least one interaction domain is presented as RNA sequence, which can be translated to the corresponding amino acid sequence upon introduction of a target cell of interest. In yet a further embodiment, the SSN polypeptide and optionally the at least one interaction domain polypeptide is inserted as DNA construct, either configured for stable expression or for transient expression in a cell of interest, so that the at least one SSN polypeptide and optionally the at least one interaction domain polypeptide is then transcribed and translated in a target cell of interest in a constitutive or inducible way. Suitable DNA constructs and associated methods for introducing a at least one SSN polypeptide and optionally the at least one interaction domain polypeptide according to the present disclosure into a target cell are known to the skilled person, whereas specific ways of introducing a at least one SSN polypeptide and optionally the at least one interaction domain polypeptide according to certain embodiments of the present disclosure specifically adapted for the application in plant cells are further detailed below.

The artificial molecular complex, or the parts thereof, i.e., the at least one SSN polypeptide, the at least one RTDD and the at least one RT and optionally the at least one interaction domain have to be introduced into a target cell of interest using a suitable delivery construct. Naturally, the type of delivery construct can vary, depending on the fact whether the molecular complex is fully assembled *in vitro* and later on introduced into a target cell, or whether the different components of the molecular complex are separately introduced into a cell and the complex is then assembled by non-covalent interactions within a target cell of interest. Introduction usually takes place by using a suitable delivery construct.

The term "delivery construct" or "(delivery) vector" as used herein according to various embodiments of the different aspects of the present invention refers to any biological or chemical, or non-chemical or particle-based means and/or methods used as a cargo for transporting a nucleotide and/or an amino acid sequence of interest into a target eukaryotic cell. Suitable delivery constructs comprise biological means for delivering nucleotide sequences into a target cell, including viral vectors, *Agrobacterium spp.,* cell-penetrating peptides (CPPs) or chemical delivery constructs, including nanoparticles, lipid or polymeric vesicles, calcium phosphate, or combinations thereof. Lipid or polymeric vesicles may be selected, for example, from lipids, liposomes, lipid encapsulation systems, nanoparticles, e.g., mesoporous silica nanoparticles, small nucleic acid-lipid particle formulations, polymers, e.g., cationic polymers like DEAE-dextran or polyethylenimine and polymersomes. In one embodiment, the polymer is selected from the group consisting of linear polymers, branched polymers, dendrimers (highly branched organic compounds), and polysaccharides. In another embodiment, the lipid encapsulation system comprises one or more of a phospholipid, cholesterol, polyethylene glycol (PEG)-lipid, and a lipophilic compound that delivers the particle to the target tissue. In a further embodiment, the delivery construct can be a mesoporous silica nanoparticle.

Physical introduction methods as used herein and as suitable for providing at least one molecular complex or at least one hybrid RNA/DNA nucleic acid sequence according to the present disclosure refer to electroporation, microinjection, particle bombardment, sonoporation, magnetofection or impalefection using elongated nanostructures and arrays of such nanostructures such as carbon nanofibers or silicon nanowires which have been functionalized with plasmid DNA, and chemical methods and can rely on the use of micro- or nanoparticles or chemicals, including polyethylenglycol (PEG).

For example, for an embodiment, where the components of the artificial molecular complex are associated *ex vivo,* the delivery vector can be a lipid-based or a polymeric vector. Lipid-based or polymeric vectors may be selected, for example, from lipids, liposomes, lipid encapsulation systems, microparticles, whiskers, nanoparticles, small nucleic acid-lipid particles, polymers, and polymersomes. In some embodiments, the polymer can be selected from the group consisting of linear polymers, branched polymers, dendrimers, and polysaccharides. In another embodiment, the lipid encapsulation system comprises one or more of a phospholipid, cholesterol, polyethylene glycol (PEG)-lipid, and a lipophilic compound that delivers the particle into a target cell.

For mammalian cells, *ex vivo* modification of immune cells for various therapeutic purposes has gained a lot of interest during the last decade to combat several tumor diseases by adoptively transferring specifically modified lymphocytes, such as T-cells. Especially CD8+ T-cell lymphocytes are interesting targets in this regard. It was described that immune responses derived from single naive T cells, single primary, and single secondary central memory T cells reached similar size and phenotypic diversity, were subjected to comparable stochastic variation, and could ultimately reconstitute immunocompetence against an otherwise lethal infection with a bacterial pathogen as measured by *in vivo* fate mapping of CD8+ T cells and their descendants across three generations of serial single-cell adoptive transfer and infectiondriven re-expansion (Graef et al., Immunity, 41, 116- 126, 2014). After *de nova* thymic T-cell development from hematopoietic cells fully mature antigen-specific T-cells can be maintained over extensive periods of time in an individual, wherein the antigen can be a foreign antigen, e.g., an antigen expressed on a virus or a cancer cell. Targeted modification of such effector T-cells, or the precursors thereof, thus represents an important strategy to provide suitable T-cells for immunotherapy. Naive T cells differentiate through a stage called stem cell memory T-cells, which give rise to central memory T-cells and effector memory T-cells and finally effector T-cells, wherein the effector T-cells represent terminally differentiated cells which can ultimately recognize and destroy a target cell. Effector memory and effector T-cells are the subsets of T-cells that have the capacity to traffic to peripheral tissues. Another subset, tissueresident memory T-cells are presently suggested, which do not circulate any more (cf., e.g., Farber et al., Nature Reviews Immunology, 14, 24-35, 2014).

Furthermore, immunotherapy of cancers has provided some of the first spectacular clinical cases showing that adoptive transfer of T-cells expressing recombinant tumor-reactive receptors can cure otherwise treatment-resistant malignancies (Brentjens et al., 2013; Grupp et al., 2013; Porter et al., 2011) and that the use of engineered T-cells in adoptive transfer therapies has shown significant promise in treating cancers, particularly haematological cancers. More and more, genetically modified T-cells of defined subset and phenotypic composition are used to increase cancer immunotherapy success (see Riddell et al., Cancer J., 20(2), 141-144, 2014). The use of chimeric antigen receptor-modified T-cells as a therapy for hematologic malignances and also for solid tumors is becoming more widespread. To this end, T-cells are modified to express tumor-directed chimeric antigen receptors (CARs) (see e.g., Anurathapan et al., Molecular Therapy, 22, 623-633, 2014). Also so-called second generation CARs, e.g., CD19-targeted CARs that incorporate CD2B or 4-1 BB signalling domains, for retargeting and reprogramming T-cells to augment their antitumor efficacy are becoming more and more important (see e.g., Sjoukje et al., Nature Reviews Drug Discovery, 14, 499-509, 2015).

Therefore, the hybrid RNA/DNA nucleic acid sequences according to the present disclosure represent an important tool to modify one or more mammalian cells *in vivo* or *ex vivo,* for instance for the treatment of a disease. For example, a lymphocyte cell, such as a T-cell or natural killer (NK) cell of any developmental stage to alter a T-cell or NK-cell expressed gene to influence T-cell or NK-cell proliferation, survival and/or function with high precision to avoid off-target effects, which could be detrimental for a therapeutic application of the modified cell or cell population.

In certain embodiments, the artificial molecular complex according to the present disclosure is thus suitable for use in a method of treatment a disease, wherein the disease is characterized by at least one genomic mutation and the artificial molecular complex is configured to target and repair the at least one genomic mutation. There is thus provided a method of treating a disease using the artificial molecular complex according to any one of the preceding claims, wherein the disease is characterized by at least one genomic mutation and the artificial molecular complex is configured to target and repair the at least one genomic mutation. The therapeutic method of treatment may comprise gene or genome editing, or gene therapy.

Suitable cells, particularly for therapeutic approaches, or for modifying a viral genome, include eukaryotic (e.g., animal) and prokaryotic cells and/or cell lines. Non-limiting examples of such cells or cell lines generated from such cells include COS, CHO (e.g., CHO-S, CHO-K1, CHO-DG44, CHO-DUXB11, CHO-DUKX, CHOK1SV), VERO, MDCK, WI38, V79, B14AF28-G3, BHK, HaK, NS0, SP2/0-Ag14, HeLa, HEK293 (e.g., HEK293-F, HEK293-H, HEK293-T), and perC6 cells as well as insect cells such as *Spodopterafugiperda* (Sf), or fungal cells such as *Saccharomyces, Pichia* and *Schizosaccharomyces.* In certain embodiments, the cell line is a CHO, MDCK or HEK293 cell line. Suitable cells also include stem cells such as, by way of example, non-human embryonic stem cells, induced pluripotent stem cells, hematopoietic stem cells, neuronal stem cells and mesenchymal stem cells.

In an aspect, there is disclosed a method of treating a subject in need thereof, comprising inducing gene editing by transforming/transfecting the subject with the components of the artificial molecular complex as herein discussed or any of the vectors herein discussed and administering an inducer energy source to the subject. The disclosure comprehends uses of such a polynucleotide or vector in the manufacture of a medicament, e.g., such a medicament for treating a subject or for such a method of treating a subject. The disclosure comprehends the polynucleotide as herein discussed or any of the vectors herein discussed for use in a method of treating a subject in need thereof comprising inducing gene editing, wherein the method further comprises administering an inducer energy source to the subject. In an aspect, in the method, a repair template is also provided, for example delivered by a vector comprising said repair template.

In one embodiment of the present disclosure, minimal non-primate lentiviral vectors based on the equine infectious anemia virus (EIAV) are also contemplated, especially for gene therapy using the artificial molecular complexes of the present disclosure (see, e.g., Balagaan, J Gene Med 2006; 8: 275-285). In another embodiment, RetinoStat®, an equine infectious anemia virus-based lentiviral gene therapy vector that expresses angiostatic proteins endostatin and angiostatin that is delivered via a subretinal injection for the treatment of the web form of agerelated macular degeneration is also contemplated (see, e.g., Binley et al., HUMAN GENE THERAPY 23:980-991 (September 2012)) and this vector may be modified for the SSN-RTDD-RT system of the present disclosure. Presently, lentiviral vectors have been disclosed as in the treatment for Parkinson's Disease, see, e.g., U.S. Patent Application No. 2012/0295960 A1 and U.S. Patent No. 7,303,910 B2. Lentiviral vectors have also been disclosed for the treatment of ocular diseases, see e.g., U.S. Patent Application Nos. 2006/0281180, 2009/0007284, 2011/0117189, 2009/0017543, 2007/0054961, and 2010/0317109. Lentiviral vectors have also been disclosed for delivery to the brain, see, e.g., U.S. Patent Application Nos. 2011/0293571, 2011/0293571, 2004/0013648, 2007/0025970, 2009/0111106 and U.S. Patent No. 7,259,015.

In another embodiment of the present disclosure, the artificial molecular complex or components thereof may be administered in liposomes, such as a stable nucleic-acid-lipid particle (SNALP) (see, e.g., Morrissey et al., Nature Biotechnology, Vol. 23, No. 8, August 2005). Daily intravenous injections of about 1, 3 or 5 mg/kg/day of a specific CRISPR Cas targeted in a SNALP are contemplated. The daily treatment may be over about three days and then weekly for about five weeks. In another embodiment, a specific encapsulated SNALP can be administered by intravenous injection to at doses of about 1 or 2.5 mg/kg are also contemplated (see, e.g., Zimmermann et al., Nature Letters, Vol. 441, 4 May 2006). The SNALP formulation may contain the lipids 3-N-[(wmethoxypoly(ethylene glycol) 2000) carbamoyl]-1,2-dimyristyloxy-propylamine (PEG-C-DMA), 1,2-dilinoleyloxy-N,N-dimethyl-3-aminopropane (DLinDMA), 1,2-distearoyl-sn-glycero-3-phosphocholine (DSPC) and cholesterol, in a 2:40:10:48 molar per cent ratio (see, e.g., Zimmermann et al., Nature Letters, Vol. 441, 2006). In another embodiment, stable nucleic-acid-lipid particles (SNALPs) have proven to be effective delivery molecules to highly vascularized HepG2-derived liver tumors but not in poorly vascularized HCT-116 derived liver tumors (see, e.g., Li, Gene Therapy (2012) 19, 775-780). The SNALP liposomes may be prepared by formulating D-Lin-DMA and PEG-C-DMA with distearoylphosphatidylcholine (DSPC), Cholesterol and siRNA using a 25:1 lipid/siRNA ratio and a 48/40/10/2 molar ratio of Cholesterol/D-Lin-DMA/DSPC/PEG-C-DMA. The resulted SNALP liposomes are about 80-100 nm in size.

In yet another embodiment of the present disclosure, a SNALP may comprise synthetic cholesterol (Sigma-Aldrich, St Louis, MO, USA), dipalmitoylphosphatidylcholine (Avanti Polar Lipids, Alabaster, AL, USA), 3-N-[(w-methoxy polyethylene glycol)2000)carbamoyl]-1,2-dimyrestyloxypropylamine, and cationic 1,2-dilinoleyloxy-3-N,Ndimethylaminopropane (see, e.g., Geisbert et al., Lancet 2010; 375: 1896-905). A dosage of about 2 mg/kg total SSN/RTDD/RT per dose administered as, for example, a bolus intravenous infusion may be contemplated.

Similarly, the artificial molecular complexes according to the present disclosure can represent a useful tool for modification of genetic material in livestock or other animal cells. For example, the correction of genetic diseases or editing for favorable characteristics such as meat, milk, e.g., milk with a reduced lactose content, or egg production in livestock or poultry.

In one embodiment there is thus disclosed a method for generating a population of immune cells of an animal comprising introducing a construct according to the present disclosure into at least one immune cell of interest, *in vivo* or *ex vivo,* to treat a disease, such as an autoimmune disease, e.g., Type I diabetes or rheumatoid arthritis, or a proliferative disease, such as a cancer, for example glioma, melanoma, neuroblastoma, colon, lungs, breast and prostate cancer, multi-drug resistant cancers as well as cancers involved with mutated p53 gene.

The preferred tissues of most plant species forming targets for genome editing are immature embryos, embryogenic callus, meristems of intact plants, pollen, pollen tube or egg cells, suspension cells, or other cell types with regenerative potential. For some plants the preferred tissues can be protoplasts or leaves. Any cell that can be treated and then regenerated into a whole plant can be considered a preferred tissue or cell. The protocols for tissue preparation, regeneration, and DNA delivery are different depending on species, tissue type, delivery method and other factors. A common delivery method is particle bombardment of cells with DNA- or protein-coated gold or tungsten particles. Other delivery methods are polyethylene glycol (PEG)-mediated transformation, electroporation, viral infection, direct injection into cells, and *Agrobacterium-med iated* transformation. In some plants delivery can be made into fertilized egg cells by slicing through the style shortly after fertilization and applying a liquid with the editing reagents into the cut pollen tube. For animal cells, such as mammalian cells, electroporation, i.e., a transfection technology based on the momentary creation of small pores in cell membranes by applying an electrical pulse, might represent a suitable approach for introducing the at least one molecular complex according to the present disclosure. Several cell-type specific protocols for direct transfection success with a multitude of different cell types, including mammalian primary cells, stem cells and hard to transfect cell lines, are available to the skilled person, which are suitable as delivery tools for the at least one molecular complex according to the present disclosure. It is important to note that the combination of two or more methods or agents suitable for delivery may provide superior results depending on the cell type the genome of which has to be edited and is thus included within the scope of the present invention.

In one embodiment, supercharged proteins can be used to deliver the artificial molecular complex, or components thereof, according to the present disclosure. Supercharged proteins are a class of engineered or naturally occurring proteins with unusually high positive or negative net theoretical charge and may be employed in delivery of artificial molecular complex(es) or component(s) thereof or nucleic acid molecule(s) coding therefor. Both supernegatively and superpositively charged proteins exhibit a remarkable ability to withstand thermally or chemically induced aggregation. Superpositively charged proteins are also able to penetrate mammalian cells. Associating cargo with these proteins, such as plasmid DNA, RNA, or other proteins, can enable the functional delivery of these macromolecules into mammalian cells both in vitro and in vivo. David Liu's lab reported the creation and characterization of supercharged proteins in 2007 (Lawrence et al., 2007, Journal of the American Chemical Society 129, 10110-10112).

The nonviral delivery of RNA and plasmid DNA is of particular interest for transferring the artificial molecular complex into mammalian cells are valuable both for research and therapeutic applications (Akinc et al., 2010, Nat. Biotech. 26, 561-569). Purified +36 GFP protein (or other superpositively charged protein, e.g., +48 GFP) is mixed with RNAs in the appropriate serum-free media and allowed to complex prior addition to cells. Inclusion of serum at this stage inhibits formation of the supercharged protein-RNA complexes and reduces the effectiveness of the treatment. The following protocol has been found to be effective for a variety of cell lines (McNaughton et al., 2009, Proc. Natl. Acad. Sci. USA 106, 6111-6116) (However, preexperiments varying the dose of protein and RNA should be performed to optimize the procedure for specificcell lines): (1) One day before treatment, plate $1 \times 10^5$ cells (e.g. HEK293, number depending on the cell type) per well in a 48-well plate. (2) On the day of treatment, dilute purified +36 GFP protein in serum-free media to a final concentration 200 nM. Add RNA to a final concentration of 50 nM. Vortex to mix and incubate at room temperature for 10 min. (3) During incubation, aspirate media from cells and wash once with PBS. (4) Following incubation of +36 GFP and RNA, add the protein-RNA complexes to cells. (5) Incubate cells with complexes at 37°C for 4h. (6) Following incubation, aspirate the media and wash three times with 20 U/mL heparin PBS. Incubate cells with serum-containing media for a further 48h or longer depending upon the assay for activity. (7) Analyze cells by immunoblot, qPCR, phenotypic assay, or other appropriate method.

Another preferred delivery method for the artificial molecular complex is to assemble in vitro the RTDD-RT hybrid nucleic acid and then load this hybrid into an in vitro produced and optionally purified SSN polypeptide before applying it to the target cells of interest. However, other useful delivery methods could be delivery of the SSN polypeptide and optionally an interaction domain, for example a monomeric streptavidin, a scFv with a given specificity, or a DNA binding domain, or an additional nuclease domain as mRNA or as a genetic DNA construct, optionally comprising further regulatory elements, into the at least one target cell for transcription and/or expression in vivo, together with application of the hybrid nucleic acid simultaneously, before or especially after the SSN polypeptide delivery. In the case of non-covalent association of the RTDD with the RT component, these molecules can also be delivered separately; in case the RTDD is a gRNA, the gRNA can be delivered as RNA or as a DNA expression cassette that can be transcribed in vivo. In cases where the at least one SSN polypeptide or the at least one gRNA is delivered as an expression cassette, it may be preferable to express them from an RNA or DNA viral replicon or viral vector, particularly, when the target cell is a plant cell.

In a preferred embodiment the at least one artificial molecular complex is associated ex vivo, the different components of the complex, i.e., the at least SSN optionally comprising at least one interaction domain, the at least one RTDD and the at least one RT repair template nucleic acid are synthesized, either chemically, or recombinantly, ex vivo/in vitro and the different components are then purified, preferably before assembly. An additional purification step can be performed after assembly of the at least one artificial molecular complex according to the present disclosure. Methods for purifying nucleic acids, including DNA and RNA, or polypeptides, or ribonucleo- and ribonucleoprotein-complexes are readily available to the skilled person. The provision of a highly pure and stochiometric molecular complex, which can optionally be analyzed in vitro, allows the provision of a precise genome editing tool with high efficiency.

For embodiments of the present disclosure relying on non-nucleic acid or non-amino acid based molecules as RTDDs or interaction domains, for example biotin (vitamin H) or a derivative thereof, fluorescein, or digoxigenin or any other cognate binding partner for a SSN-RTDD, or RTDD-interaction domain interaction, or SSN-interaction domain interaction, it is possible that the RT is synthesized ex vivo and the RT is then chemically linked to the respective molecule.

In a further embodiment according to the various aspects according to the present invention, a conventional repair template nucleic acid sequence, either in the form of a plasmid or in the form of a nucleic acid oligonucleotide can be used in addition to the RTDD/RT to further increase the efficiency of the targeted genome editing event. Usually, the decisive factor whether a plasmid or another double-stranded DNA repair template is applied or whether a single-stranded oligonucleotide is used as repair template depends on the size of the intended modification to be introduced. The skilled person can easily define a further conventional repair template which can be used in addition to the hybrid nucleic acid construct according to the present disclosure. Those conventional repair templates can be introduced into at least one target cell of interest by a delivery vector, for example a geminiviral vector, in case the target cell is a plant cell, or by direct transfection or introduction as also detailed herein for the introduction of the RTDD/RT sequence according to the present disclosure.

In one aspect, there are kits disclosed containing any one or more of the elements disclosed herein. In some embodiments of the present disclosure, the kit comprises a vector system as taught herein and instructions for using the kit. Elements may be provided individually or in combinations, and may be provided in any suitable container, such as a vial, a bottle, or a tube. The kits may include gRNA and an unbound protector strand to stabilize the gRNA. The kits may include the gRNA as RTDD directly interacting with a RT of interest and optionally with a further protector strand bound to at least partially to the guide sequence. Thus the kits may include the gRNA in the form of a partially double-stranded nucleotide sequence. In some embodiments, the kit includes instructions in one or more languages, for example in more than one language. The instructions may be specific to the applications and methods described herein.

In a further aspect according to the present disclosure there is thus disclosed a kit comprising the at least one component or all components of the at least one artificial molecular complex of the present disclosure, wherein the at least one molecular complex can be provided as preassembled complex, or wherein the at least one molecular complex can be provided in the form of its separate constituents, comprising at least one SSN polypeptide, or an expressible sequence encoding the same, at least one RTDD sequence and at least one repair template nucleic acid sequence. The separate provision of the different constituents of the molecular complex, for instance in the form of a dried or lyophilized powder for nucleic acid sequences, guarantees a higher stability of the nucleic acid sequences particularly of the RTDD/RT construct, particularly if RNA sequences being much less stable than polypeptides are provided within the kit. At least one SSN protein and optionally at least one interaction domain interacting therewith or connected thereto can be delivered within a suitable storage buffer, e.g., comprising 300 mM NaCl, 10 mM Tris-HCl, 0.1 mM EDTA, 1 mM OTT, 50% Glycerol, pH 7.4 at 25°C for a Cas9 polypeptide. The kit can further comprise a suitable reaction buffer including suitable ions, e.g., $Mg2+$ for a Cas9 enzyme, required for the activity of a respective CRISPR polypeptide.

In some embodiments of the present disclosure, a kit comprises one or more reagents for use in a process utilizing one or more of the elements described herein. Reagents may be provided in any suitable container. For example, a kit may provide one or more reaction or storage buffers. Reagents may be provided in a form that is usable in a particular assay, or in a form that requires addition of one or more other components before use (e.g., in concentrate or lyophilized form). A buffer can be any buffer, including but not limited to a sodium carbonate buffer, a sodium bicarbonate buffer, a borate buffer, a Tris buffer, a MOPS buffer, a HEPES buffer, and combinations thereof. In some embodiments, the buffer is alkaline. In some embodiments, the buffer has a pH from about 7 to about 10. In some embodiments, the kit comprises one or more oligonucleotides corresponding to a guide sequence for insertion into a vector so as to operably link the guide sequence and a regulatory element. In some embodiments, the kit comprises a homologous recombination template polynucleotide. In some embodiments, the kit comprises one or more of the vectors and/or one or more of the polynucleotides described herein. The kit may advantageously allow to provide all elements of the systems of the disclosure.

Alternatively, the kit can comprise the SSN components as lyophilized mRNA or as lyophilized protein, respectively. In a further embodiment according to this aspect, the kit can comprise a further component providing a suitable delivery vehicle or delivery system in addition to a component comprising the SSN component(s) as molecular complex. In a further embodiment according to this aspect, at least one SSN polypeptide and at least one RTDD/RT sequence are presented as at least two components, the more than one SSN and/or RTDD/RT being mutually compatible. The at least one SSN polypeptide can be presented as vector to be transformed or transfected into a cell of interest, whereas the at least one RTDD/RT sequence can be presented as separate component. A kit according to the present disclosure can thus be suitable for the simultaneous or subsequent use of the different components in case more than one component is present. Optionally, a kit according to this aspect can comprise instructions for use, particularly instructions for use specific for a target cell to be edited. In a further embodiment according to this aspect of the present disclosure, the kit is specifically developed to provide a trait development kit for a specific plant of interest including specific tools to achieve the desired trait modification. According to this embodiment, the kit comprises a specific repair template, which is configured to transfer the trait of interest or to treat a disease of interest, or to modify a DNA target sequence of interest into a DNA target locus of interest in a cell, such as a mammalian cell or a plant cell. In addition, the kit comprises a suitable SSN enzyme, or two SSN nickases, associated as complex with at least one RTDD, wherein the RTDD comprises at least one first sequence portion directly interacting with the at least one SSN a second sequence portion configured to directly interact with at least one repair template nucleic acid sequence (RT), and wherein the at least one RTDD is configured to be associated with or to be able to associate with a repair template carrying the specific trait of interest.

A kit according to one embodiment of the present disclosure is both plant cell as well as trait specific, and the use of said kit allows the rapid targeting and modification of a genomic DNA locus of interest to achieve trait development.

In one embodiment, the RTDD is a gRNA, wherein the gRNA components are already designed to interact with PAM motifs and a CRISPR enzyme of interest and the provided repair template presents the sequence to be inserted or modified in a convenient way.

In one aspect according to the present disclosure there is thus provided plant, plant cell, a plant material, or a derivative, or a progeny thereof comprising or edited by at least one artificial molecular complex according to the present disclosure (embodiments of the invention relating to a plant, plant cell, or a plant material comprising a complex of the present disclosure). In a further aspect according to the present disclosure there is provided a plant, plant cell, a plant material, or a derivative, or a progeny thereof that has been modified with at least one artificial molecular complex.

In yet a further aspect according to the present disclosure there is provided a method of modifying at least one DNA target sequence, comprising the following steps: (i) providing at least one prokaryotic, eukaryotic, or viral cell and/or genome (embodiments of the present invention being related to at least one plant cell and/or genome) comprising at least one genomic complementarity sequence and at least one DNA target sequence in a genomic region of interest; (ii) providing at least one artificial molecular complex as defined herein; (iii) contacting the at least one artificial molecular complex with the at least one DNA target sequence under suitable conditions to achieve (a) interaction of the at least one site-specific nuclease with the at least one DNA target sequence; and (b) complementary base pairing of the at least one repair template nucleic acid sequence with the at least one genomic complementarity sequence to achieve recognition of the at least one complementarity sequence and induction of at least one DNA break by the at least one site-specific nuclease, wherein the at least one repair template nucleic acid sequence directs homology directed repair at the site of the at least one DNA target sequence; and (iv) obtaining at least one prokaryotic, eukaryotic, or viral cell and/or genome comprising a modification in the at least one DNA target sequence; wherein, in embodiments of the present invention, the at least one repair template docking domain, or the nucleic acid sequence encoding the same, is selected from an Agrobacterium VirD2 protein..

Due to the fact that the artificial molecular complex can be used within any cell type of interest, it is possible to design a SSN/RTDD/RT pair for the modification of any genomic, including episomal or epigenetic region of an organism of interest, comprising prokaryotic, eukaryotic or viral DNA target sequences or epigenetic sequences of interest. For embodiments modifying the genome of a virus, it is suitable to transfer the viral genome, or the relevant part thereof, into a vector of interest and to propagate and modify the viral genome within a suitable host cell (e.g., a prokaryotic or a eukaryotic cell) carrying the vector comprising the viral genome, or the relevant part thereof.

A "prokaryotic" cell as used herein refers to a unicellular organism that lacks a membrane-bound nucleus (karyon), mitochondria, or any other membrane-bound organelle and comprises archaea and bacteria.

A viral genome can be derived from any virus comprising an RNA or DNA encoded genome.

In one embodiment of the present disclosure, the at least one repair template nucleic acid sequence and/or the at least one repair template docking domain of the artificial molecular complex is/are provided to the at least one prokaryotic or eukaryotic cell (with embodiments of the invention relating to at least one plant cell) independently of the at least one site-specific nuclease of the at least one molecular complex and the at least one artificial molecular complex is assembled, or partially assembled, within the at least one prokaryotic, eukaryotic, or viral cell and/or genome.

In one embodiment, the at least one RTDD/RT sequence of the artificial molecular complex is provided to the at least one prokaryotic or eukaryotic cell independently of the at least one SSN polypeptide of the at least one molecular complex and the at least one artificial molecular complex is assembled within, or partially assembled, the at least one prokaryotic or eukaryotic cell (with embodiments of the invention relating to at least one plant cell).

The at least one artificial molecular complex, as detailed above, can be provided as *in vitro* assembled complex which is then introduced into at least one target cell of interest. Alternatively, some or all of the at least one SSN polypeptide and/or the at least one RTDD sequence and/or the at least one repair template nucleic acid sequence can be inserted as genetic RNA or DNA construct and can be produced *in vivo* so that the final assembly of the at least one molecular complex takes place *in vivo.* In a preferred embodiment, the at least one molecular complex is associated *ex vivo* and the at least one molecular complex comprising at least one SSN polypeptide, at least one guide nucleic acid sequence and at least one repair template nucleic acid sequence is then simultaneously provided to the at least one cell by a suitable delivery vector allowing the functional introduction of the at least one molecular complex into the at least one target cell comprising at least one DNA target sequence of interest.

In another preferred embodiment, the at least one SSN and optionally at least one interaction domain are provided as fusion protein on a plasmid to be produced within a cell comprising a DNA target sequence to be modified. The further components of the artificial molecular complex can then be produced *ex vivo.* For example, an inducible vector system can be used to produce the at least one SSN and optionally at least one interaction domain. As soon as a sufficient expression level is achieved, the RTDD/RT complex can be introduced into a target cell and the artificial molecular complex according to the present disclosure can be assembled *in situ.*

In another embodiment, the complete at least one artificial molecular complex is an *ex vivo* assembled artificial molecular complex.

"Suitable conditions" or "suitable reaction conditions" as referred to herein in the context of the methods according to the present disclosure refer to conditions, which allow both, the growth and development of a cell or organism, including prokaryotic or eukaryotic cells, being transformed or manufactured and the conditions necessary for achieving either stable integration or transient introduction of a genetic construct of interest in the at least one cell or organism of interest. Conditions to promote prokaryotic or bacterial growth and/or transformation are known to the skilled person (see also: Green and Sambrook, Molecular Cloning, A Laboratory Manual, 2012, Cold Spring Harbor Laboratory Press). Conditions to promote animal cell growth and/or for introducing genetic material into animal, particularly mammalian cells, are available to the skilled person for a variety of different cell lines (see Green and Sambrook *supra*). Conditions to promote plant or plant cell growth and development, including *inter alia* temperature, light, water, oxygen, mineral nutrients and soil support, which can vary for different plant species and can be readily determined by the skilled person in knowledge of the disclosure provided herein. The further suitable conditions to achieve stable integration or transient introduction of at least one molecular complex of interest depend on the transformation method selected for introduction of at least one molecular complex of interest, the developmental stage of the plant material or plant cell to be transformed and at least one molecular complex of interest to be introduced. Said suitable conditions can be defined by the skilled person in light of the present disclosure defining the suitable conditions for the methods in combination with exemplary molecular complexes and suitable delivery vectors and delivery techniques as disclosed and claimed herein.

In one embodiment according to the above method of the present disclosure, the at least one eukaryotic cell is a plant cell, preferably a plant cell from a plant selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and *Allium tuberosum,* or any variety or subspecies belonging to one of the aforementioned plants.

Concerning plant cells as targets, for example, a variety of transformation and/or transfection methods is available to the skilled person in the field. For maize protoplasts, for example, a suitable method is disclosed in Sheen, J. 2002 ("A transient expression assay using maize mesophyll protoplasts"). For *Arabidopsis* protoplasts, a suitable protocol is available from: doi.org/10.1038/ngrot.2007.199 or can be retrieved from http://www.nature.com/nprot/journal/v2/n7/full/nprot.2007.199.html. For tobacco and other dicot protoplasts, a suitable protocol is available from www.plantphysiol.org/cgi/doi/10.1104/pp.112.205179. The skilled person having knowledge of the present disclosure and being aware of the cited protocols can thus define a suitable method for introducing a molecular complex according to the present disclosure into a plant protoplast derived from a monocot or a dicot plant.

Protoplasts are very useful for testing gene editing technologies and reagents, but for regeneration of gene edited plants they are not always the preferred cell type, as very few plant species regenerate efficiently from protoplast. In these cases the preferred tissues for most plant species are immature embryos, embryogenic callus, fertilized embryos, meristems of intact plants, pollen, pollen tube or egg cells, embryogenic suspension cells, or other cell types with regenerative potential. A common physical delivery method is particle bombardment of cells with DNA- or protein-coated gold or tungsten particles, whereas a common biologically assisted method uses *Agrobacterium* or a (modified) viral vector as disclosed herein.

"Meristematic cell(s)" as referred to according to the present disclosure belong to a tissue type within a plant which is also referred to as meristem or cambium or formative tissue. Like stem cells in animal organisms, meristematic cells of plants representing undifferentiated cells have the intrinsic capability to develop and differentiate into specialized cell types, depending on genetic predisposition and further environmental and developmental factors. In plant organisms, meristems are not only present during the embryo development, but they can be found during the whole life cycle of a plant so that a targeted genetic modification of meristematic cells or tissues according to the present disclosure is not restricted to plant embryos or seedlings, but it can rather also be conducted in larger seedlings and more mature plants, for example when targeting meristems which build the basis for the reproductive plant organs, for example the tassel or ear in maize.

According to one embodiment according to the various aspects according to the present disclosure a meristematic cell can be a mature or immature plant cell of a plant embryo or seedling of a plant comprising at least one meristematic cell or meristematic tissue.

For certain genome editing approaches, a stable integration of the molecular complex encoding expression cassette(s) might be desirable, where a transgenic organism carrying a desired construct of interest, or a part thereof, can inherit a stably inserted construct to the progeny of a plant cell of interest initially transformed or transfected. Said stable integration can take place into any genomic region of an organism, such as a eukaryotic organism, including the nuclear genome as well as the extra nuclear genome, including the genome of plastids.

A transient introduction might be desirable, in case a certain effect is desired by the introduction of a molecular complex of interest, or a part thereof, but the construct *per se* should not be inherited to a progeny of the cell initially. Due to regulatory reasons, such an approach might be especially suitable for certain applications, particularly with plant cells, tissues, organs or material as structure comprising the DNA target sequence to be modified.

The term "targeted integration" or "functional integration" as used herein refers to the integration of a genetic construct of interest into at least one cell, which allows the transcription and/or translation and/or the catalytic activity and/or binding activity, including the binding of a nucleic acid molecule to another nucleic acid molecule, including DNA or RNA, or the binding of a protein to a target structure within the at least one cell. Where pertinent, the functional integration takes place in a certain cellular compartment of the at least one cell, including the nucleus, the cytosol, the mitochondrium, the chloroplast, the vacuole, the membrane, the cell wall and the like. Consequently, the term "functional integration" - in contrast to the term "stable integration" detailed above - implies that the molecular complex of interest is introduced into the at least one cell by any means of transformation, transfection or transduction by biological means, including *Agrobacterium* transformation, or physical means, including particle bombardment, as well as the subsequent step, wherein the molecular complex exerts its effect within or onto the at least one cell in which it was introduced. Depending on the nature of the genetic construct to be introduced, said effect naturally can vary and including, alone or in combination, *inter alia,* the transcription of a DNA encoded by the genetic construct to a ribonucleic acid, the translation of an RNA to an amino acid sequence, the activity of an RNA molecule within a cell, comprising the activity of a guide RNA, or an miRNA or an siRNA for use in RNA interference, and/or a binding activity, including the binding of a nucleic acid molecule to another nucleic acid molecule, including DNA or RNA, or the binding of a protein to a target structure within the at least one cell, or including the integration of a sequence delivered via a vector or a genetic construct, either transiently or in a stable way. Said effect can also comprise the catalytic activity of an amino acid sequence representing an enzyme or a catalytically active portion thereof within the at least one cell and the like. Said effect achieved after functional integration of the molecular complex according to the present disclosure can depend on the presence of regulatory sequences or localization sequences which are comprised by the genetic construct of interest as it is known to the person skilled in the art.

As detailed above, the methods according to the present disclosure targeting pluripotent or multipotent cells provide the advantage that both the transformation and the further development of a transformed at least one cell, particularly a meristematic cell, can take place *in planta* obviating the need for cumbersome *in vitro* cultivation steps for the regeneration of a plant or plant material therefrom. In certain embodiments, it might, however, be suitable to explant or dissect a plant cell, tissue, organ or material for further cultivation, screening or testing depending on the specific needs. Several methods for the *in vitro* cultivation of a plant cell, tissue, organ or material are available to the skilled person.

A stable integration might thus be desirable, where a transgenic plant carrying a desired construct of interest, or a part thereof, is stably inserted and the inserted construct or part thereof is inherited to the progeny of a plant cell of interest initially transformed. Said stable integration can take place into any genomic region of the plant, including the nuclear genome as well as the extranuclear genome, including the genome of plastids of a plant cell. Furthermore, the artificial molecular complexes according to the present disclosure can be used to create an epigenetic modification. In another aspect, there is disclosed a method of functional evaluation and screening of genes. The artificial molecular complex of the present disclosure can thus be used to precisely deliver functional domains, to activate or repress genes or to alter epigenetic state by precisely altering the methylation site on a specific locus of interest. A method of the disclosure may be used to create a plant, an animal or cell that may be used to model and/or study genetic or epigenetic conditions of interest, such as a through a model of mutations of interest or a disease model. As used herein, "disease" refers to a disease, disorder, or indication in a subject. For example, a method of the disclosure may be used to create an animal or cell that comprises a modification in one or more nucleic acid sequences associated with a disease, or a plant, animal or cell in which the expression of one or more nucleic acid sequences associated with a disease are altered. Such a nucleic acid sequence may encode a disease associated protein sequence or may be a disease associated control sequence. Accordingly, it is understood that in the embodiments of the disclosure, a plant, subject, patient, organism or cell can be a non-human subject, patient, organism or cell. Thus, the disclosure provides a plant, animal or cell, produced by the present methods, or a progeny thereof. The progeny may be a clone of the produced plant or animal, or may result from sexual reproduction by crossing with other individuals of the same species to introgress further desirable traits into their offspring. The cell may be provided *in vivo* or ex *vivo* in the cases of multicellular organisms, particularly animals or plants. In the instance where the cell is in cultured, a cell line may be established if appropriate culturing conditions are met and if the cell is suitably adapted for this purpose (for instance a stem cell). Bacterial cell lines produced by the disclosure are also envisaged. Hence, cell lines are also envisaged.

A transient introduction might be desirable, in case a certain effect, e.g., a silencing effect, a targeted manipulation, comprising a knock-in or a knock-out, is desired by the introduction of a genetic construct of interest, or a part thereof, but the construct perse should not be inherited to a progeny of the cell initially transformed.

In yet another embodiment of the above aspect according to the present invention, the introduction of the at least one molecular complex of interest, or parts thereof including the gRNA and/or the RT, is conducted using a means selected from the group consisting of a device suitable for particle bombardment, including a gene gun, including a hand-held gene gun (e.g., Helios® Gene Gun System, BIO-RAD) or a stationary gene gun, transformation, including transformation using *Agrobacterium spp.* or using a viral vector, microinjection, electroporation, whisker technology, including silicon carbide whisker technology, and chemical, e.g., using calcium phosphate, dendrimers, liposomes or cationic polymers, and non-chemical, e.g., using electroporation, sonoporation, optical transfection using a laser, protoplast fusion, impalefection, hydrodynamic gene delivery of DNA by injecting a delivery construct into an organ, such as the liver, of an animal, such as a rodent animal, transfection, or a combination thereof.

In certain embodiments, the at least one eukaryotic cell is a meristematic plant cell, and the plant cell, after introduction of the artificial molecular complex according to the present disclosure is further cultivated under suitable conditions until the developmental stage of maturity of the inflorescence is achieved to obtain a plant or plant material comprising a modification of interest mediated by the at least one molecular complex according to the present disclosure. Several protocols are, for example, available to the skilled person for producing germinable and viable pollen from *in vitro* cultured maize tassels, for example in Pareddy DR et al. (1992) Maturation of maize pollen in vitro. Plant Cell Rep 11 (10):535-539. doi:10.1007/BF00236273, Stapleton AE et al. (1992) Immature maize spikelets develop and produce pollen in culture. Plant Cell Rep 11 (5-6):248-252 or Pareddy DR et al. (1989) Production of normal, germinable and viable pollen from in vitro-cultured maize tassels. Theor Appl Genet 77 (4):521-526. Those protocols are *inter alia* based on excision of the tassel, surface sterilization and culture in a media with kinetin to promote tassel growth and maturation. After the spikelets are formed, a continuous harvest of anthers can be performed. After extrusion, anthers will be desiccated until the pollen comes out. Alternatively, anthers can be dissected and the pollen is shed in liquid medium that is subsequently used to pollinate ears.

"Maturity of the inflorescence" as used herein refers to the state, when the immature inflorescence of a plant comprising at least one meristematic cell has reached a developmental stage, when a mature inflorescence, i.e., a staminate inflorescence (male) or a pistillate inflorescence (female), is achieved and thus a gamete of the pollen (male) or of the ovule (female) or both is present. Said stage of the reproductive phase of a plant is especially important, as obtained plant material can directly be used for pollination of a further plant or for fertilization with the pollen of another plant.

In a further embodiment according to the above method of the present disclosure, the modification of the at least one DNA target sequence is a genome editing approach selected from the group consisting of yield improvement, tolerance to abiotic stress, including drought stress, osmotic stress, heat stress, cold stress, oxidative stress, heavy metal stress, salt stress or waterlogging, tolerance to biotic stress including tolerance to insects, tolerance to bacteria, tolerance to viruses, tolerance to fungi or tolerance to nematodes, resistance to herbicides, including glyphosate, glufosinate, acetolactate synthase (ALS) inhibitors, and Dicamba, lodging resistance, flowering time, shattering resistance, seed color, endosperm composition, nutritional content, phenotypic marker modification, or metabolic engineering, including genome editing to allow a molecular pharming approach in at least one plant cell. Phenotypic markers can be preferred targets for co-editing approaches, for example to monitor the editing efficiency.

In another embodiment of the present disclosure, the trait development is effected for a prokaryotic cell or a viral genome, for example to provide a prokaryotic cell with a suitably modified metabolic pathway to produce a product of interest, or to provide an attenuated viral genome.

In another embodiment according to the above method of the present disclosure, the modification of the at least one DNA target sequence is a genome editing approach for ex *vivo* modifying an immune cell in at least one eukaryotic cell, or a mammalian cell, or a mammalian leukocyte, for obtaining a modified cell suitable for treating a viral disease or for immunotherapy, especially cancer immunotherapy.

In one embodiment the above method according to the present disclosure is a method for modifying a eukaryotic cell, with embodiments of the invention relating to at least one plant cell, in a targeted way to provide a genetically modified, preferably non-transgenic plant, wherein the method may *inter alia* be a method for trait development. For example, a highly site-specific substitution of 1, 2, 3 or more nucleotides in the coding sequence of a plant gene can be introduced so as to produce substitutions of one or more amino acids that will confer tolerance to at least one herbicide such as glyphosate, glufosinate, Dicamba or an acetolactate synthase (ALS) inhibiting herbicide. Furthermore, in another embodiment, substitutions of one or more amino acids in the coding sequence of a nucleotide binding siteleucine-rich repeat (NBS-LRR) plant gene that will alter the pathogen recognition spectrum of the protein to optimize the plant's disease resistance. In yet a further embodiment, a small enhancer sequence or transcription factor binding site can be modified in an endogenous promoter of a plant gene or can be introduced into the promoter of a plant gene so as to alter the expression profile or strength of the plant gene regulated by the promoter. The expression profile can be altered through various modifications, introductions or deletions in other regions, such as intrans, 3' untranslated regions, *cis*- or *trans*-enhancer sequences. In yet a further embodiment, the genome of a plant cell, preferably a meristematic plant cell, can be modified in a way so that the plant resulting from the modified meristematic cell, can produce a chemical substance or compound of agronomic or pharmaceutical interest, for example insulin or insulin analoga, antibodies, a protein with an enzymatic function of interest, or any other pharmaceutically relevant compound suitable as medicament, as dietary supplement, or as health care product.

In a further aspect, the trait editing according to the methods of the present disclosure provides a method of trait editing to achieve treating of a disease and/or condition and/or preventing insect infection/infestation in a plant comprising modifying chromosomal or extrachromosomal genetic material of said plant by use of any of the foregoing methods. Non-limiting examples of the diseases and/or conditions treatable by the invented methods include Anthracnose Stalk Rot, Aspergillus Ear Rot, Common Corn Ear Rots, Corn Ear Rots, Common Rust of Corn, Diplodia Ear Rot, Diplodia Leaf Streak, Diplodia Stalk Rot, Downy Mildew, Eyespot, Fusarium Ear Rot, Fusarium Stalk Rot, Gibberella Ear Rot, Gibberella Stalk Rot, Goss's Wilt and Leaf Blight, Gray Leaf Spot, Head Smut, Northern Corn Leaf Blight, Physoderma Brown Spot, Pythium, Southern Leaf Blight, Southern Rust, and Stewart's Bacterial Wilt and Blight, and combinations thereof.

Non-limiting examples of the insects causing, directly or indirectly, diseases and/or conditions treatable by the invented methods include Armyworm, Asiatic Garden Beetle, Black Cutworm, Brown Marmorated Stink Bug, Brown Stink Bug, Common Stalk Borer, Corn Billbugs, Corn Earworm, Corn Leaf Aphid, Corn Rootworm, Corn Rootworm Silk Feeding, European Corn Borer, Fall Armyworm, Grape Colaspis, Hop Vine Borer, Japanese Beetle, Scouting for Fall Armyworm, Seedcorn Beetle, Seedcorn Maggot, Southern Corn Leaf Beetle, Southwestern Corn Borer, Spider Mite, Sugarcane Beetle, Western Bean Cutworm, White Grub, and Wireworms, and combinations thereof. The invented methods are also suitable for preventing infections and/or infestations of a plant by any such insect(s).

Non limiting examples of traits that can be introduced by this method are resistance or tolerance to insect pests, such as to rootworms, stem borers, cutworms, beetles, aphids, leafhoppers, weevils, mites and stinkbugs. These could be made by modification of plant genes, for example, to increase the inherent resistance of a plant to insect pests or to reduce its attractiveness to said pests. Other traits can be resistance or tolerance to nematodes, bacterial, fungal or viral pathogens or their vectors. Still other traits could be more efficient nutrient use, such as enhanced nitrogen use, improvements or introductions of efficiency in nitrogen fixation, enhanced photosynthetic efficiency, such as conversion of C3 plants to C4. Yet other traits could be enhanced tolerance to abiotic stressors such as temperature, water supply, salinity, pH, tolerance for extremes in sunlight exposure, nitrogen use efficiency, phosphorus use efficiency, water use efficiency and crop or biomass yield. Additional traits can be characteristics related to taste, appearance, nutrient or vitamin profiles of edible or feedable portions of the plant, or can be related to the storage longevity or quality of these portions. Finally, traits can be related to agronomic qualities such resistance to lodging, shattering, flowering time, ripening, emergence, harvesting, plant structure, vigor, size, yield, and other characteristics. To achieve the above trait modification the method according to the present disclosure comprises modifying chromosomal or extrachromosomal genetic material of a plant or plant cell by use of any of the foregoing methods.

In one embodiment according to the above method according to the present disclosure, the target cell is a prokaryotic cell and the modification comprises at least one modification of a genomic target region of interest of at least one prokaryotic cell, wherein the modification is suitable to modulate or increase resistance of the bacterium against biotic or abiotic stress, including resistance against antibiotics, or wherein the modification is suitable to improve phage resistance of the at least one prokaryotic cell. In another embodiment, the modification comprises inserting a gene of interest into a DNA target site of at least one prokaryotic cell of interest, e.g., to insert sequence encoding a fluorescent marker protein or another selectable marker into at least one DNA target site of interest. In another embodiment, the modification comprises knocking-out, i.e., deleting at least one DNA target site of interest in at least one prokaryotic cell. As prokaryotic cells will not further differentiate, but can directly inherit at least one introduce modification of interest to their progeny and as prokaryotic cells usually have a very short generation time in comparison to eukaryotic cells, a modification as introduced by at least one RTDD/RT in the form of at least one artificial molecular complex according to the present disclosure can be accomplished quickly and the resulting population of modified cells can be obtained and analyzed in a very short time period.

In certain embodiments of the present disclosure, the above method according to the present disclosure can further comprise the following step: (v) identifying and/or selecting at least one prokaryotic or eukaryotic cell (with embodiments of the invention relating to at least one plant cell and/or genome) comprising the modification in the at least one DNA target sequence, or identifying a modification to a viral genome as propagated in a prokaryotic or eukaryotic cell.

Methods for analyzing or identifying a modification according to the present disclosure as effected in the genome of at least one prokaryotic or eukaryotic cell or a viral genome are known to the person skilled in the art and comprise, but are not limited to polymerase chain reaction (PCR), including *inter alia* real time quantitative PCR, multiplex PCR, RT-PCR, nested PCR, analytical PCR and the like, microscopy, including bright and dark field microscopy, dispersion staining, phase contrast, fluorescence, confocal, differential interference contrast, deconvolution, electron microscopy, UV microscopy, IR microscopy, scanning probe microscopy, the analysis of the metabolite of a cell, the analysis of an altered resistance spectrum of a modified cell, RNA analysis, proteome analysis, functional assays for determining a functional integration, e.g., of a marker gene or a transgene of interest, or of a knock-out, Southern-Blot analysis, sequencing, including deep sequencing and combinations thereof. Cells comprising the desired modification can then be selected for further cultivation or any other downstream manufacturing step.

In a further aspect according to the present invention there is provided a method for manufacturing a plant or plant cell comprising the following steps: (i) performing a method of modifying at least one DNA target sequence in a eukaryotic cell as detailed above, wherein the at least one eukaryotic cell is a plant cell; (ii) obtaining at least one plant or a progeny thereof from the at least one plant cell from step (i); (iii) optionally: determining the modification in the at least one DNA target sequence in the at least one cell of the at least one plant or a progeny thereof.

Suitable plant cells, tissues, organs and materials for performing this aspect are detailed above. The term "manufacturing" according to the present disclosure is to be construed broadly and comprises any form of genetic manipulation performed on the genetic material of a plant or plant cell. The provision of the at least one artificial molecular complex comprising at least one RTDD/RT sequence comprising at least one repair template docking domain and at least one repair template nucleic acid and at least one SSN polypeptide, optionally comprising an interaction domain, can take place in a way to allow transient action or stable integration, or a combination thereof, of the different components as detailed above. Preferably, the at least one artificial molecular complex, or the different components thereof, are provided in a transient way so that no integration of any of those effector components as such, including a sequence encoding a guide nucleic acid RNA, a sequence encoding a repair template nucleic acid DNA, and a sequence encoding a CRISPR polypeptide, into the genome of target cell of interest takes place.

In one embodiment according to the above manufacturing method according to the present invention, the at least one plant or plant cell is selected from a monocotyledonous or a dicotyledonous plant, preferably, wherein the plant is selected from the group consisting of *Zea spp.,* including *Zea mays, Nicotiana benthamiana,* or *Beta spp,* including *Beta vulgaris,* or *Secale ssp.,* including *Secale cereal,* or *Triticum ssp.,* including *Triticum aestivum.*

As detailed throughout the present disclosure, the methods according to the present disclosure are suitable and can be adapted to target cells belonging to all kingdoms of life, as the gist of using a RTDD/RT construct which is associated in a functional way in combination with a suitable site-specific nuclease interacting with the RTDD is species and cell independent, provided there is a homologous recombination mechanism for DNA repair in the cell, yet dictated by the covalent or non-covalent interaction of the at least on gRNA and the at least one RT. What has to be determined individually for each target cell and each target are (i) the site-specific nuclease or catalytically active fragment thereof and whether the use of a interaction domain, e.g., as fusion protein, might be suitable; (ii) a suitable RTDD-SSN or RTDD-interaction domain pair allowing a direct interaction of said components by recognition of cognate binding partners; and (iii) a suitable RT and its connection with the RTDD, wherein the design of the RT is relevant to introduce a custom-made repair at a DNA target sequence of interest cleaved by the at least one SSN of the artificial molecular complex and optionally (for CRISPR nucleases) (iv) a gRNA and the CRISPR polypeptide, which have to be compatible as detailed above; (v) a matching of the gRNA of interest with a PAM site within the DNA target region of interest; and (vi) the DNA target sequence and the target modification to be introduced. For any sequenced genome publicly available, the design of suitable nucleic acid sequences can thus be made *in silico* based in the present disclosure.

In yet a further aspect according to the present disclosure there is provided the use of at least one RTDD/RT sequence according the present disclosure, or use of an artificial molecular complex according to the present disclosure for genome editing in a prokaryotic or a eukaryotic cell. In one embodiment of this aspect, the use is for a eukaryotic cell, such as a fungal, an animal or a plant cell or organism, or a viral organism as propagated in a prokaryotic or a eukaryotic cell. Embodiments of the invention relate to the use of an artificial molecular complex according to the present disclosure for genome engineering in a plant cell or organism.

According to the various aspects and embodiments according to the present disclosure, a eukaryotic cell or a method or use for modifying a eukaryotic cells, including stem cells, does explicitly not include any process of cloning human beings, a process for modifying the germ line genetic identity of human beings or the use of human embryos, or a method needing the destruction of human embryos to gain cells therefrom. Specifically human germ line cells or human embryos are thus specifically excluded as target cells or organisms to be modified with the artificial molecular complexes or by the methods according to the present disclosure. The present invention is further described with reference to the following non-limiting examples.

## Examples

The present invention is further illustrated by the following examples.

Example 1: Hybrid nucleic acid sequence as RTDD/RT pair suitable to be combined with a Cas or Cpf1 or Argonaute polypeptide

In one experiment, the tailed sgRNA or sgDNA are hybridized via both complementary base pairing and RNA-DNA or DNA-DNA ligation with a single-stranded repair template. For covalent association, synthesized DNA oligonucleotides are covalently ligated to the 3'-end of RNA/DNA oligonucleotides using the ssRNA ligase manufacturer's protocol. For non-covalent association, RNA/DNA and DNA oligonucleotides with partially complementary sequence are mixed and allowed to complex via Watson-Crick base pairing. Successful hybridization can be ascertained in gel shift assays. Treatment of aliquots of the hybrid nucleic acid with RNase and DNase enzymes prior to the gel shift assays indicates that some of the hybrid nucleic acid is composed of RNA and some of DNA for those experiments using sgRNAs. The nucleic acid hybrid is then complexed with recombinant Cas9 protein or another CRISPR- or Argonautederived nuclease. Successful complexing can be verified by treating with proteinase K, RNase, DNase and a mock treatment, and observing the relative gel shift patterns. Recombinant Cas polypeptides were produced and subsequently purified either through an external commercial entity or by in-house capability. Different architectures of hybrid nucleic acid sequences between a guide nucleic acid sequence as RTDD and a repair template (RT) nucleic acid sequence tested are shown in **Figures 1** and **2.**

Example 2: *In vitro* cutting of a DNA target by a complex of Cas9 protein with a hybrid RNA-DNA nucleic acid

In one experiment, the functionality of the Cas protein as a site-specific endonuclease was tested when used with the nucleic acid hybrid technology described. A linearized plasmid containing at least one target site for the sgRNA was be mixed with a Cas9-sgRNA-RT complex as described in the present disclosure. After incubation under conditions suitable for nuclease activity, including the right pH, temperature and cofactors and the like which are known to the skilled person for various CRISPR nucleases and variants thereof, the DNA target plasmid was run on an agarose gel and observed for band sizes indicating cutting a the expected target site. *In vitro* cleavage of the target DNA indicated that the RT associated with the sgRNA as "cargo" did not interfere with the normal function of the Cas9 complex as a site-specific endonuclease.

Example 3: *In vivo* editing by Cas9 protein complexed with a hybrid RNA-DNA nucleic acid

To demonstrate that a target gene can be edited *in vivo* by a delivered complex comprising Cas9 protein and a hybrid RNA-DNA nucleic acid, a non-functional tdTomato gene contained within a transformed plasmid was repaired by exchanging a single nucleotide to restore the fluorescent signal from the tdTomato gene. To determine the optimal use for editing by provision of a ssDNA repair template with complementarity to the target strand or non-target strand, complexes carrying repair templates of either strand were compared.

The hybrid nucleic acid RNA/DNA-Cas polypeptide complex obtained in Example 1 was used to repair an episomal plasmid target, encoding a tdTomato gene with a single point mutation from A to T that creates an early stop signal at codon position 51. This plasmid was introduced into a corn protoplast system together with the editing complex comprising Cas9 protein and a hybrid RNA-DNA nucleic acid through PEG- or electroporation-mediated delivery. A single-stranded repair template is then linked to the sgRNA through complementary base pairing. The repair template is complementary to the region 80 base pairs downstream and -40 base pairs upstream of the cut site. Successful editing then results in some cells displaying a tdTomato fluorescence phenotype due to repair of the tdTomato gene in at least one plasmid contained within them. The relative efficiency of editing with the different repair templates can thus easily be assessed by measuring the abundance of fluorescent cells resulting from each treatment.

Example 4: *In vivo* editing by Cas9 protein complexed with a RT attached to the RNA component by covalent attachment or associated by complementary base pairing

To demonstrate editing with hybrid nucleic acid molecules manufactured in various ways, the optimal conditions identified in Example 3 were used to assess repair of the same episomal plasmid target with hybrid nucleic acids covalent linkage or non-covalent base pairing of the repair template to the sgRNA.

In case a marker, particularly a fluorescent marker is used, successful editing will result in some cells displaying a fluorescence phenotype due to repair of the fluorescence encoding gene, such as a tdTomato gene, in at least one plasmid contained within them. The relative efficiency of editing with the different repair templates can then be assessed by measuring the abundance of fluorescent cells resulting from each treatment.

Example 5: *In vivo* editing by Cas9 protein complexed with a nucleic acid hybrid formed by linking the RT to the 5'- or 3'-end of the sgRNA

In one example, the method described in Example 3 can be used to identify a preference for the repair template hybridized or linked to the 5'- or 3'-end of the sgRNA. The preferable linkage covalency determined in Example 4 can be employed here. Based on results presented in Tsai et al. ("Dimeric CRISPR RNA-guided FokI nucleases for highly specific genome editing", Nature Biotechnology, 32, 569-576 (2014), doi:10.1038/nbt.2908) and further, Shechner et al. ("Multiplexable, locus-specific targeting of long RNAs with CRISPR-Display", Nature Methods, 12(7), 664-670 (2015), doi:10.1038/nmeth.3433), a 3' fusion is expected to be preferable.

Successful editing results in some cells displaying a fluorescence phenotype, such as a tdTomato phenotype, due to repair of the tdTomato gene in at least one plasmid contained within them. The relative efficiency of editing with the different repair templates can then be assessed by measuring the abundance of fluorescent cells resulting from each treatment.

Example 6: Determining the optimal linker length between sgRNA and repair template for *in vivo* editing by Cas9 protein complexed with a hybrid nucleic acid

In one example, an increasing linker length in 50 base pair increments up to a length of 500 base pairs between sgRNA and repair template was used to identify optimal conditions for homologous recombination to repair the target described in Example 3. Employing a set of linker lengths will help determine the necessary flexibility needed within the hybrid to overcome the protein target strand geometry. This is particularly necessary, when working with different CRISPR nucleases and thus specific gRNAs and individual repair templates (RTs) to coordinate the interplay of the molecular complex and to guarantee that the CRISPR complex also in the presence of the RT can exert its effect. The conditions of Example 3 were used together with the optimized parameters determined within the Examples 3 through 5. The linker was DNA with complementarity to sequence near the target gene.

Successful editing will result in some cells displaying a tdTomato fluorescence phenotype, in case a tdTomato marker is used, due to repair of the tdTomato gene in at least one plasmid contained within them. The relative efficiency of editing with the different linker lengths can then be assessed by measuring the abundance of fluorescent cells resulting from each treatment. Likewise, any other selectable marker of interest can be used including any fluorescent marker suitable for a cell type of interest, antibiotic markers, tag sequences, regulatory sequences and the like.

Example 7: Determining the optimal configuration of the repair template for *In vivo* editing by Cas9 protein complexed with a hybrid nucleic acid

To demonstrate editing with single- and double-stranded repair templates, the *in vivo* assay described in Example 3 was used for a relative comparison of the two configurations. Single-stranded repair templates are expected to be better based on the lower molecular weight and published higher rates of editing with short ssDNA oligos than with short dsDNA oligos. However, using a double-stranded repair template may be necessary in cases where large sequences need to be edited or inserted. The optimal conditions of Examples 4 and 6 can be used in this example.

A successful editing event results in some cells displaying a fluorescence phenotype, such as a tdTomato phenotype, due to repair of the tdTomato gene in at least one plasmid contained within them. The relative efficiency of editing with the different repair templates can then be assessed by measuring the abundance of fluorescent cells resulting from each treatment.

Example 8: *In vivo* editing of a chromosomal target by Cas9 protein complexed with a hybrid RNA-DNA nucleic acid

In one example, the method optimized by Examples 3 through 7 can be used to make edits to a chromosomal target gene. Here, a transgenic corn plant with a stable insertion of the early stop codon tdTomato cassette was used to demonstrate the utility of the invention for a chromosomal target. Successful editing resulted in some cells displaying a tdTomato fluorescence phenotype due to repair of the tdTomato gene integrated in the genomic DNA. The efficiency of editing was assessed by measuring the abundance of fluorescent cells resulting from each treatment.

Example 9: *In vivo* insertion of a gene cassette into a chromosomal target by Cas9 protein complexed with a hybrid RNA-DNA nucleic acid

To demonstrate the utility of the invention for insertion of a full length gene into a chromosomal target, a tdTomato fluorescent reporter gene and terminator were integrated into the hmg13 gene of corn, resulting in a tdTomato fluorescent signal due to expression driven by the endogenous promoter for hmg13. The results could demonstrate that long inserts can be made using the invented method and will help optimize the conditions for said insertion.

Successful editing results in some cells displaying a tdTomato fluorescence phenotype due to insertion of the *tdTomato* gene into the hmg13 target and subsequent tdTomato protein expression could be confirmed. The corresponding efficiency of editing for each cell type tested can then be assessed by measuring the abundance of fluorescent cells resulting from each treatment.

Example 10: Use of a cell penetrating peptide to deliver into plant cells the Cas9 protein complexed with a hybrid RNA-DNA nucleic acid

The optimal system identified in Examples 8 or 9 were used in this example to test the effectiveness of PEG based transformation versus transformation with a cell penetrating peptide (CPP). Previous publications and applications suggest that use of CPPs for delivery will enable introduction into cells with a cell wall the Cas9 protein complexed with a hybrid RNA-DNA nucleic acid. CPPs were thus used within a Cas fusion protein or linked to Cas though a disulfide bond formed between an N-terminal cysteine on the Cas protein and an N-terminal cysteine on the CPP. Free CPPs can also be used to aid the import of the Cas nucleic acid complex through transient binding on the nucleic acid strand. Initial CPPs can include the HIV TAT peptide (see e.g., SEQ ID NOs: 17 and 18), or a sequence derived therefrom and/or an $(Arg)_9$ sequence. The effectiveness can be tested using the optimized method of Examples 3-9 through successful tdTomato expression in a protoplast system.

Example 11: Further CRISPR nucleases

As detailed above, the hybrid nucleic acid sequences according to the present disclosure are suitable for a variety of CRISPR nucleases of different CRISPR systems. For any effector nuclease, e.g., Cas9 or Cpf1, the optimal conditions and lengths of the gRNA and the RT will have to be evaluated as detailed in Examples 1 to 10 above to achieve optimum results for a genome editing event of interest for each cell type of interest. Furthermore, first experiments with Cas9 nickases were conducted the same way as detailed above using more than one gRNA and either one or two individual RTs associated with at least one of the gRNAs. First results demonstrate that this seems to be a promising approach for precison genome editing in eukaryotic cells as well.

Example 12: Animal-cell constructs

The disclosed method can be used in eukaryotic cells provided they are capable of homologous recombination. In one first example, murine T-cells or T-cell precursors have been modified *in vitro* to modulate them to be suitable for cancer immunotherapy. It could be demonstrated that the hybrid nucleic acid constructs according to the present disclosure, when specifically optimized (codon optimization) and designed (PAMs, target sites) for an animal system can be used for high precision genome editing in a eukaryotic animal cell type of interest. The modification of an expressed gene regulating the proliferation or function of the T-cell using the method described in this disclosure can thus be used for therapy, particularly in a mammal, and more particularly to treat a disease or disorder in a subject by modification of a cell type of interest with the constructs according to the present disclosure.

Example 13: Transformation/transfection of exposed immature tassel tissue

As detailed above, a variety of physical/mechanical as well as biological means for transforming plant cells, tissues, organs or whole plants or parts thereof have been described for introducing genetic material into a plant or plant target structure. These methods are likewise suitable to introduce the at least one hybrid RNA/DNA nucleic acid sequence and/or at least one gRNA, and/or at least one repair template, and/or at least one CRISPR polypeptide according to the present disclosure. After having exposed and thus obtained a meristematic cell, for example a tassel tissue from a male maize plant, the following methods can be applied to transform this tissue:
Concerning biological means, plant tissues or cells thereof can be transformed with *Agrobacterium,* including *Agrobacterium tumefaciens* or *Agrobacterium rhizogenes* mediated transformation. This kind of transformation is well known to the person having skill in the art (see e.g., Jones, H.D. et al., "Review of methodologies and a protocol for the Agrobacterium-med iated transformation of wheat", plant methods, 2005; or Frame, B.R. et al., "Agrobacterium tumefaciens-med iated transformation of maize embryos using a standard binary vector system", Plant, 2002). To this end, an *Agrobacterium* culture comprising a construct of interest is, for example, cultivated over night at 28°C in fluid Luria Broth medium containing a suitable antibiotic, 10 mM MES and 200 mM ACE. The next day, the over night cultured is centrifuged at 4,400 rpm for 15 min and the supernatant is discarded. The pellet is then again centrifuged for 15 min at 4,400 rpm for 2 min and the remaining supernatant is discarded. The pellet is resuspended (5 ml $H_2O$, 10 mM MES, 10 mM $MgCl_2$ + $20\mu M$ ACE). The optical density at 600 nm is adjusted to 1.5. The possibly diluted suspension can then be further used.

Another possibility for transforming meristematic cells or tissues of a plant via biological means is the use of viral vectors. Viral vectors have the advantage that they can be introduced either as DNA or as RNA and to a plant target structure of interest. Furthermore, viral vectors or plant viruses have the capability of spreading into different cells and tissues.

For the purpose of the present invention, virus particles, *in vitro* transcripts of viruses or *Agrobacteria* carrying a virus encoding T-DNA can be introduced into a plant target structure of interest via filtration (vacuum and non-vacuum). Alternative experiments can be carried out using plant sap. To this end, either tobacco or spinach can be infected with the virus of interest to subsequently isolate said virus of interest from the plant sap for infecting another plant target structure, especially meristematic cells or tissues from different plants with the plant sap containing the virus.

Despite the biological means of transforming tassel structures of interest, further physical/mechanical means for transformation in addition to particle bombardment can be used.

One suitable method is microinjection. Microinjection can be used for any kind of meristematic structure tested, preferentially using a microscope with a micromanipulator. Due to the size of certain meristematic structure like tassel or ear meristems microinjection can be conducted under microscope control or, in case where the target structures are large enough, without microscope assistance. The injection can be conducted, using a variety of methods for a variety of different target molecules to be introduced into a plant target structure of interest including double-stranded plasma DNA, linear double-stranded DNA, RNA and proteins as well as virus particles in liquid solution. These different molecules can be applied with the help of a micro- or nano-needles which assist in injecting the target molecules into the meristematic cell or structure of interest. The target molecules are first coated onto the needle which is then inserted into the meristematic cell or structure of interest.

Another suitable means is particle bombardment, e.g., using a particle delivery system, this method being further disclosed above.

A further development of this technology is the use of a combination of silicon carbide (SiC) whiskers (e.g., Silar® Silicon Carbide Whisker) and microinjection. To this end, double-stranded (optionally plasmid) DNA, linear double-stranded DNA, RNA, protein, or a molecular ribonucleo-complex according to the present disclosure, or virus particles are precipitated onto the silicion carbide whisker to be injected via a microinjection needle into the meristematic structure or cell of interest. This technique has the advantage that it is not only possible to transfect a single cell, but there is the possibility to penetrate different cells in parallel due to the spread of the whiskers. Furthermore, the cells get less destructed, as the needle does not have to penetrate into the cell and the whiskers are quite small in size.

Example 14: Means for detecting a modification

Any transient or stable modification as introduced into at least one DNA target sequence according to the present disclosure can be detected using a fluorescence detection means, in case a fluorescent reporter is used. As tassel tissues like anthers and dry pollen have a strong autofluorescence, other means should be used for these cells and tissues. Detection can thus be accomplished and confirmed by further molecular methods, like PCR, including enrichment PCR, PCR-digest, a combination of enrichment PCR with PCR-digest, quantitative PCR, or sequencing, or RT-PCR, including deep or next generation sequencing or Southern or Northern blot analysis. Levels of protein can be analyzed by Western-Blotting and the like. In case, a phenotypically detectable trait was introduced into at least one cell of interest, it is also possible to perform an assay to detect whether said trait, for example, a resistance, a fluorescence, a morphological mutant phenotype, or any further trait, is present or absent in the at least one modified cell or a progeny or derivative thereof. The above detection methods are known to the skilled person.

As usual set up for analyzing a stable integration event in different target plants and cells thereof can be conducted as follows: First, DNA and/or RNA are extracted of different material, including, for example, tassel, anther or pollen tissue/cells transformed with different constructs encoding a fluorescent protein, e.g., a red fluorescent protein. In sum, samples can be analyzed via quantitative PCR (qPCR). From the above samples, several samples will show a clear, i.e., a very intense, (red) fluorescent signal, which is indicative of a positive event and which can then be selected. From those samples cDNA will be generated including controls without reverse transcriptase to exclude that the later results are not associated with undigested DNA. Out of the samples with positive DNA signal used for the transcription measurement, several samples could show a clear transcription and others a potential transcription (at the border of what could be clearly measured).

Example 15: Fusion protein of Cas9 and scFv

In one experiment, a fusion protein of Cas9 nuclease as SSN and a single-chain antibody against fluorescein as interaction domain can be expressed *in vitro* or *in vivo* and exposed to a FAM-labeled oligonucleotide to act as a repair template. The RT was synthesized and covalently linked to FAM as repair template docking domain. The editing efficiency was measured by a fluorescent signal indicating repair or sequence based measurements of repair frequency as detailed above. The SSN-interaction domain pair of a Cas9 and a scFv with a specific affinity for a selected ligand, e.g., FAM, can thus be produced and purified separately and can then be cross-linked or connected, or the SSN and the interaction domain (IA) can be produced as fusion molecule. Depending on the assay, the SSN-IA molecule can be transfected into a cell or added to an assay as protein, or the construct can be introduced into a target cell on a vector (inducible or active in a constitutive way) to be transcribed and translated *in vivo*. Furthermore, the sequence encoding the SSN-IA can be introduced into a target cell comprising a DNA target sequence of interest to be modified as RNA construct to be translated *in vivo*. Exemplary SSN-IA fusion molecules according to the present disclosure combining the functionality of a CRISPR-derived SSN with the extremely high binding affinity of a specialized protein to its cognate partner are shown with SEQ ID NO: 44 (Cas/mSA fusion construct) and SEQ ID NO: 45 (Cas/scFv(FAM) fusion construct).

**Figure 4 A** to **C** schematically illustrates a genome engineering approach using fusions of a SSN and a monomeric streptavidin or a scFv as IA. Notably, the use of monomeric streptavidin or scFvs or any other IA or RTDD is not restricted to the use of a CRISPR or Argonaute nuclease.

Example 16: Nucleic acid binding by a scFv-linked Cas9 fusion protein

To demonstrate the ability of the fusion protein of Example 15 to bind a single-stranded or double-stranded repair template, the binding assay described will be repeated with a fluorescein (FAM)-labeled oligonucleotide. FAM-labeled oligonucleotides can be obtained commercially. A successful interaction can be tested by co-migration of protein, DNA, and the fluorescent dye and the corresponding molecular weight increase. The functionality of the nuclease part of the fusion protein will be tested using an *in vitro* cleavage assay of a specific guide RNA and a linearized plasmid harboring the corresponding target. After incubation under conditions suitable for nuclease activity, including the right pH, temperature and cofactors and the like which are known to the skilled person for various CRISPR nucleases and variants thereof, the DNA target plasmid was run on an agarose gel and observed for band sizes indicating cutting at the expected target site. *In vitro* cleavage of the target DNA indicated that the RT associated with the nuclease did not interfere with the normal function of the Cas9 complex as a site-specific endonuclease.

Example 17: Fusion protein of Cas9 and mSA2

In one experiment, a fusion protein of Cas9 nuclease and a modified streptavidin tag (based on SEQ ID NO: 34) was expressed and exposed to a biotin-labeled oligonucleotide acting as repair template, the biotin acting as RTDD and the oligonucleotide representing a RT. The editing efficiency was measured by a fluorescent signal indicating repair or sequence based measurements of repair frequency.

Example 18: Nucleic acid binding by an mSA2-linked Cas9 fusion protein

To demonstrate the ability of the fusion protein of Example 17 to bind a single-stranded or double-stranded repair template, the binding assay described was repeated with a biotin-labeled oligonucleotide. Biotin-labeled oligonucleotides can be obtained commercially or generated using terminal deoxynucleotidyl transferase. A successful interaction can be tested by co-migration of protein and DNA and the corresponding molecular weight increase. The functionality of the nuclease part of the fusion protein will be tested using an *in vitro* cleavage assay of a specific guide RNA and a linearized plasmid harboring the corresponding target. After incubation under conditions suitable for nuclease activity, including the right pH, temperature and cofactors and the like which are known to the skilled person for various CRISPR nucleases and variants thereof, the DNA target plasmid was run on an agarose gel and observed for band sizes indicating cutting at the expected target site. *In vitro* cleavage of the target DNA indicated that the RT associated with the nuclease did not interfere with the normal function of the Cas9 complex as a site-specific endonuclease.

Example 19: *In vivo* editing of an episomal target by Cas9 fusion protein complexed with a FAM- or biotin-labeled repair template nucleic acid to restore gene functionality

To demonstrate that a target gene can be edited *in vivo* by a delivered complex comprising Cas9 protein and a FAM- or biotin-labeled nucleic acid, a nonfunctional tdTomato gene contained within a transformed plasmid was repaired by exchanging a single nucleotide to restore the fluorescent signal from the tdTomato gene. To determine the optimal use for editing by provision of a ssDNA repair template with complementarity to the target strand or non-target strand, complexes carrying repair templates of either strand are compared.

The nucleic acid complexed fusion protein of Example 16 or 18 respectively was used to repair an episomal plasmid target, encoding a tdTomato gene with a single point mutation from A to T that creates an early stop signal at codon position 51. This plasmid was introduced into a corn protoplast system together with the editing complex comprising Cas9-ScFV or Cas9-mSA2 fusion protein and a FAM or biotin-labeled nucleic acid through PEG-mediated delivery.

Successful editing then results in some cells displaying a tdTomato fluorescence phenotype due to repair of the tdTomato gene in at least one plasmid contained within them. The relative efficiency of editing with the different repair templates can thus easily be assessed by measuring the abundance of fluorescent cells resulting from each treatment.

Example 20: *In vivo* editing of a chromosomal target by Cas9 fusion protein complexed with a FAM- or biotin-labeled repair template nucleic acid to integrate DNA sequence in a specific locus

To demonstrate that a target gene can be edited *in vivo* by a delivered complex comprising Cas9 protein and a FAM- or biotin-labeled nucleic acid, a specific, known DNA sequence will be integrated at a specific site within the genomic DNA.

The fusion protein of Cas9 and a single-chain variable fragment with affinity to fluorescein (Example 16) or the fusion protein of Cas9 and the modified streptavidin (Example 18) was expressed and exposed to tagged repair template DNA and used to integrate a known DNA sequence in a genomic locus. Successful editing will be analyzed by fluorescent signal indication repair or molecular assays at the target site.

Example 21: Nucleic acid binding by a scFv-linked Argonaute fusion protein

To demonstrate the ability of binding a repair template nucleic acid to a non-CRISPR nuclease, a binding assay was performed showing the weight increase in a co-migration study using a FAM-labeled repair nucleic acid oligo and a fusion protein of an Argonaute nuclease (see SEQ ID NO: 46) and a single-chain variable fragment with affinity to FAM (see SEQ ID NOs: 43 and 45). Likewise, an Argonaute SSN could be linked to a monomeric streptavidin (see SEQ ID NOs: 42 and 44) as binding complex for a RT. The functionality of the nuclease part of the fusion protein was tested using an *in vitro* cleavage assay of a specific guide nucleic acid and a linearized plasmid harboring the corresponding target. After incubation under conditions suitable for nuclease activity, including the right pH, temperature and cofactors and the like which are known to the skilled person for various non-CRISPR nucleases and variants thereof, the DNA target plasmid was run on an agarose gel and observed for band sizes indicating cutting at the expected target site. *In vitro* cleavage of the target DNA indicated that the RT associated with the nuclease did not interfere with the normal function of the Argonaute complex as a site-specific endonuclease.

Example 22: *In vivo* editing of a chromosomal target by an Argonaute fusion protein complexed with a FAM -labeled repair template nucleic acid to integrate DNA sequence in a specific locus

To demonstrate that a target gene can be edited *in vivo* by a delivered complex comprising the non-CRISPR nuclease Argonaute protein and a FAM- or biotin-labeled nucleic acid, a specific, known DNA sequence will be integrated at a specific site within the genomic DNA.

The fusion protein of the Argonaute nuclease and a single-chain variable fragment with affinity to fluorescein (see Example 21) was expressed and exposed to tagged repair template DNA and used to integrate a known DNA sequence in a genomic locus. Successful editing will be analyzed by fluorescent signal indication repair or molecular assays at the target site.

Example 23: Fusion protein of CRISPR nuclease (like Cas9 or Cpf1) and an RTDD1

To demonstrate that the tethering strategy is working, a purified CRISPR nuclease like Cas9 or Cpf1 was fused with a RTDD1 , in this case it is tethered to a single chain variable fragment (SEQ ID NO: 54) and expressed in bacteria *E. coli.* It ran on a denaturing, continuous gradient (4-10%) SDS gel and shows the quantity and purity of the protein. The protein was stained in this gel. The right panel of Fig. 5 shows the tethering. This is a 4% non-denaturing acrylamide gel (Blue Native PAGE) and here the DNA is stained using GelRed. The FAM-labeled (RTDD2-) repair template was either incubated in the nuclease buffer without or with the nuclease-RTDD1 shown on the left panel of **Fig. 5.** If the protein was present, tethering occurred as seen by DNA being detected at a higher molecular weight level (arrow in **Figure 5).**

Example 24: Detection of HDR events

To demonstrate that next generation sequencing, more specifically amplicon deep sequencing, is able to detect the HDR event at the target site, the encoded nuclease (in this case it was a CRISPR nuclease) fused to streptavidin variant was transformed on a plasmid together with the repair template. The repair template had a 5' biotin tag and was delivered as single stranded oligonucleotide. Twenty-four hours after transformation, the protoplasts were collected and the DNA was extracted. The target site was amplified using a set of primers that were designed to not overlap with the homology arms of the repair template. Line 4 of **Figure 6** shows the correct HDR event. The event replaces the sequence AAGGTGCTCGGCCCCGAGCTC (SEQ ID NO: 52; encoding the amino acid sequence of KVLGPEL) with AAGTGGTCCAGCGCCGCGACCTAGCTC (SEQ ID NO: 53; encoding the amino acid sequence of KWSSAAT-L). SEQ ID NO: 51 is the full repair template demonstrating that the homology arms are not extending past the amplicon, meaning that PCR artifacts with remaining repair template are unlikely.

Example 25: Tethering of the repair template improves HDR efficiency

For this experiment, the components of Example 24 were transformed into corn leaf protoplasts. In the case of tethering, the nuclease (in this case it was a CRISPR nuclease) was fused to a native streptavidin sequence. In either case, the nuclease was delivered in form of a plasmid. The repair template DNA was delivered as oligonucleotide with a 5' biotin tag. Twenty-four hours after transformation, the protoplasts were collected and the DNA was extracted. The target site was amplified using a set of primers that were designed to not overlap with the homology arms of the repair template. Amplicon deep sequencing (see Example 24) and subsequent computational analysis allows for quantification of INDEL and HDR events at the target site. The HDR frequency was normalized to the INDEL frequency as a measure of double-strand break occurrence. The average HDR frequency increased from 0.92% ($\pm$0.06%) without tethering to 1.26% ($\pm$0.06%) when the repair template is tethered to the nuclease **(Figure 7).**

**Claims**

1. A method of modifying at least one DNA target sequence, comprising the following steps:

(i) providing at least one plant cell and/or genome comprising at least one genomic complementarity sequence and at least one DNA target sequence in a genomic region of interest;
(ii) providing at least one artificial molecular complex comprising

(A) at least one site-specific nuclease (SSN) or a catalytically active fragment thereof, or a nucleic acid sequence encoding the same, and directly interacting therewith
(B) at least one repair template docking domain (RTDD), or a nucleic acid sequence encoding the same, wherein the repair template docking domain is configured to directly interact covalently with at least one repair template nucleic acid sequence (RT);
(C) optionally comprising at least one interaction domain (IA), or a nucleic acid sequence encoding the same, wherein the at least one interaction domain is directly interacting with the at least one site-specific nuclease or the catalytically active fragment thereof, and wherein the at least one interaction domain is configured to provide at least one of the functionalities selected from the group consisting of

(I) interaction with the at least one repair template docking domain; and/or
(II) interaction with the at least one repair template nucleic acid sequence; and/or
(III) sequence-specific interaction with genomic DNA;

wherein the at least one repair template nucleic acid sequence comprises at least one portion being complementary to at least one genomic complementarity sequence, and wherein the at least one repair template nucleic acid sequence is configured to mediate repair of a DNA target sequence and wherein the repair template nucleic acid sequence comprises a single-stranded DNA nucleotide sequence; and
wherein the at least one repair template docking domain, or the nucleic acid sequence encoding the same, is selected from an Agrobacterium VirD2 protein;

(iii) contacting the at least one artificial molecular complex with the at least one DNA target sequence under suitable conditions to achieve

(a) interaction of the at least one site-specific nuclease with the at least one DNA target sequence; and
(b) complementary base pairing of the at least one repair template nucleic acid sequence with the at least one genomic complementarity sequence to achieve recognition of the at least one complementarity sequence and induction of at least one DNA break by the at least one site-specific nuclease, wherein the at least one repair template nucleic acid sequence directs homology directed repair at the site of the at least one DNA target sequence; and

(iv) obtaining at least one plant cell and/or genome comprising a modification in the at least one DNA target sequence.

2. The method according to claim 1, wherein the at least one repair template nucleic acid sequence and the at least one repair template docking domain of the artificial molecular complex are provided to the at least one plant cell independently of the at least one site-specific nuclease of the at least one artificial molecular complex and
the at least one artificial molecular complex is assembled, or partially assembled, within the at least one plant cell.

3. The method according to claim 1, wherein the at least one artificial molecular complex is an *ex vivo* assembled artificial molecular complex.

4. The method according to any one of claims 1 to 3, wherein the at least one plant cell is selected from a plant of the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and *Allium tuberosum,* or any variety or subspecies belonging to one of the aforementioned plants.

5. The method according to claim 1, wherein the modification of the at least one DNA target sequence causes a trait editing selected from the group consisting of yield improvement, tolerance to abiotic stress, including drought stress, osmotic stress, heat stress, cold stress, oxidative stress, heavy metal stress, salt stress or waterlogging, tolerance to biotic stress including tolerance to insects, tolerance to bacteria, tolerance to viruses, tolerance to fungi or tolerance to nematodes, resistance to herbicides, including glyphosate, glufosinate, ALS inhibitors, and Dicamba, lodging resistance, flowering time, shattering resistance, seed color, endosperm composition, nutritional content, or metabolic engineering.

6. The method according to claim 1, further comprising the following step:
(v) identifying and/or selecting at least one plant cell and/or genome comprising the modification in the at least one DNA target sequence.

7. The method of anyone of claims 1 to 6, wherein the site-specific nuclease, or the nucleic acid sequence encoding the same, is selected from at least one of a CRISPR nuclease, including Cas or Cpf1 nucleases, a TALEN, a ZFN, a meganuclease, a restriction endonuclease, including FokI or a variant thereof, or two site-specific nicking endonucleases, or a variant or a catalytically active fragment thereof.

8. The method of anyone of claims 1 to 7, wherein the at least one site-specific nuclease and/or the at least one interaction domain comprises at least one nuclear localization sequence, a plastid localization sequence, preferably a mitochondrion localization sequence or a chloroplast localization sequence.

9. The method of anyone of claims 1 to 8, wherein the at least one repair template nucleic acid sequence comprises at least one end portion, preferably the 3'-end, wherein this end portion does not interact with any other component of the artificial molecular complex and is thus configured to hybridize to at least one genomic complementarity sequence to mediate repair of the DNA target sequence, and/or wherein the at least one repair template nucleic acid sequence is provided as plasmid.

10. The method of anyone of the preceding claims, wherein the at least one site-specific nuclease, or the sequence encoding the same, and the at least one interaction domain, or the sequence encoding the same, and/or the at least one repair template docking domain, or the sequence encoding the same, are connected by at least one linker domain.

**11.** A method for manufacturing a plant or plant cell comprising the following steps:

(i) performing a method according to claim 1;
(ii) obtaining at least one plant from the at least one plant cell from step (i);
(iii) optionally: determining the modification in the at least one DNA target sequence in the at least one cell of the at least one plant.

**12.** A plant, plant cell, or a plant material comprising at least one artificial molecular complex as defined in claim 1.

**13.** Use of at least one artificial molecular complex as defined in claim 1 for genome engineering in a plant cell or organism for homology directed repair at the site of the at least one DNA target sequence in a genomic region of interest of at least one plant cell.

**Patentansprüche**

**1.** Verfahren zur Modifizierung mindestens einer DNA-Zielsequenz, umfassend die folgenden Schritte:

(i) Bereitstellen mindestens einer Pflanzenzelle und/oder eines Genoms, das mindestens eine genomische Komplementärsequenz und mindestens eine DNA-Zielsequenz in einer genomischen Region von Interesse aufweist;
(ii) Bereitstellen mindestens eines künstlichen molekularen Komplexes, umfassend

(A) mindestens eine ortsspezifische Nuklease (SSN) oder ein katalytisch aktives Fragment davon, oder eine Nukleinsäuresequenz, welche für das selbige kodiert, und welche direkt mit diesem interagiert
(B) mindestens eine Reparaturmatrizen-Andockdomäne (RTDD), oder eine Nukleinsäuresequenz, welche die selbige kodiert, wobei die Reparaturmatrizen-Andockdomäne derart konfiguriert ist, direkt mit mindestens einer Reparaturmatrizen-Nukleinsäuresequenz (RT) kovalent zu interagieren;
(C) gegebenenfalls mindestens eine Interaktionsdomäne (IA), oder eine Nukleinsäuresequenz, umfassend, die diese kodiert, wobei die mindestens eine Interaktionsdomäne direkt mit der mindestens einen ortsspezifische Nuklease oder dem katalytisch aktiven Fragment davon interagiert, und wobei die mindestens eine Interaktionsdomäne so konfiguriert ist, dass sie mindestens eine der Funktionalitäten bereitstellt, ausgewählt aus der Gruppe bestehend aus

(I) Interaktion mit der mindestens einen Reparaturmatrizen-Andockdomäne; und/oder
(II) Interaktion mit der mindestens einen Reparaturmatrizen-Nukleinsäuresequenz; und/oder
(III) sequenzspezifische Interaktion mit genomischer DNA;

wobei die mindestens eine Reparaturmatrizen-Nukleinsäuresequenz mindestens einen Abschnitt umfasst, der komplementär zu mindestens einer genomischen Komplementärsequenz ist, und wobei die mindestens eine Reparaturmatrizen-Nukleinsäuresequenz so konfiguriert ist, dass sie die Reparatur einer DNA-Zielsequenz vermittelt, und wobei die Reparaturmatrizen-Nukleinsäuresequenz eine einzelsträngige DNA-Nukleotidsequenz umfasst; und wobei die mindestens eine Reparaturmatrizen-Andockdomäne, oder die Nukleinsäuresequenz, die für dieselbe kodiert, aus einem Agrobakterium VirD2-Protein ausgewählt ist;

(iii) Inkontaktbringen des mindestens einen künstlichen molekularen Komplexes mit der mindestens einen DNA-Zielsequenz unter geeigneten Bedingungen, um Folgendes zu erzielen:

(a) Interaktion der mindestens einen ortsspezifischen Nuklease mit der mindestens einen DNA-Zielsequenz; und
(b) komplementäre Basenpaarung der mindestens einen Reparaturmatrizen-Nukleinsäuresequenz mit der mindestens einen genomischen Komplementärsequenz, um eine Erkennung der mindestens einen Komplementärsequenz und Erzeugung von mindestens einem DNA-Bruch durch die mindestens eine ortsspezifische Nuklease zu erreichen, wobei die mindestens eine Reparaturmatrizen-Nukleinsäuresequenz die Homologie-gerichtete Reparatur an der Stelle der mindestens einen DNA-Zielsequenz lenkt; und

(iv) Erhalten mindestens einer Pflanzenzelle und/oder eines Genoms, welche eine Modifikation in der mindestens einen DNA-Zielsequenz umfasst.

2. Verfahren nach Anspruch 1, wobei die mindestens eine Reparaturmatrizen-Nukleinsäuresequenz und die mindestens eine Reparaturmatrizen-Andockdomäne des künstlichen molekularen Komplexes der mindestens einen Pflanzenzelle unabhängig von der mindestens einen ortsspezifischen Nuklease des mindestens einen künstlichen molekularen Komplexes bereitgestellt werden, und wobei der mindestens eine molekulare Komplex innerhalb der mindestens einen Pflanzenzelle zusammengesetzt, oder teilweise zusammengesetzt, wird.

3. Verfahren nach Anspruch 1, wobei der mindestens eine künstliche molekulare Komplex ein ex vivo zusammengesetzter künstlicher molekularer Komplex ist.

4. Verfahren nach einem der Ansprüche 1 bis 3, wobei die mindestens eine Pflanzenzelle ausgewählt ist aus einer Pflanze aus der Gruppe bestehend aus *Hordeum vulgare, Hordeum bulbusom, Sorghum bicolor, Saccharum officinarium, Zea spp.,* einschließlich *Zea mays, Setaria italica, Oryza minuta, Oryza sativa, Oryza australiensis, Oryza alta, Triticum aestivum, Triticum durum, Secale cereale, Triticale, Malus domestica, Brachypodium distachyon, Hordeum marinum, Aegilops tauschii, Daucus glochidiatus,* Beta-Arten, einschließlich *Beta vulgaris, Daucus pusillus, Daucus muricatus, Daucus carota, Eucalyptus grandis, Nicotian a sylvestris,Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and *Allium tuberosum,* oder jede Sorte oder Unterart, die zu einer der vorgenannten Pflanzen gehört.

5. Verfahren nach Anspruch 1, wobei die Modifikation der mindestens einen DNA Zielsequenz eine Merkmalsbearbeitung bewirkt, ausgewählt aus der Gruppe bestehend aus Ertragsverbesserung, Toleranz gegenüber abiotischem Stress, einschließlich Trockenstress, osmotischem Stress, Hitzestress, Kältestress, oxidativem Stress, Schwermetallstress, Salzstress oder Staunässe, Toleranz gegenüber biotischem Stress, einschließlich Toleranz gegenüber Insekten, Toleranz gegenüber Bakterien, Toleranz gegenüber Viren, Toleranz gegenüber Pilzen oder Toleranz gegenüber Nematoden, Resistenz gegen Herbizide, einschließlich Glyphosat, Glufosinat, ALS-Inhibitoren und Dicamba, Lagerungsresistenz, Blütezeit, Bruchresistenz, Samenfarbe, Endosperm-Zusammensetzung, Nährstoffgehalt, oder Stoffwechseleingriffe.

6. Verfahren nach Anspruch 1, das außerdem den folgenden Schritt umfasst:
(v) Identifizieren und/oder Selektieren mindestens einer Pflanzenzelle und/oder eines Genoms, das die Modifikation in der mindestens einen DNA-Zielsequenz aufweist.

7. Verfahren nach einem der Ansprüche 1 bis 6, wobei die ortsspezifische Nuklease, oder die Nukleinsäuresequenz, die diese kodiert, ausgewählt ist aus mindestens einer CRISPR Nuklease, einschließlich Cas- oder Cpf1-Nukleasen, einer TALEN, einer ZFN, einer Meganuklease, einer Restriktionsendonuklease, einschließlich Fokl oder einer Variante davon, oder aus zwei ortsspezifischen Einzelstrangbruch-Endonukleasen, oder einer Variante, oder einem katalytisch aktiven Fragment davon.

8. Verfahren nach einem der Ansprüche 1 bis 7, wobei die mindestens eine ortsspezifische Nuklease und/oder die mindestens eine Interaktionsdomäne mindestens eine Kernlokalisierungssequenz, eine Plastiden-Lokalisierungssequenz, vorzugsweise eine Mitochondrien-Lokalisierungssequenz oder eine Chloroplasten-Lokalisierungssequenz umfasst.

9. Verfahren nach einem der Ansprüche 1 bis 8, wobei die mindestens eine Reparaturmatrizen-Nukleinsäuresequenz mindestens einen Endabschnitt, vorzugsweise das 3'-Ende, umfasst, wobei dieser Endabschnitt nicht mit irgendeiner anderen Komponente des künstlichen molekularen Komplexes interagiert und so konfiguriert ist, dass dieser mit mindestens einer genomischen Komplementärsequenz hybridisiert, um die Reparatur der DNA-Zielsequenz zu vermitteln, und/oder wobei die mindestens eine Reparaturmatrizen-Nukleinsäuresequenz als Plasmid bereitgestellt wird.

**10.** Verfahren nach einem der vorhergehenden Ansprüche, wobei die mindestens eine ortsspezifische Nuklease, oder die für selbige kodierende Sequenz, und die mindestens eine Interaktionsdomäne, oder die für selbige kodierende Sequenz, und/oder die mindestens eine Reparaturmatrizen-Andockdomäne, oder die Sequenz, die für selbige kodiert, durch mindestens eine Linker-Domäne verbunden sind.

**11.** Verfahren zur Herstellung einer Pflanze oder Pflanzenzelle, umfassend die folgenden Schritte:

(i) Durchführen eines Verfahrens nach Anspruch 1;
(ii) Gewinnen mindestens einer Pflanze aus der mindestens einen Pflanzenzelle aus Schritt (i);
(iii) gegebenenfalls: Bestimmen der Modifikation in der mindestens einen DNA-Zielsequenz in der mindestens einen Zelle der mindestens einen Pflanze.

**12.** Pflanze, Pflanzenzelle, oder ein Pflanzenmaterial, umfassend mindestens einen künstlichen molekularen Komplex, wie in Anspruch 1 definiert.

**13.** Verwendung mindestens eines künstlichen molekularen Komplexes, wie in Anspruch 1 definiert, zur Genom-Modifikation in einer Pflanzenzelle oder einem Organismus zur Homologie-gerichteten Reparatur an der Stelle der mindestens einen DNA-Zielsequenz in einer genomischen Region von Interesse in mindestens einer Pflanzenzelle.

**Revendications**

**1.** Procédé de modification d'au moins une séquence cible d'ADN, comprenant les étapes suivantes consistant à:

(i) fournir au moins une cellule végétale et/ou un génome comprenant au moins une séquence de complémentarité génomique et au moins une séquence cible d'ADN dans une région génomique d'intérêt;
(ii) fournir au moins un complexe moléculaire artificiel comprenant

(A) au moins une nucléase spécifique de site (SSN) ou un fragment catalytiquement actif de celle-ci, ou une séquence d'acide nucléique codant pour celui-ci, et interagissant directement avec celui-ci,
(B) au moins un domaine d'ancrage de matrice de réparation (RTDD), ou une séquence d'acide nucléique codant pour celui-ci, dans lequel le domaine d'ancrage de matrice de réparation est configuré pour interagir directement de manière covalente avec au moins une séquence d'acide nucléique de matrice de réparation (RT);
(C) comprenant le cas échéant au moins un domaine d'interaction (IA), ou une séquence d'acide nucléique codant pour celui-ci, dans lequel ledit au moins un domaine d'interaction interagit directement avec ladite au moins une nucléase spécifique de site ou le fragment catalytiquement actif de celle-ci, et dans lequel ledit au moins un domaine d'interaction est configuré pour fournir au moins l'une des fonctionnalités sélectionnées dans le groupe consistant en

(I) interaction avec ledit au moins un domaine d'ancrage de matrice de réparation; et/ou
(II) interaction avec ladite au moins une séquence d'acide nucléique de matrice de réparation; et/ou
(III) interaction spécifique à la séquence avec de l'ADN génomique;

dans lequel ladite au moins une séquence d'acide nucléique de matrice de réparation comprend au moins une portion qui est complémentaire d'au moins une séquence de complémentarité génomique, et dans lequel ladite au moins une séquence d'acide nucléique de matrice de réparation est configurée pour médier la réparation d'une séquence cible d'ADN et dans lequel la séquence d'acide nucléique de matrice de réparation comprend une séquence nucléotidique d'ADN simple brin; et dans lequel ledit au moins un domaine d'ancrage de matrice de réparation, ou la séquence d'acide nucléique codant pour celui-ci, est choisi parmi une protéine d'Agrobacterium VirD2;

(iii) mettre en contact ledit au moins un complexe moléculaire artificiel avec ladite au moins une séquence cible d'ADN dans des conditions appropriées pour obtenir

(a) interaction de ladite au moins une nucléase spécifique de site avec ladite au moins une séquence cible d'ADN; et
(b) appariement de base complémentaire de ladite au moins une séquence d'acide nucléique de matrice de réparation avec ladite au moins une séquence de complémentarité génomique pour obtenir une reconnaissance de ladite au moins une séquence de complémentarité et de l'induction d'au moins une rupture d'ADN par ladite au moins une nucléase spécifique de site, dans lequel ladite au moins une séquence d'acide nucléique de matrice de réparation dirige la réparation dirigée par homologie sur le site de ladite au moins une séquence cible d'ADN; et

(iv) obtenir au moins une cellule végétale et/ou un génome comprenant une modification dans ladite au moins une séquence cible d'ADN.

2. Procédé selon la revendication 1, dans lequel ladite au moins une séquence d'acide nucléique de matrice de réparation et ledit au moins un domaine d'ancrage de matrice de réparation du complexe moléculaire artificiel sont fournis à ladite au moins une cellule végétale indépendamment de ladite au moins une nucléase spécifique de site dudit au moins un complexe moléculaire artificiel, et dans lequel ledit au moins un complexe moléculaire artificiel est assemblé, ou partiellement assemblé, au sein de ladite au moins une cellule végétale.

3. Procédé selon la revendication 1, dans lequel ledit au moins un complexe moléculaire artificiel est un complexe moléculaire artificiel assemblé *ex vivo.*

4. Procédé selon l'une quelconque des revendications 1 à 3, dans lequel ladite au moins une cellule végétale est choisie parmi une plante du groupe constitué par *Hordeum vulgare, Hordeum bulbusom, Sorghum bicolor, Saccharum officinarium, Zea spp., y compris Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* y compris *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and *Allium tuberosum,* ou toute variété ou sous-espèce appartenant à l'une des plantes précitées.

5. Procédé selon la revendication 1, dans lequel la modification de ladite au moins une séquence cible d'ADN provoque une édition de trait sélectionné dans le groupe consistant en l'amélioration du rendement, la tolérance au stress abiotique, y compris le stress de la sécheresse, le stress osmotique, le stress thermique, le stress froid, le stress oxydatif, le stress des métaux lourds, le stress salin ou l'engorgement, la tolérance au stress biotique dont la tolérance aux insectes, la tolérance aux bactéries, la tolérance aux virus, la tolérance aux champignons ou la tolérance aux nématodes, la résistance aux herbicides, dont le glyphosate, le glufosinate, les inhibiteurs de l'ALS, et Dicamba, la résistance à la verse, la durée de floraison, la résistance à la rupture, la couleur des graines, la composition de l'endosperme, le contenu nutritionnel ou l'ingénierie métabolique.

6. Procédé selon la revendication 1, comprenant en outre l'étape suivante consistant à:
(v) identifier et/ou sélectionner au moins une cellule végétale et/ou un génome comprenant la modification dans ladite au moins une séquence cible d'ADN.

7. Procédé selon l'une quelconque des revendications 1 à 6, dans lequel la nucléase spécifique de site, ou la séquence d'acide nucléique codant pour celle-ci, est choisie parmi au moins l'une parmi une nucléase CRISPR, y compris les nucléases Cas ou Cpf1, une TALEN, une ZFN, une méganucléase, une endonucléase de restriction, y compris FokI ou un variant de ceci, ou deux endonucléases de coupure spécifiques de site, ou un variant ou un fragment catalytiquement actif de ceci.

8. Procédé selon l'une quelconque des revendications 1 à 7, dans lequel ladite au moins une nucléase spécifique de site et/ou ledit au moins un domaine d'interaction comprend au moins une séquence de localisation nucléaire, une séquence de localisation de plastide, de préférence une séquence de localisation de mitochondrie ou une séquence de localisation de chloroplastes.

**9.** Procédé selon l'une quelconque des revendications 1 à 8, dans lequel ladite au moins une séquence d'acide nucléique de matrice de réparation comprend au moins une portion terminale, de préférence l'extrémité 3', dans lequel cette portion terminale n'interagit avec aucun autre composant du complexe moléculaire artificiel et est ainsi configurée pour s'hybrider à au moins une séquence de complémentarité génomique pour médier la réparation de la séquence cible d'ADN, et/ou dans lequel ladite au moins une séquence d'acide nucléique de matrice de réparation est fournie en tant que plasmide.

**10.** Procédé selon l'une quelconque des revendications précédentes, dans lequel ladite au moins une nucléase spécifique de site, ou la séquence codant pour celle-ci, et ledit au moins un domaine d'interaction, ou la séquence codant pour celui-ci, et/ou ledit au moins un domaine d'ancrage de matrice de réparation, ou la séquence codant pour celui-ci, sont connectés par au moins un domaine de liaison.

**11.** Procédé de fabrication d'une plante ou cellule végétale, comprenant les étapes suivantes consistant à:

(i) mettre en oeuvre un procédé selon la revendication 1 ;
(ii) obtenir au moins une plante à partir de ladite au moins une cellule végétale de l'étape (i);
(iii) le cas échéant: déterminer la modification dans ladite au moins une séquence cible d'ADN dans ladite au moins une cellule de ladite au moins une plante.

**12.** Plante, cellule végétale ou matériel végétal comprenant au moins un complexe moléculaire artificiel tel que défini dans la revendication 1.

**13.** Utilisation d'au moins un complexe moléculaire artificiel tel que défini dans la revendication 1 pour l'ingénierie génomique dans une cellule végétale ou un organisme, pour la réparation dirigée par homologie sur le site de ladite au moins une séquence cible d'ADN dans une région génomique d'intérêt d'au moins une cellule végétale.

# Figure 1

Guide nucleic acid

IA

**A** 5'  3'

ssDNA (RT)

3' 5'

RTDD

Guide nucleic acid

(RTDD or IA)

ssDNA (RT)

**B** 5' 3'

Guide nucleic acid

IA dsDNA (RT)

**C** 5' 3'

3' 5'

RTDD

Guide nucleic acid

(RTDD or IA)

dsDNA (RT)

**D** 5' 3'

3' 5'

# Figure 2

**A** Guide nucleic acid (RTDD or IA) 5' ──── RT ──── 3'

**B** 5' ──── RT ──── Guide nucleic acid (RTDD or IA) 3'

**C** Guide nucleic acid (RTDD or IA) 5' ──── RT ──── 3'

# Figure 3

## Figure 4

A

Guide nucleic acid (RTDD)

SSN (e.g., NgAgo, Cas or Cpf1)

Genomic DNA

modified streptavidin or scFv (IA)

Labelled repair template (RTDD (label) and RT, respectively)

B

Guide nucleic acid (RTDD)

SSN (e.g., NgAgo, Cas or Cpf1)

modified streptavidin or scFv (IA)

Labelled repair template (RTDD (label) and RT, respectively)

Genomic DNA

C

Edited genomic DNA

# Figure 5

# Figure 6

# Figure 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016021973 A1
- EP 3009511 A2
- EP 2958996 A1
- WO 2015191693 A2
- WO 2016065364 A1
- US 62345448
- US 7022896 B
- WO 9741228 A
- US 2009029861 A1
- US 20120295960 A1
- US 7303910 B2

- US 20060281180 A
- US 20090007284 A
- US 20110117189 A
- US 20090017543 A
- US 20070054961 A
- US 20100317109 A
- US 20110293571 A
- US 20040013648 A
- US 20070025970 A
- US 20090111106 A
- US 7259015 B

**Non-patent literature cited in the description**

- **ZETSCHE et al.** Cpf1 Is a Single RNA-Guides Endonuclease of a Class 2 CRISPR-Cas System. *Cell,* October 2015, vol. 163, 1-13
- **MAKAROVA et al.** *Nature Rev. Microbial.,* 2015
- **JINEK et al.** A programmable dual-RNA-guided DNA endonuclease in adaptive bacterial immunity. *Science,* 2012, vol. 337, 816-821
- **MAKAROVA ; KOONIN.** *Methods. Mol. Biol.,* 2015, vol. 1311, 47-753
- **BURSTEIN et al.** New CRISPR-Cas systems from uncultivated microbes. *Nature,* December 2016
- **WYMAN C. ; KANAAR R.** DNA double-strand break repair: all's well that ends well. *Annu. Rev. Genet.,* 2006, vol. 40, 363-83
- **KWON, T. ; HUQ, E. ; HERRIN, D. L.** Microhomology-mediated and nonhomologous repair of a double-strand break in the chloroplast genome of Arabidopsis. *Proceedings of the National Academy of Sciences of the United States of America,* 2010, vol. 107 (31), 13954-13959
- **SWARTS, D.C. et al.** DNA-guided DNA interference by a prokaryotic Argonaute. *Nature,* 2014, vol. 507, 258-261
- **SWARTS, D.C. et al.** Argonaute of the archaeon Pyrococcus furiosus is a DNA-guided nuclease that targets cognate. *DNA Nucleic Acids Res.,* 2015, vol. 43, 5120-5129
- **MA et al.** CRISPR-Cas9 nuclear dynamics and target recognition in living cells. *JCB,* 2016, vol. 214 (5), 529
- **MACH.** *Plant Cell,* 2014

- **BALTES et al.** *Plant Cell,* 2014
- **LAITINEN et al.** Rational Design of an Active Avidin Monomer. *Journal of Biological Chemistry,* 2003, vol. 278 (6), 4010-4014
- **MANN et al.** Cell labeling and proximity dependent biotinylation with engineered monomeric streptavidin. *TECHNOLOGY,* 2016, vol. 4 (3), 1-7
- **KAY et al.** High-throughput Biotinylation of Proteins. *Methods in molecular biology (Clifton, N.J.),* 2009, vol. 498, 185-196
- **TISSOT et al.** Protein biotinylation in higher plants: characterization of biotin holocarboxylase synthetase activity from pea (Pisum sativum) leaves. *Biochemical Journal,* 1996, vol. 314, 391-395
- **KUFER et al.** *Trends in Biotechnology,* 2004, vol. 22 (5), 238-244
- **XIONG et al.** *Protein Engineering Design and Selection,* 2006, vol. 19 (8), 359-367
- **SAMBROOK ; FRITSCH ; MANIATIS.** Molecular Cloning: A Laboratory Manual. Cold Spring Harbor Laboratory Press, 1989
- DNA Cloning: A Practical Approach. 1985, vol. I,II
- Oligonucleotide Synthesis. 1984
- Nucleic Acid Hybridization. 1985
- Transcription and Translation. 1984
- Animal Cell Culture. 1986
- Immobilized Cells and Enzymes. IRL Press, 1986
- **B. PERBAL.** A Practical Guide To Molecular Cloning. 1984
- Current Protocols in Molecular Biology. John Wiley & Sons, Inc, 1994

- **SMITH, T.F. ; WATERMAN, M.S.** Identification of common molecular subsequences. *Journal of Molecular Biology,* 1981, vol. 147 (1), 195-197
- **NIEMEYER et al.** Functionalization of covalent DNA-streptavidin conjugates by means of biotinylated modulator components. *Bioconjug Chem,* 1999, vol. 10 (5), 708-719
- **ROY et al.** Prediction of DNA-binding specificity in zinc finger proteins. *J Biosci,* 2012, vol. 37 (3), 483-491
- **KUBO et al.** Cys2/His2 zinc-finger protein family of petunia: evolution and general mechanism of target-sequence recognition. *Nucleic Acids Research,* 1998, vol. 26 (2), 608-615
- **HUBBARD et al.** Continuous directed evolution of DNA-binding proteins to improve TALEN specificity. *Nat Methods,* 2015, vol. 12 (10), 939-942
- **SCHENK et al.** Generation and application of a fluorescein-specific single-chain antibody. *Biochimie,* 2007, vol. 89 (11), 1304-1311
- **RICHARDSON et al.** *Nature Biotechnology,* 2016
- **PORT ; BULLOCK.** *Nat. Methods,* 2016, vol. 13 (10), 852-854
- **TSAI et al.** *Nature Biotechnology,* 2014, vol. 32, 569-576
- **SHECHNER et al.** *Nature Methods,* 2015, vol. 12 (7), 664-670
- **TSAI et al.** *Nature Biotech.,* 2014, vol. 32
- **SHECHNER et al.** Multiplexable, locus-specific targeting of long RNAs with CRISPR-Display. *Nature Methods,* 2015, vol. 12 (7), 664-670
- **GRAEF et al.** *Immunity,* 2014, vol. 41, 116-126
- **FARBER et al.** *Nature Reviews Immunology,* 2014, vol. 14, 24-35
- **RIDDELL et al.** *Cancer J.,* 2014, vol. 20 (2), 141-144
- **ANURATHAPAN et al.** *Molecular Therapy,* 2014, vol. 22, 623-633
- **SJOUKJE et al.** *Nature Reviews Drug Discovery,* 2015, vol. 14, 499-509
- **BALAGAAN.** *J Gene Med,* 2006, vol. 8, 275-285
- **BINLEY et al.** *HUMAN GENE THERAPY,* September 2012, vol. 23, 980-991
- **MORRISSEY et al.** *Nature Biotechnology,* August 2005, vol. 23 (8
- **ZIMMERMANN et al.** *Nature Letters,* 04 May 2006, vol. 441
- **ZIMMERMANN et al.** *Nature Letters,* 2006, vol. 441
- **LI.** *Gene Therapy,* 2012, vol. 19, 775-780
- **GEISBERT et al.** *Lancet,* 2010, vol. 375, 1896-905
- **LAWRENCE et al.** *Journal of the American Chemical Society,* 2007, vol. 129, 10110-10112
- **AKINC et al.** *Nat. Biotech.,* 2010, vol. 26, 561-569
- **MCNAUGHTON et al.** *Proc. Natl. Acad. Sci. USA,* 2009, vol. 106, 6111-6116
- **GREEN ; SAMBROOK.** Molecular Cloning, A Laboratory Manual. Cold Spring Harbor Laboratory Press, 2012
- **SHEEN, J.** *A transient expression assay using maize mesophyll protoplasts,* 2002
- **PAREDDY DR et al.** Maturation of maize pollen in vitro. *Plant Cell Rep,* 1992, vol. 11 (10), 535-539
- **STAPLETON AE et al.** Immature maize spikelets develop and produce pollen in culture. *Plant Cell Rep,* 1992, vol. 11 (5-6), 248-252
- **PAREDDY DR et al.** Production of normal, germinable and viable pollen from in vitro-cultured maize tassels. *Theor Appl Genet,* 1989, vol. 77 (4), 521-526
- **TSAI et al.** Dimeric CRISPR RNA-guided Fokl nucleases for highly specific genome editing. *Nature Biotechnology,* 2014, vol. 32, 569-576
- **JONES, H.D. et al.** Review of methodologies and a protocol for the Agrobacterium-med iated transformation of wheat. *plant methods,* 2005
- **FRAME, B.R. et al.** Agrobacterium tumefaciens-med iated transformation of maize embryos using a standard binary vector system. *Plant,* 2002